# EUROPÄISCHE PATENTSCHRIFT

(11) **EP 2 417 112 B1**
(45) Veröffentlichungstag und Bekanntmachung des Hinweises auf die Patenterteilung: **09.07.2014**
(21) Anmeldenummer: 10713311.8
(22) Anmeldetag: 30.03.2010
(51) Int. Cl.: C07D 239/22, C07D 401/12, C07D 403/12, C07D 405/12, C07D 417/12, C07D 471/04, A61K 31/4412, A61K 31/443, A61K 31/4433, A61K 31/4436, A61K 31/4439, A61P 11/08, A61P 9/10

(54) **SULFONAMID- UND SULFOXIMIN-SUBSTITUIERTE DIARYLDIHYDROPYRIMIDINONE UND IHRE VERWENDUNG**
SULFONIC AMIDE AND SULFOXIMINE-SUBSTITUTED DIARYL-DIHYDROPYRIMIDINONES AND USAGE THEREOF
DIARYLDIHYDROPYRIMIDINONES À SUBSTITUTION SULFONAMIDE ET SULFOXIMINE ET LEUR UTILISATION

(30) Priorität: 06.04.2009 DE 102009016553
(43) Veröffentlichungstag der Anmeldung: 15.02.2012
(73) Patentinhaber: Bayer Intellectual Property GmbH, 40789 Monheim (DE)
(72) Erfinder: VON NUSSBAUM, Franz, 40219 Düsseldorf (DE); KARTHAUS, Dagmar, 42697 Solingen (DE); ANLAUF, Sonja, 42929 Wermelskirchen (DE); DELBECK, Martina, 45239 Essen (DE); LI, Volkhart, Min-Jian, 42553 Velbert (DE); MEIBOM, Daniel, 51373 Leverkusen (DE); LUSTIG, Klemens, 42113 Wuppertal (DE); SCHNEIDER, Dirk, 42115 Wuppertal (DE)
(74) Vertreter: BIP Patents
(86) Internationale Anmeldenummer: PCT/EP2010/002000
(87) Internationale Veröffentlichungsnummer: WO 2010/115548

(56) Entgegenhaltungen:
- WO-A1-2004/024700
- WO-A1-2009/080199

## Beschreibung

Die vorliegende Anmeldung betrifft neue, Sulfonamid- oder Sulfoximin-substituierte 1,4-Diaryl-dihydropyrimidin-2-on-Derivate, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von Erkrankungen der Lunge und des Herz-KreislaufSystems.

Die Humane Leukozyten-Elastase (HLE, EC 3.4.21.37), auch Humane Neutrophile Elastase (HNE, hNE) genannt, gehört zur Familie der Serinproteasen. Das proteolytische Enzym findet sich in den azurophilen Granula der polymorphkernigen Leukozyten (engl. *polymorphonuclear leukocytes, PMN leukocytes*). Die intrazelluläre Elastase nimmt eine wichtige Funktion in der Pathogenabwehr wahr, indem über Phagozytose aufgenommene Fremdpartikel abgebaut werden. Aktivierte neutrophile Zellen setzen die HNE aus den Granula in den Extrazellulärraum frei (extrazelluläre HNE), wobei ein Teil der freigesetzten HNE an der Außenseite der neutrophilen Zellmembran verbleibt (membranständige HNE). Das hochaktive Enzym ist in der Lage, eine Vielzahl von Bindegewebsproteinen abzubauen, z.B. die Proteine Elastin, Kollagen und Fibronektin. Elastin kommt in hohen Konzentrationen in allen Gewebetypen vor, die eine hohe Elastizität zeigen, z.B. in der Lunge und in Arterien. Bei einer Vielzahl von pathologischen Prozessen (z.B. Gewebeverletzungen) spielt die HNE eine Rolle beim Gewebeab- und -umbau (engl. *tissue remodeling*). Darüber hinaus ist die HNE ein wichtiger Modulator bei entzündlichen Prozessen. HNE induziert beispielsweise eine erhöhte Genexpression von Interleukin-8 (IL-8).

Es wird daher angenommen, dass die HNE bei vielen Erkrankungen, Verletzungen und pathologischen Veränderungen, deren Entstehung und/oder Progression mit einem entzündlichen Geschehen und/oder einem proliferativen und hypertrophen Gewebe- und Gefäßumbau in Zusammenhang steht, eine wichtige Rolle spielt. Dies können insbesondere Erkrankungen und/oder Schädigungen der Lunge oder des Herz-Kreislauf-Systems sein, oder es kann sich hierbei um eine Sepsis, um Krebs-Erkrankungen oder um andere entzündliche Erkrankungen handeln.

In diesem Zusammenhang zu nennende Erkrankungen und Schädigungen der Lunge sind insbesondere die chronisch-obstruktive Lungenerkrankung (engl. *chronic obstructive pulmonary disease,* COPD), das akute Atemwegssyndrom (engl. *acute respiratory distress syndrome,* ARDS), Bronchiektasien, Bronchiolitis obliterans, die zystische Fibrose (engl. *cystic fibrosis,* CF; auch Mukoviszidose genannt), das Lungenemphysem (engl. *lung emphysema*) und die akute Lungenschädigung (engl. *acute lung injury,* ALI). Erkrankungen und Schädigungen des Herz-KreislaufSystems, in denen die HNE involviert ist, sind zum Beispiel Gewebeveränderungen bei einer Herz-insuffizienz und Reperfusionsschäden nach einem Myokardinfarkt (engl. *acute myocardial infarct,* AMI), der kardiogene Schock, das akute Koronarsyndrom (engl. *acute coronary syndrome,* ACS) sowie Aneurysmen. Erkrankungen in Zusammenhang mit einer Sepsis sind beispielsweise eine systemische entzündliche Reaktion (engl. *systemic inflammatory response syndrome,* SIRS), die schwere Sepsis, der septische Schock und das multiple Organversagen (engl. *multi-organ failure,* MOF; *multi-organ dysfunction,* MODS) sowie die intravaskuläre Gerinnung (engl. *disseminated intravascular coagulation,* DIC). Beispiele für einen Gewebeab- und -umbau bei Krebsprozessen sind das Einwandern von Krebszellen in das gesunde Gewebe (Metastasenbildung) und die Neuausbildung von versorgenden Blutgefäßen (Neo-Angiogenese). Andere entzündliche Krankheiten, bei denen die HNE eine Rolle spielt, sind rheumatoide Erkrankungen, zum Beispiel die rheumatoide Arthritis, chronische Darmentzündungen (engl. *inflammatory bowel disease,* IBD; Morbus Crohn, engl. *Crohn's disease,* CD; Colitis ulcerosa, engl. *ulcerative colitis,* UC) und die Arteriosklerose.

Im Allgemeinen geht man davon aus, dass Elastase-vermittelten pathologischen Prozessen ein verschobenes Gleichgewicht zugrunde liegt zwischen der freien Elastase und dem körpereigenen Elastase-Inhibitorprotein (hauptsächlich das alpha-1-Antitrypsin, AAT) [Neutrophils and protease/antiprotease imbalance, Stockley, Am. J. Respir. Crit. Care Med. 160, 49-52 (1999)]. AAT liegt im Plasma im hohen Überschuss vor und neutralisiert so sehr schnell freie HNE. In verschiedenen pathologischen Prozessen ist die Konzentration an freier Elastase erhöht, so dass lokal die Balance zwischen Protease und Protease-Inhibitor zu Gunsten der Protease verschoben ist. Zudem ist die membranständige Elastase der aktivierten PMN-Zellen vor einer Inhibition durch AAT weitestgehend geschützt. Gleiches gilt für die freie Elastase, die sich in einem erschwert zugänglichen Mikrokompartiment zwischen der neutrophilen Zelle und der angrenzenden Gewebezelle (z.B. Endothelzelle) befindet. Zusätzlich herrschen stark oxidierende Bedingungen im Umfeld von aktivierten Leukozyten (engl. *oxidative burst*), wodurch AAT oxidiert wird und in der inhibitorischen Wirkung mehrere Größenordnungen verliert.

Neue Elastase-inhibierende Wirkstoffe (exogen verabreichte Inhibitoren der HNE) sollten demnach ein niedriges Molekulargewicht aufweisen, um in der Lage zu sein, auch die membranständige HNE und die im geschützten Mikrokompartiment befindliche HNE (s.o.) zu erreichen und zu inhibieren. Hierzu ist auch eine gute Stabilität der Substanzen *in vivo* notwendig (geringe *in vivo*-Clearance). Außerdem sollten diese Verbindungen stabil sein unter oxidativen Bedingungen, um im Krankheitsgeschehen nicht an inhibitorischer Potenz zu verlieren.

Die Pulmonale Arterielle Hypertonie (PAH) ist eine progrediente Lungenerkrankung, die unbehandelt durchschnittlich innerhalb von 2.8 Jahren nach Diagnosestellung zum Tode führt. Eine zunehmende Verengung der Lungenstrombahn führt zu einer Mehrbelastung des rechten Herzens, die bis zum Rechtsherzversagen gehen kann. Definitionsgemäß liegt bei einer chronischen pulmonalen Hypertonie ein pulmonal-arterieller Mitteldruck (mPAP) von > 25 mmHg in Ruhe oder > 30 mmHg unter Belastung vor (Normalwert < 20 mmHg). Die Pathophysiologie der pulmonal-arteriellen Hypertonie ist gekennzeichnet durch Vasokonstriktion und Remodeling der Pulmonalgefäße. Bei der chronischen PAH kommt es zu einer Neomuskularisierung primär nicht muskularisierter Lungengefäße, und die Gefäßmuskulatur der bereits muskularisierten Gefäße nimmt an Umfang zu. Durch diese zunehmende Obliteration der Lungenstrombahn kommt es zu einer progredienten Belastung des rechten Herzens, die zu einer verminderten Auswurfleistung des rechten Herzens führt und letztlich in einem Rechtsherzversagen endet (M. Humbert et al., J. Am. Coll. Cardiol. 2004, 43, 13S-24S). Mit einer Prävalenz von 1-2 pro einer Million handelt es sich bei PAH um eine äußerst seltene Erkrankung. Das mittlere Alter der Patienten wurde auf 36 Jahre geschätzt, nur 10% der Patenten waren über 60 Jahre alt. Deutlich mehr Frauen als Männer sind betroffen (G.E. D'Alonzo et al., Ann. Intern. Med. 1991, 115, 343-349).

Trotz aller Fortschritte in der Therapie der pulmonal-arteriellen Hypertonie gibt es bisher keine Aussicht auf Heilung dieser schwerwiegenden Erkrankung. Auf dem Markt befindliche Standardtherapien (z.B. Prostacyclin-Analoga, Endothelinrezeptor-Antagonisten, Phosphodiesterase-Inhibitoren) sind in der Lage, die Lebensqualität, die körperliche Belastbarkeit und die Prognose der Patienten zu verbessern. Hierbei handelt es sich um primär hämodynamische Therapieprinzipien, die den Gefäßtonus beeinflussen, jedoch keinen direkten Einfluss auf die pathogenen Remodeling-Prozesse haben. Darüber hinaus ist die Anwendbarkeit dieser Medikamente durch die z.T. gravierenden Nebenwirkungen und/oder aufwendigen Applikationsformen eingeschränkt. Der Zeitraum, über den unter einer spezifischen Monotherapie die klinische Situation der Patienten verbessert oder stabilisiert werden kann, ist begrenzt (z.B. aufgrund einer Toleranzentwicklung). Es erfolgt schließlich eine Therapieeskalation und somit eine Kombinationstherapie, bei der mehrere Medikamente gleichzeitig gegeben werden müssen.

Neue Kombinationstherapien sind eine der aussichtsreichsten zukünftigen Therapieoptionen zur Behandlung der pulmonalen arteriellen Hypertonie. In diesem Zusammenhang ist die Erkundung neuer pharmakologischer Mechanismen zur Behandlung der PAH von besonderem Interesse (Ghofrani et al., Herz 2005, 30, 296-302; E.B. Rosenzweig, Expert Opin. Emerging Drugs 2006, 11, 609-619; T. Ito et al., Curr. Med. Chem. 2007, 14, 719-733). Vor allem solche Therapieoptionen, die direkt in das Remodeling-Geschehen eingreifen (anti-Remodeling-Mechanismen, reverse-Remodeling-Mechanismen), könnten Grundlage einer mehr ursächlichen Behandlung sein und somit einen großen Vorteil für die Patienten bringen. Neue Therapien sollten hierbei mit den bekannten kombinierbar sein. Um in einer solchen Kombinationstherapie das Risiko für störende Wechselwirkungen zwischen den Medikamenten zu minimieren, sollten diese neuen Wirkstoffe metabolisierende P450 CYP-Enzyme nicht oder in nur sehr geringem Maße hemmen.

Heute geht man davon aus, dass die Elastase beim pathologischen Remodeling eine zentrale Rolle spielt. In Tiermodellen und in Patienten mit einem erhöhten arteriellen Lungenblutdruck (pulmonale arterielle Hypertension) konnte eine Fragmentierung des Bindegewebes (interne elastische Lamina) festgestellt werden [Rabinovitch et al., Lab. Invest. 55, 632-653 (1986)], und in Tiermodellen für die pulmonale arterielle Hypertension (hypoxisches Ratten- und Mausmodell, Monocrotalin-Rattenmodell) konnte eine erhöhte Elastase-Aktivität gezeigt werden, die mit der Fragmentierung des Bindegewebes einherging [Todorovich-Hunter et al., Am. Rev. Respir. Dis. 146, 213-223 (1992)]. Man vermutet, dass der zu beobachtende Gewebeumbau im Verlauf des Krankheitsgeschehens der pulmonalen arteriellen Hypertonie durch ein Elastase-vermitteltes Freisetzen von bindegewebsständigen Wachstumsfaktoren induziert wird, z.B. des basischen Fibroblasten-Wachstumsfaktors (engl. *basic fibroblast growth factor,* bFGF) [Rabinovitch, Am. J. Physiol. 277, L5-L12 (1999)]. Im hypoxischen Mausmodell für die pulmonale arterielle Hypertension konnte ein positiver Effekt mit einem überexprimierten Elastase-Inhibitorprotein gezeigt werden [Zaidi et al., Circulation 105, 516-521 (2002)]. Im Monocrotalin-Rattenmodell für die pulmonale arterielle Hypertension konnte eine positive Wirkung mit synthetischen, niedermolekularen Elastase-Inhibitoren gezeigt werden; hierbei war auch ein günstiger Effekt beim Gewebeumbau zu verzeichnen [Cowan et al., Nature Med. 6, 698-702 (2000)]. Alle bisher bekannten niedermolekularen Elastase-Inhibitoren sind jedoch wenig selektiv, chemisch reaktiv und/oder nur begrenzt oral verfügbar, was eine klinische Entwicklung eines oralen Elastase-Inhibitors in diesen Indikationen bisher vereitelte.

Der Begriff "pulmonale arterielle Hypertonie" umfasst bestimmte Formen der pulmonalen Hypertonie, wie sie z.B. von der Weltgesundheitsorganisation (WHO) festgelegt worden sind (Clinical Classification of Pulmonary Hypertension, Venedig 2003; G. Simonneau et al., J. Am. Coll. Cardiol. 2004, 43, 5S-12S).

Die pulmonale arterielle Hypertonie beinhaltet nach dieser Einteilung die Idiopathische Pulmonale Arterielle Hypertonie (IPAH, früher auch als primäre pulmonale Hypertonie, PPH, bezeichnet), die Familiär bedingte Pulmonale Arterielle Hypertonie (FPAH), die persistierende pulmonale Hypertonie der Neugeborenen sowie die Assoziierte Pulmonal-Arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (z.B. von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditäre Teleangiektasie, Hämoglobinopathien, myeloproliferative Erkrankungen und Splenektomie.

Andere Formen der pulmonalen Hypertonie umfassen beispielsweise die mit Linksherzerkrankungen assoziierte pulmonale Hypertonie, z.B. bei ventrikulären oder valvulären Erkrankungen, die mit Erkrankungen der Atemwege und/oder der Lunge assoziierte pulmonale Hypertonie, z.B. bei chronisch-obstruktiver Lungenerkrankung, interstitieller Lungenkrankheit oder Lungenfibrose, die auf chronische thrombotische und/oder embolische Erkrankungen zurückzuführende pulmonale Hypertonie, z.B. bei thromboembolischer Obstruktion von Lungenarterien, sowie die durch allgemein entzündliche Krankheitsprozesse oder durch spezielle Ursachen hervorgerufene pulmonale Hypertonie (z.B. bei Schistosomiasis, Sarkoidose und Tumorerkrankungen).

Die chronisch-obstruktive Lungenerkrankung (COPD) ist eine langsam fortschreitende Lungenerkrankung, die durch eine Behinderung der Atemströmung charakterisiert ist, welche durch ein Lungenemphysem und/oder eine chronische Bronchitis hervorgerufen wird. Die ersten Symptome der Erkrankung zeigen sich in der Regel ab dem vierten bis fünften Lebensjahrzehnt. In den darauffolgenden Lebensjahren verschlimmert sich häufig die Kurzatmigkeit und es manifestiert sich Husten, verbunden mit einem ausgiebigen und stellenweise eitrigen Auswurf und einer Stenose-Atmung bis hin zu einer Atemnot (Dyspnoe). COPD ist in erster Linie eine Krankheit von Rauchern: Rauchen ist verantwortlich für 90% aller COPD-Fälle und 80-90% aller COPD-Todesfälle. COPD ist ein großes medizinisches Problem und stellt weltweit die sechsthäufigste Todesursache dar. Von den über 45-jährigen Menschen sind ca. 4-6% betroffen.

Obwohl die Behinderung der Atemströmung nur partiell und zeitlich befristet sein kann, ist COPD nicht heilbar. Behandlungsziel ist folglich eine Verbesserung der Lebensqualität, die Linderung der Symptome, die Verhinderung akuter Verschlechterungen und die Verlangsamung der fortschreitenden Beeinträchtigung der Lungenfunktion. Bestehende Pharmakotherapien, die sich seit den letzten zwei bis drei Jahrzehnten kaum geändert haben, sind das Verwenden von Bronchodilatoren, um blockierte Atemwege zu öffnen, und in bestimmten Situationen Kortikosteroide, um die Entzündung der Lunge einzudämmen [P.J. Barnes, N. Engl. J. Med. 343, 269-280 (2000)]. Die chronische Entzündung der Lunge, hervorgerufen durch Zigarettenrauch oder andere Reizstoffe, ist die treibende Kraft der Krankheitsentwicklung. Der zugrunde liegende Mechanismus beinhaltet Immunzellen, die im Zuge der inflammatorischen Reaktion der Lunge verschiedene Chemokine ausschütten. Hierdurch werden neutrophile Zellen und im weiteren Verlauf alveolare Makrophagen zum Lungenbindegewebe und Lumen gelockt. Neutrophile Zellen sezernieren einen Protease-Cocktail, der hauptsächlich HNE und Proteinase 3 enthält. Hierdurch wird lokal die Protease/Antiprotease-Balance zu Gunsten der Proteasen verschoben, was u.a. zu einer unkontrollierten Elasase-Aktivität und in Folge hiervon zu einem überschießenden Abbau des Elastins der Alveolaren führt [J.E. Gadek et al., J. Clin. Invest. 68, 889-898 (1981); Z. Werb et al., J. Invest. Dermatol. 79, 154-159 (1982); A. Janoff, Am. Rev. Respir. Dis. 132, 417-433 (1985); P.J. Barnes, N. Engl. J. Med. 343, 269-280 (2000)]. Dieser Gewebeabbau verursacht einen Kollaps der Bronchien. Dies geht einher mit einer verminderten Elastizität der Lunge, was zu einer Behinderung der Atemströmung und beeinträchtigter Atmung führt. Darüber hinaus kann eine häufige und andauernde Entzündung der Lunge zu einem Remodeling der Bronchien und in der Folge zu einer Ausbildung von Läsionen führen. Solche Läsionen tragen zum Auftreten des chronischen Hustens bei, der eine chronische Bronchitis kennzeichnet.

Alpha-1-Antitrypsin (AAT) ist ein kleines körpereigenes Protein und stellt, wie oben erwähnt, den wichtigsten endogenen Elastase-Hemmer dar. Bei Patienten mit einer genetisch bedingten Defizienz dieses Proteins (AATD) ist die Protease/Antiprotease-Balance verschoben. Der Wirkradius und die Wirkdauer der HNE ist in AATD-Patienten entsprechend um einen Faktor 2.5 bzw. 6.5 erhöht [T.G. Liou und E.J. Campbell, Biochemistry 1995, 16171-16177]. AATD-Patienten haben ein erhöhtes Risiko, ein Lungenemphysem oder COPD zu entwickeln, und bei vielen AATD-Patienten ist eine Lungentransplantation indiziert.

Unter einer Bronchiektasie versteht man eine abnorme Ausweitung des Bronchialbaums. Zwei Formen können unterschieden werden: sackförmige, lokalisierte Bronchiektasen und generalisierte, zylindrische Bronchiektasen. Bronchiektasen können in angeborener Form auftreten, sind aber meist erworben und finden sich insbesondere bei Rauchern. Durch die Ausweitung ist die Entleerung des Bronchialsekrets erschwert, und durch das retinierte Bronchialsekret werden Infektionen begünstigt. Bronchiektasen finden sich oft auch bei angeborenen Schleimhauterkrankungen wie der Mukoviszidose mit abnormer Viskosität des Bronchialsekrets und beim Syndrom der ziliären Dyskinesie. Bei diesem Syndrom (Kartagener-Syndrom) sind die Zilienarchitektur und -funktion und dadurch die Sekretdrainage gestört. Andere Ursachen von Bronchiektasen können Obstruktionen proximal der Ektasie sein, z.B. durch Tumore oder Fremdkörper. Als ursächlich werden auch rezidivierende und persistierende Infekte angenommen, die die Bronchuswände schwächen. Ferner gibt es Bronchiektasien, die nicht eindeutig mit Infektionszuständen oder exogenen Noxen in Zusammenhang gebracht werden können (idiopathische Bronchiektasien).

Die Bronchiektasie ist charakterisiert durch eine Einwanderung von Neutrophilen in das Lungengewebe. Die Patienten zeigen ein starkes Ungleichgewicht zwischen Neutrophilenaktivität und schützenden Inhibitor-Proteinen, was zu einer Schädigung des Lungengewebes durch die von den Neutrophilen sezernierten Proteasen (hauptsächlich HNE) führt [Schaaf et al., Respiration 67, 52-59 (2000)].

Die Bronchiolitis obliterans ist eine in den Bronchioli angreifende Entzündung mit Epitheldestruktion und Bildung eines fibrinreichen Exsudats in den Bronchioli und den angrenzenden Alveolen. Durch Organisation des Exsudats entstehen Bindegewebspfröpfe, die aus den Bronchioli in die Alveolen hineinreichen. Die Erkrankung ist gekennzeichnet durch eine erhöhte Anzahl von Neutrophilen in den Atemwegen und ein Ungleichgewicht zwischen der freien Elastase und dem körpereigenen Elastase-Inhibitorprotein [Elssner et al., Transpl. Infect. Dis. 3, 168-176 (2001)]. Als Ursachen werden vorangegangene Infektionen sowie Medikamente diskutiert. Die Erkrankung kann auch im Rahmen einer Abstoßungsreaktion eines Transplantats vorkommen.

Die akute Lungenschädigung (ALI) sowie die ausgeprägtere Form hiervon, das akute Lungenversagen (ARDS), sind schwerwiegende Erkrankungen, die mit einer Mortalität von 50-60% einhergehen. Nach der Definition der North American-European Consensus Conference (NAECC) von 1994 sind ALI und ARDS definiert durch eine akute auslösende Erkrankung, bilaterale radiologisch sichtbare Infiltrate, einen PaO₂/FiO₂-Index von ≤ 300 mmHg (ALI) bzw. ≤ 200 mmHg (ARDS), einen pulmonal-kapillären Verschlussdruck von < 18 mmHg bzw. fehlende klinische Hinweise auf linksatriale Hypertension.

Der Entstehung einer akuten Lungenschädigung können sowohl pulmonale als auch extrapulmonale Erkrankungen vorausgehen. Als lungenspezifische prädisponierende Faktoren gelten Aspiration von Mageninhalt, Pneumonien, Rauchgasvergiftung, Lungenkontusion sowie ein Beinahe-Ertrinken. Vor allem Magensaftaspiration und Pneumonien werden häufig als Ausgangserkrankung für ALI/ARDS pulmonalen Ursprungs gesehen. Als indirekte Ereignisse kommen vor allem Polytrauma, Sepsis, mehrfache Bluttransfusionen, akute Pankreatitis und Verbrennungen vor. Die Inzidenz liegt bei 17.9 Fällen für ALI bzw. 13.5 Fällen für ARDS pro 100 000 Einwohner und Jahr [Luhr et al., Am. J. Respir. Crit. Care Med. 159, 1849-1861 (1999)].

Eine zentrale Rolle für die Entstehung dieser Erkrankungen stellen die massiven entzündlichen Veränderungen in der Lunge dar, die durch ein weitverzweigtes System von Mediatoren ausgelöst werden. Eine wichtige Rolle bei der Entwicklung der Lungenschädigung spielen auch die neutrophilen Granulozyten, deren Anzahl sich mit dem Andauern des entzündlichen Prozesses ständig erhöht [Chollet-Martin et al., Am. J. Respir. Crit. Care Med. 154, 594-601 (1996)]. Die Wirkung der Mediatoren bedingt eine Schädigung der alveolokapillären Membranen, hieraus resultiert eine Permeabilitätserhöhung der alveolär-kapillären Barriere. Durch die Permeabilitätserhöhung kann proteinreiche Flüssigkeit in die Alveolen und auch in das Interstitium eindringen; es bildet sich ein pulmonales Niederdrucködem aus. Charakteristisch für ALI/ARDS ist, dass es sich hierbei um ein nicht-kardiogen induziertes Ödem handelt. Die Ödemflüssigkeit enthält vor allem Fibrin, Erythrozyten, Leukozyten, hyaline Membranen und andere Proteine. Das proteinreiche Exsudat führt zusammen mit den Produkten von aktivierten Neutrophilen zu einer Dysfunktion des Surfactants. Die inflammatorischen Prozesse führen zur Schädigung und zum Verlust von Pneumozyten des Typs II, die Surfactant bilden, so dass ein Herabsinken der Surfactant-Produktion resultiert. Durch den Surfactant-Mangel erhöht sich die Oberflächenspannung in den Alveolen; Alveolen kollabieren, es bilden sich Atelektasen aus. Bei weiter bestehender Perfusion entsteht somit eine Ventilations-Perfusions-Störung, die in einer Erhöhung des pulmonalen Rechts-Links-Shunts mündet. Des Weiteren sinkt die Compliance herab, der alveoläre Totraum hingegen nimmt zu, denn es gibt auch Areale, die zwar belüftet, aber aufgrund einer pulmonalen Hypertension nicht mehr ausreichend perfundiert werden.

In der bronchoalveolaren Waschflüssigkeit von ARDS-Patienten (BALF) konnte eine erhöhte Elastase-Aktivität gemessen werden, die mit dem Schweregrad der Lungenschädigung einhergeht. In Tiermodellen, in denen die Lunge geschädigt wird (z.B. durch Gabe von LPS), kann dieser Effekt nachgestellt werden. Eine Behandlung mit Elastase-Hemmern (z.B. Sivelestat oder Elafin, s.u.) vermindert hier deutlich die Elastase-Aktivität in der BALF und verbessert die Lungenfunktion.

Zur Behandlung einer akuten Lungenschädigung, die mit SIRS assoziiert ist, ist ein Elastase-Hemmer in Japan und Südkorea zugelassen (Sivelestat, Elaspol^{®}). Die reversible, aber reaktive Verbindung besitzt nur eine relativ schwache Wirksamkeit gegenüber der HNE (Kᵢ 200 nM) und wirkt ebenfalls gegenüber der Elastase des Pankreas (IC₅₀ 5.6 µM). Der Wirkstoff wird intravenös verabreicht, eine orale Applikation ist nicht möglich.

Auch Elafin und strukturelle Analoga werden als therapeutisch nutzbare Elastase-Inhibitoren untersucht. Elafin ist ein körpereigenes kleines Protein, welches sowohl Elastase als auch Proteinase 3 hemmt. Aufgrund des proteinergen Charakters ist jedoch eine orale Verabreichung von Elafin nicht möglich.

Die Aufgabe der vorliegenden Erfindung liegt in der Bereitstellung neuer Substanzen, die als niedermolekulare, nicht-reaktive und selektive Inhibitoren der humanen neutrophilen Elastase (HNE) wirken und sich als solche zur Behandlung und/oder Prävention insbesondere von Erkrankungen der Lunge und des Herz-Kreislauf-Systems eignen.

In WO 2004/024700, WO 2004/024701, WO 2005/082863 und WO 2005/082864 werden verschiedene 1,4-Diaryl-dihydropyrimidin-2-on-Derivate als HNE-Inhibitoren zur Behandlung von chronisch-obstruktiven Lungenerkrankungen, akutem Koronarsyndrom, Myokardinfarkt und Herzinsuffizienz offenbart. Di- und Multimere solcher Verbindungen zur Behandlung von Atemwegserkrankungen werden in WO 2006/082412, WO 2006/136857 und WO 2007/042815 beansprucht. In WO 2008/003412 wird die Verwendung bestimmter 1,4-Diaryl-dihydropyrimidin-2-on-Derivate zur Behandlung der pulmonalen arteriellen Hypertonie offenbart. 4-Aryl-dihydropyrimidin-2-on-Derivate werden in WO 2005/009392 als Inhibitoren der Calciumkanal-Funktion zur Behandlung von Hypertonie beschrieben.

Es wurde nun gefunden, dass sich bestimmte 1,4-Diaryl-dihydropyrimidin-2-on-Derivate in besonderem Maße für die Behandlung und/oder Prävention von Erkrankungen eignen. Diese im Folgenden beschriebenen Verbindungen sind niedermolekulare, nicht-reaktive und selektive Inhibitoren der humanen neutrophilen Elastase (HNE), die überraschenderweise eine deutlich stärkere Inhibition dieser Protease im Vergleich zu den aus dem Stand der Technik bekannten Verbindungen bewirken. Darüber hinaus zeigen die erfindungsgemäßen Verbindungen eine unerwartet niedrige *in vitro*-Clearance gegenüber Hepatozyten und weisen damit eine verbesserte metabolische Stabilität auf. Manche der erfindungsgemäßen Verbindungen verfügen zudem über eine gute Löslichkeit in wässrigen Systemen, was vorteilhaft für ihre Formulierbarkeit und/oder intravenöse Anwendbarkeit ist. Die Substanzen der vorliegenden Erfindung stellen somit vielversprechende Ausgangspunkte für neue Arzneimittel zur Behandlung und/oder Prävention insbesondere von Erkrankungen der Lunge und des Herz-Kreislauf-Systems dar.

Im Einzelnen betrifft die vorliegende Erfindung Verbindungen der allgemeinen Formel (I) 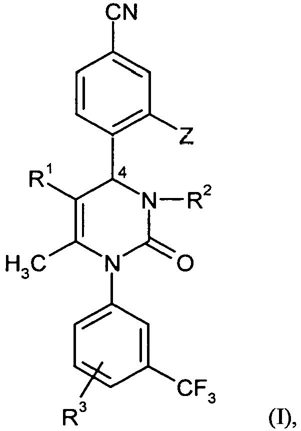 in welcher
- Z: für eine Sulfonamid-Gruppierung der Formel 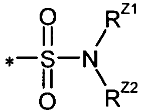 oder für eine Sulfoximin-Gruppierung der Formel 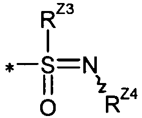 steht, worin
* die Verknüpfungsstelle mit dem Phenyl-Ring kennzeichnet,
R^{Z1} Wasserstoff oder (C₁-C₆)-Alkyl, das mit Hydroxy, (C₁-C₄)-Alkoxy, Amino, Mono- oder Di-(C₁-C₄)-alkylamino sowie bis zu dreifach mit Fluor substituiert sein kann, bedeutet,
R^{Z2} Wasserstoff, (C₃-C₆)-Cycloalkyl, 4- bis 6-gliedriges Heterocyclyl oder 5- oder 6-gliedriges Heteroaryl bedeutet
oder
(C₁-C₆)-Alkyl bedeutet, das mit Hydroxy, (C₁-C₄)-Alkoxy, Amino, Mono- oder Di-(C₁-C₄)-alkylamino, (C₁-C₄)-Alkylcarbonylamino, (C₁-C₄)-Alkoxycarbonylamino, (C₁-C₄)-Alkylsulfinyl, (C₁-C₄)-Alkylsulfonyl, (C₃-C₆)-Cycloalkyl, Phenyl, 4- bis 6-gliedrigem Heterocyclyl, 5- oder 6-gliedrigem Heteroaryl oder einer Gruppe der Formel -C(=O)-NR^{Z5}R^{Z6} sowie bis zu dreifach mit Fluor substituiert sein kann,
wobei der genannte Alkoxy-Substituent seinerseits bis zu dreifach mit Fluor substituiert sein kann,
und wobei
die genannten Heterocyclyl-Gruppen bis zu zweifach, gleich oder verschieden, mit Fluor, (C₁-C₄)-Alkyl, Oxo, Hydroxy, (C₁-C₄)-Alkoxy, Amino, Mono- und Di-(C₁-C₄)-alkylamino substituiert sein können
sowie
die genannte Phenyl-Gruppe und die genannten Heteroaryl-Gruppen bis zu zweifach, gleich oder verschieden, mit Fluor, Chlor, Cyano, (C₁-C₄)-Alkyl, Difluormethyl, Trifluormethyl und (C₁-C₄)-Alkoxy substituiert sein können,
und worin
R^{Z5} und R^{Z6} gleich oder verschieden sind und unabhängig voneinander Wasserstoff oder (C₁-C₄)-Alkyl darstellen oder
R^{Z5} und R^{Z6} zusammen mit dem Stickstoffatom, an das sie gebunden sind, einen 4- bis 6-gliedrigen Aza-Heterocyclus bilden, der ein weiteres Ring-Heteroatom aus der Reihe N, O oder S enthalten und mit (C₁-C₄)-Alkyl, Oxo, Hydroxy, (C₁-C₄)-Alkoxy, Amino, Mono- oder Di-(C₁-C₄)-alkylamino substituiert sein kann, oder
R^{Z1} und R^{Z2} zusammen mit dem Stickstoffatom, an das sie gebunden sind, einen 4- bis 10-gliedrigen Aza-Heterocyclus bilden, der ein weiteres Ring-Heteroatom aus der Reihe N, O oder S enthalten und bis zu zweifach, gleich oder verschieden, mit Fluor, (C₁-C₄)-Alkyl, Oxo, Hydroxy, (C₁-C₄)-Alkoxy, Amino, Mono- und Di-(C₁-C₄)-alkylamino substituiert sein kann,
R^{Z3} (C₁-C₆)-Alkyl, das mit (C₃-C₆)-Cycloalkyl oder bis zu dreifach mit Fluor substituiert sein kann, oder Phenyl, das bis zu zweifach, gleich oder verschieden, mit Fluor, Chlor, Cyano, (C₁-C₄)-Alkyl, Difluormethyl und Trifluormethyl substituiert sein kann, oder (C₃-C₆)-Cycloalkyl bedeutet, und
R^{Z4} Wasserstoff, (C₁-C₄)-Alkyl oder (C₃-C₆)-Cycloalkyl bedeutet,
- R¹: für Cyano oder Acetyl steht,
- R²: für Wasserstoff, (C₁-C₄)-Alkyl oder (C₁-C₄)-Alkylsulfonyl, welche jeweils bis zu dreifach mit Fluor substituiert sein können, oder eine Gruppe der Formel -CH₂-C(=O)-NH-R⁴ steht, worin
R⁴ Wasserstoff, (C₁-C₄)-Alkyl, das mit (C₃-C₆)-Cycloalkyl oder bis zu dreifach mit Fluor substituiert sein kann, oder (C₃-C₆)-Cycloalkyl bedeutet,
und
- R³: für Wasserstoff, Fluor oder Chlor steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachstehend aufgeführten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfosäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, 1-Ethylpropyl, *n*-Pentyl, Neopentyl und *n*-Hexyl.
(C₁-C₄)-Alkylcarbonyl steht im Rahmen der Erfindung für einen geradkettigen oder verzweigten Alkylrest mit 1 bis 4 Kohlenstoffatomen, der über eine Carbonylgruppe verknüpft ist. Beispielhaft und vorzugsweise seien genannt: Acetyl, Propionyl, *n*-Butyryl, *iso*-Butyryl, *n*-Pentanoyl und Pivaloyl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy und *tert*.-Butoxy.
(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, *n*-Propoxycarbonyl, Isopropoxycarbonyl, *n*-Butoxycarbonyl und *tert*.-Butoxycarbonyl.
Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino und *tert*.-Butylamino.
Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N-*Diethylamino, *N*-Ethyl-*N*-methylamino, *N*-Methyl-*N*-*n*-propylamino, *N*-Isopropyl-*N*-methylamino, *N-*Isopropyl-*N*-*n*-propylamino, *N,N*-Diisopropylamino, *N*-*n*-Butyl-*N*-methylamino und *N-tert*.-Butyl-*N*-methylamino.
(C₁-C₄)-Alkylcarbonylamino steht im Rahmen der Erfindung für eine Amino-Gruppe mit einem geradkettigen oder verzweigten Alkylcarbonyl-Substituenten, der 1 bis 4 Kohlenstoffatome im Alkylrest aufweist und über die Carbonylgruppe mit dem N-Atom verknüpft ist. Beispielhaft und vorzugsweise seien genannt: Acetylamino, Propionylamino, *n*-Butyrylamino, *iso*-Butyrylamino, *n*-Pentanoylamino und Pivaloylamino.
(C₁-C₄)-Alkoxycarbonylamino steht im Rahmen der Erfindung für eine Amino-Gruppe mit einem geradkettigen oder verzweigten Alkoxycarbonyl-Substituenten, der 1 bis 4 Kohlenstoffatome im Alkoxyrest aufweist und über die Carbonylgruppe mit dem N-Atom verknüpft ist. Beispielhaft und vorzugsweise seien genannt: Methoxycarbonylamino, Ethoxycarbonylamino, *n*-Propoxycarbonylamino, Isopropoxycarbonylamino, *n*-Butoxycarbonylamino und *tert*.-Butoxycarbonylamino.
(C₁-C₄)-Aklsulfinyl steht im Rahmen der Erfindung für einen geradkettigen oder verzweigten Alkylrest mit 1 bis 4 Kohlenstoffatomen, der über eine Sulfinylgruppe [-S(=O)-] verknüpft ist. Beispielhaft und vorzugsweise seien genannt: Methylsulfinyl, Ethylsulfinyl, *n*-Propylsulfinyl, Isopropylsulfinyl, *n*-Butylsulfinyl und *tert*.-Butylsulfinyl.
(C₁-C₄)-Alkylsulfonyl steht im Rahmen der Erfindung für einen geradkettigen oder verzweigten Alkylrest mit 1 bis 4 Kohlenstoffatomen, der über eine Sulfonylgruppe [-S(=O)₂-] verknüpft ist. Beispielhaft und vorzugsweise seien genannt: Methylsulfonyl, Ethylsulfonyl, *n*-Propylsulfonyl, Isopropylsulfonyl, *n*-Butylsulfonyl und *tert*.-Butylsulfonyl.
(C₃-C₆)-Cycloalkyl steht im Rahmen der Erfindung für eine monocyclische, gesättigte Cycloalkylgruppe mit 3 bis 6 Ring-Kohlenstoffatomen. Beispielhaft und vorzugsweise seien genannt: Cyclopropyl, Cyclobutyl, Cyclopentyl und Cyclohexyl.
Ein 4- bis 10-gliedriger Aza-Heterocyclus steht im Rahmen der Erfindung für einen mono- oder gegebenenfalls bicyclischen, gesättigten Heterocyclus mit insgesamt 4 bis 10 Ringatomen, der ein Ring-Stickstoffatom enthält, über das er zugleich verknüpft ist, und der darüber hinaus ein weiteres Ring-Heteroatom aus der Reihe N, O oder S enthalten kann. Beispielhaft und vorzugsweise seien genannt: Azetidinyl, Pyrrolidinyl, Pyrazolidinyl, 1,3-Oxazolidinyl, 1,3-Thiazolidinyl, Piperidinyl, Piperazinyl, Morpholinyl, Thiomorpholinyl, Hexahydroazepinyl, Hexahydro-1,4-diazepinyl, Octahydroazocinyl, Octahydropyrrolo[3,4-b]pyrrolyl, Octahydroindolyl, Octahydroisoindolyl, Octa-hydropyrrolo[3,2-b]pyridyl, Octahydropyrrolo[3,4-b]pyridyl, Octahydropyrrolo[3,4-c]pyridyl, Octahydropyrrolo[1,2-a]pyrazinyl, Decahydrochinolinyl, Decahydroisochinolinyl, Octahydro-pyrido[1,2-a]pyrazinyl, 7-Azabicyclo[2.2.1]heptyl, 3-Azabicyclo[3.2.0]heptyl, 3-Azabicyclo-[3.2.1]octyl, 8-Azabicyclo[3.2.1]octyl, 8-Oxa-3-azabicyclo[3.2.1]octyl und 9-Azabicyclo[3.3.1]-nonyl. Bevorzugt ist ein mono- oder gegebenenfalls bicyclischer 5- bis 10-gliedriger Aza-Heterocyclus, der neben dem Stickstoffatom ein weiteres Ring-Heteroatom aus der Reihe N oder O enthalten kann, wie beispielsweise Pyrrolidinyl, Pyrazolidinyl, 1,3-Oxazolidinyl, Piperidinyl, Piperazinyl, Morpholinyl, Hexahydroazepinyl, Hexahydro-1,4-diazepinyl, Octahydroazocinyl, Octahydropyrrolo[3,4-b]pyrrolyl, Octahydroindolyl, Octahydroisoindolyl, Octahydropyrrolo[3,2-b]-pyridyl, Octahydropyrrolo[3,4-b]pyridyl, Octahydropyrrolo[3,4-c]pyridyl, Octahydropyrrolo-[1,2-a]pyrazinyl, Decahydrochinolinyl, Decahydroisochinolinyl, Octahydropyrido[1,2-a]pyrazinyl, 7-Azabicyclo[2.2.1]heptyl, 3-Azabicyclo[3.2.0]heptyl, 3-Azabicyclo[3.2.1]octyl, 8-Azabicyclo-[3.2.1]octyl, 8-Oxa-3-azabicyclo[3.2.1]octyl und 9-Azabicyclo[3.3.1]nonyl. Besonders bevorzugt ist ein monocyclischer 5- oder 6-gliedriger Aza-Heterocyclus, der neben dem Stickstoffatom ein weiteres Ring-Heteroatom aus der Reihe N oder O enthalten kann, wie beispielsweise Pyrrolidinyl, 1,3-Oxazolidinyl, Piperidinyl, Piperazinyl und Morpholinyl.
4- bis 6-gliedriges Heterocyclyl steht im Rahmen der Erfindung für einen monocyclischen, gesättigten Heterocyclus mit insgesamt 4 bis 6 Ringatomen, der ein oder zwei Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Azetidinyl, Oxetanyl, Pyrrolidinyl, Pyrazolidinyl, Tetrahydrofuranyl, 1,3-Oxazolidinyl, Thiolanyl, 1,3-Thiazolidinyl, Piperidinyl, Piperazinyl, Tetrahydropyranyl, 1,4-Dioxanyl, Tetrahydrothiopyranyl, Morpholinyl und Thiomorpholinyl. Bevorzugt ist ein 4- bis 6-gliedriger Heterocyclus mit ein oder zwei Ring-Heteroatomen aus der Reihe N und/oder O, wie beispielsweise Azetidinyl, Oxetanyl, Pyrrolidinyl, Tetrahydrofuranyl, Piperidinyl, Piperazinyl, Tetrahydropyranyl, 1,4-Dioxanyl und Morpholinyl. Besonders bevorzugt ist ein 5- oder 6-gliedriger Heterocyclus mit ein oder zwei Ring-Heteroatomen aus der Reihe N und/oder O, wie beispielsweise Pyrrolidinyl, Tetrahydrofuranyl, Piperidinyl, Piperazinyl, Tetrahydropyranyl, 1,4-Dioxanyl und Morpholinyl.
5- oder 6-gliedriges Heteroaryl steht im Rahmen der Erfindung für einen aromatischen Heterocyclus (Heteroaromaten) mit insgesamt 5 bzw. 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Oxazolyl, Thiazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind 5- oder 6-gliedrige Heteroaryl-Reste mit ein oder zwei Ring-Heteroatomen aus der Reihe N, O und/oder S, wie beispielsweise Furyl, Pyrrolyl, Thienyl, Pyrazolyl, Imidazolyl, Oxazolyl, Thiazolyl, Isoxazolyl, Isothiazolyl, Pyridyl, Pyrimidinyl, Pyridazinyl und Pyrazinyl.
Ein Oxo-Substituent steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I), in welcher
- Z: für eine Sulfonamid-Gruppierung der Formel 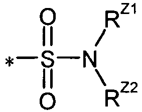 oder für eine Sulfoximin-Gruppierung der Formel 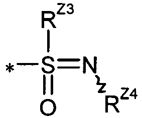 steht, worin
* die Verknüpfungsstelle mit dem Phenyl-Ring kennzeichnet,
R^{Z1} Wasserstoff oder (C₁-C₄)-Alkyl, das mit Hydroxy, Methoxy oder Ethoxy substituiert sein kann, bedeutet,
R^{Z2} Wasserstoff, (C₃-C₆)-Cycloalkyl, 5- oder 6-gliedriges Heterocyclyl oder 5- oder 6-gliedriges Heteroaryl bedeutet
oder
(C₁-C₄)-Alkyl bedeutet, das mit Hydroxy, (C₁-C₄)-Alkoxy, Amino, Mono- oder Di-(C₁-C₄)-alkylamino, (C₁-C₄)-Alkylcarbonylamino, (C₁-C₄)-Alkoxycarbonylamino, (C₁-C₄)-Alkylsulfinyl, (C₁-C₄)-Alkylsulfonyl, (C₃-C₆)-Cycloalkyl, Phenyl, 5- oder 6-gliedrigem Heterocyclyl, 5- oder 6-gliedrigem Heteroaryl oder einer Gruppe der Formel -C(=O)-NR^{Z5}R^{Z6} sowie bis zu dreifach mit Fluor substituiert sein kann,
wobei der genannte Alkoxy-Substituent seinerseits bis zu dreifach mit Fluor substituiert sein kann,
und wobei
die genannten Heterocyclyl-Gruppen bis zu zweifach, gleich oder verschieden, mit (C₁-C₄)-Alkyl, Oxo, Hydroxy und (C₁-C₄)-Alkoxy substituiert sein können
sowie
die genannte Phenyl-Gruppe und die genannten Heteroaryl-Gruppen bis zu zweifach, gleich oder verschieden, mit Fluor, Chlor, Cyano, (C₁-C₄)-Alkyl, Trifluormethyl und (C₁-C₄)-Alkoxy substituiert sein können,
und worin
R^{Z5} und R^{Z6} gleich oder verschieden sind und unabhängig voneinander Wasserstoff oder (C₁-C₄)-Alkyl darstellen oder
R^{Z5} und R^{Z6} zusammen mit dem Stickstoffatom, an das sie gebunden sind, einen 5- oder 6-gliedrigen Aza-Heterocyclus bilden, der ein weiteres Ring-Heteroatom aus der Reihe N oder O enthalten und mit (C₁-C₄)-Alkyl, Oxo, Hydroxy oder (C₁-C₄)-Alkoxy substituiert sein kann, oder
R^{Z1} und R^{Z2} zusammen mit dem Stickstoffatom, an das sie gebunden sind, einen 5- bis 10-gliedrigen Aza-Heterocyclus bilden, der ein weiteres Ring-Heteroatom aus der Reihe N oder O enthalten und bis zu zweifach, gleich oder verschieden, mit (C₁-C₄)-Alkyl, Oxo, Hydroxy und (C₁-C₄)-Alkoxy substituiert sein kann,
R^{Z3} (C₁-C₄)-Alkyl, das mit (C₃-C₆)-Cycloalkyl oder bis zu dreifach mit Fluor substituiert sein kann, oder Phenyl, das bis zu zweifach, gleich oder verschieden, mit Fluor, Chlor, Cyano, Methyl und Trifluormethyl substituiert sein kann, oder (C₃-C₆)-Cycloalkyl bedeutet, und
R^{Z4} Wasserstoff, Methyl oder Cyclopropyl bedeutet,
- R¹: für Cyano steht,
- R²: für Wasserstoff, (C₁-C₄)-Alkyl oder (C₁-C₄)-Alkylsulfonyl, welche jeweils bis zu dreifach mit Fluor substituiert sein können, oder eine Gruppe der Formel -CH₂-C(=O)-NH-R⁴ steht, worin
R⁴ Wasserstoff, Methyl, Cyclopropyl oder Cyclopropylmethyl bedeutet,
und
- R³: für Wasserstoff oder Fluor steht,
sowie ihre Salze, Solvate und Solvate der Salze.

Besonders bevorzugt im Rahmen der vorliegenden Erfindung sind Verbindungen der Formel (I), in welcher
- Z: für eine Sulfonamid-Gruppierung der Formel 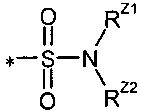 steht, worin
* die Verknüpfungsstelle mit dem Phenyl-Ring kennzeichnet,
R^{Z1} Wasserstoff, Methyl oder 2-Hydroxyethyl bedeutet,
R^{Z2} Wasserstoff, Cyclopropyl, 5- oder 6-gliedriges Heterocyclyl oder 5- oder 6-gliedriges Heteroaryl bedeutet
oder
(C₁-C₄)-Alkyl bedeutet, das mit Hydroxy, Methoxy, Ethoxy, Amino, Methylamino, Ethylamino, Dimethylamino, Diethylamino, Acetylamino, Cyclopropyl, 5- oder 6-gliedrigem Heterocyclyl oder einer Gruppe der Formel -C(=O)-NR^{Z5}R^{Z6} substituiert sein kann,
wobei die genannten Methoxy- und Ethoxy-Substituenten ihrerseits bis zu dreifach mit Fluor substituiert sein können,
und wobei
die genannten Heterocyclyl-Gruppen bis zu zweifach, gleich oder verschieden, mit Methyl, Ethyl, Oxo, Hydroxy, Methoxy und Ethoxy substituiert sein können
sowie
die genannte Heteroaryl-Gruppe bis zu zweifach, gleich oder verschieden, mit Fluor, Chlor, Cyano, Methyl, Ethyl, Trifluormethyl, Methoxy und Ethoxy substituiert sein kann,
und worin
R^{Z5} und R^{Z6} unabhängig voneinander Wasserstoff oder Methyl darstellen oder zusammen mit dem Stickstoffatom, an das sie gebunden sind, einen Pyrrolidin-, Piperidin- oder Morpholin-Ring bilden, oder
R^{Z1} und R^{Z2} zusammen mit dem Stickstoffatom, an das sie gebunden sind, einen Pyrrolidin-, Piperidin- oder Morpholin-Ring bilden,
- R¹: für Cyano steht,
- R²: für Wasserstoff, Methyl, Methylsulfonyl oder die Gruppe der Formel -CH₂-C(=O)-NH₂ steht,
und
- R³: für Wasserstoff steht,
sowie ihre Salze, Solvate und Solvate der Salze.

Besonders bevorzugt im Rahmen der vorliegenden Erfindung sind auch Verbindungen der Formel (I), in welcher
- Z: für eine Sulfoximin-Gruppierung der Formel 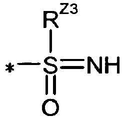 steht, worin
* die Verknüpfungsstelle mit dem Phenyl-Ring kennzeichnet und
R^{Z3} (C₁-C₄)-Alkyl, das mit Cyclopropyl oder bis zu dreifach mit Fluor substituiert sein kann, oder Cyclopropyl bedeutet,
- R¹: für Cyano steht,
- R²: für Wasserstoff, Methyl, Methylsulfonyl oder die Gruppe der Formel -CH₂-C(=O)-NH₂ steht,
und
- R³: für Wasserstoff steht,
sowie ihre Salze, Solvate und Solvate der Salze.

Ganz besonders bevorzugt im Rahmen der vorliegenden Erfindung sind Verbindungen der Formel (I), in welcher
- Z: für eine Sulfonamid-Gruppierung der Formel 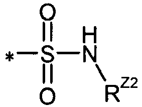 steht, worin
* die Verknüpfungsstelle mit dem Phenyl-Ring kennzeichnet und
R^{Z2} Wasserstoff, Methyl oder die Gruppe der Formel -CH₂-C(=O)-NH₂ bedeutet,
- R¹: für Cyano steht,
- R²: für Wasserstoff, Methyl oder Methylsulfonyl steht,
und
- R³: für Wasserstoff steht,
sowie ihre Salze, Solvate und Solvate der Salze.

Ganz besonders bevorzugt im Rahmen der vorliegenden Erfindung sind auch Verbindungen der Formel (I), in welcher
- Z: für eine Sulfoximin-Gruppierung der Formel 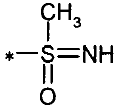 steht, worin
* die Verknüpfungsstelle mit dem Phenyl-Ring kennzeichnet,
- R¹: für Cyano steht,
- R²: für Wasserstoff, Methyl oder Methylsulfonyl steht,
und
- R³: für Wasserstoff steht,
sowie ihre Salze, Solvate und Solvate der Salze.

Von besonderer Bedeutung sind Verbindungen gemäß Formel (I) mit der in Formel (I-*ent*) wiedergegebenen *S*-Konfiguration an der 4-Position des Dihydropyrimidinon-Rings 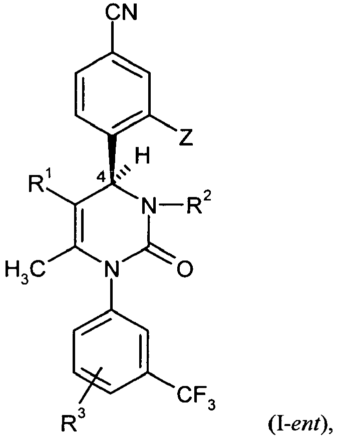 worin Z, R¹, R² und R³ jeweils die oben angegebenen Bedeutungen haben,
und ihre Salzt, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung von erfindungsgemäßen Verbindungen der Formel (I), in welcher
- Z: für eine Sulfonamid-Gruppierung der Formel 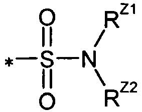 steht, worin
* die Verknüpfungsstelle mit dem Phenyl-Ring kennzeichnet und
R^{Z1} und R^{Z2} die oben angegebenen Bedeutungen haben,
dadurch gekennzeichnet, dass man ein Anilin-Derivat der Formel (II) 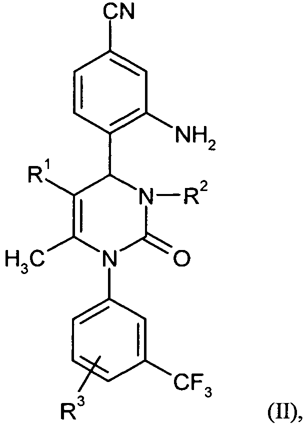 in welcher R¹, R² und R³ die oben angegebenen Bedeutungen haben,
zunächst mit Natriumnitrit und Salzsäure in das entsprechende Diazonium-Salz überführt, anschließend in einer Eintopf-Reaktion mit Schwefeldioxid in Gegenwart von Kupfer(I)chlorid zu einem Sulfochlorid der Formel (III) 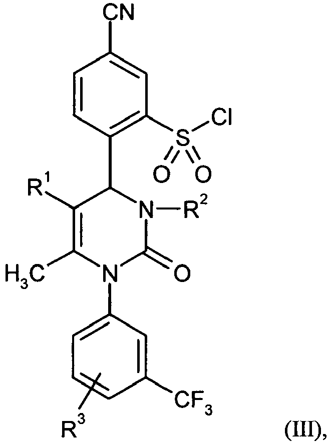 in welcher R¹, R² und R³ die oben angegebenen Bedeutungen haben,
umsetzt und dieses dann mit einem Amin der Formel (IV) 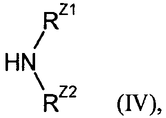 in welcher R^{Z1} und R^{Z2} die oben angegebenen Bedeutungen haben,
gegebenenfalls in Gegenwart einer Hilfsbase zum Sulfonamid der Formel (I-A) 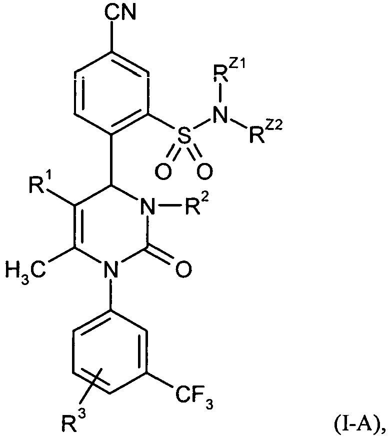 in welcher R¹, R², R³, R^{Z1} und R^{Z2} die oben angegebenen Bedeutungen haben,
reagiert
und gegebenenfalls die so erhaltenen Verbindungen der Formel (I-A) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Die Diazotierung und nachfolgende Sulfochlorierung im Verfahrensschritt (II) → (III) erfolgt nach üblichen, dem Fachmann geläufigen Methoden, indem das Anilin-Derivat der Formel (II) zunächst durch Reaktion mit Natriumnitrit in wässriger Salzsäure bei -20°C bis 0°C in das Diazonium-Salz überführt wird, welches dann *in situ* bei -20°C bis +20°C mit einer gesättigten Lösung von Schwefeldioxid in Essigsäure in Gegenwart von Kupfer(I)chlorid als Katalysator weiter umgesetzt wird.

Inerte Lösungsmittel für die Sulfonamid-Bildung im Verfahrensschritt (III) + (IV) → (I-A) sind übliche organische Lösungsmittel, die sich unter den Reaktionsbedingungen nicht verändern. Dazu zählen beispielsweise Ether wie Diethylether, Diisopropylether, Methyl-*tert*.-butylether, 1,2-Dimethoxyethan, 1,4-Dioxan oder Tetrahydrofuran, Kohlenwasserstoffe wie Pentan, Hexan, Cyclohexan, Benzol, Toluol oder Xylol, Halogenkohlenwasserstoffe wie Dichlormethan, 1,2-Dichlorethan, Trichlormethan oder Chlorbenzol, oder andere Lösungsmittel wie Ethylacetat, Acetonitril, Pyridin, Dimethylsulfoxid (DMSO), *N,N*-Dimethylformamid (DMF), *N,N'*-Dimethylpropylenharnstoff (DMPU) oder *N*-Methylpyrrolidinon (NMP). Ebenso ist es möglich, Gemische solcher Lösungsmittel einzusetzen. Bevorzugt wird Tetrahydrofuran, 1,4-Dioxan, Dichlormethan oder 1,2-Dichlorethan verwendet.

Die Umsetzung (III) + (IV) → (I-A) wird üblicherweise in Gegenwart einer Hilfsbase durchgeführt. Hierfür eignen sich insbesondere tertiäre organische Amin-Basen wie Triethylamin, *N,N-*Diisopropylethylamin, N-Methylmorpholin, N-Methylpiperidin, 1,5-Diazabicyclo[4.3.0]non-5-en (DBN), 1,8-Diazabicyclo[5.4.0]undec-7-en (DBU), Pyridin oder 4-*N,N*-Dimethylaminopyridin; bevorzugt wird Triethylamin oder *N,N*-Diisopropylethylamin verwendet. Gegebenenfalls kann die Umsetzung aber auch mit einem Überschuss des Amins (IV), ohne weiteren Zusatz einer Hilfsbase, erfolgen.

Der Verfahrensschritt (III) + (IV) → (I-A) wird im Allgemeinen in einem Temperaturbereich von -20°C bis +60°C, bevorzugt bei 0°C bis +40°C durchgeführt. Die Reaktion kann bei normalem, bei erhöhtem oder bei erniedrigtem Druck erfolgen (z.B. von 0.5 bis 5 bar); in der Regel arbeitet man bei Normaldruck. Weiterer Gegenstand der Erfindung ist ein Verfahren zur Herstellung von erfindungsgemäßen Verbindungen der Formel (I), in welcher
- Z: für eine Sulfoximin-Gruppierung der Formel 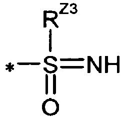 steht, worin
* die Verknüpfungsstelle mit dem Phenyl-Ring kennzeichnet und
R^{Z3} die oben angegebene Bedeutung hat,
dadurch gekennzeichnet, dass man ein Phenylthioether-Derivat der Formel (V) 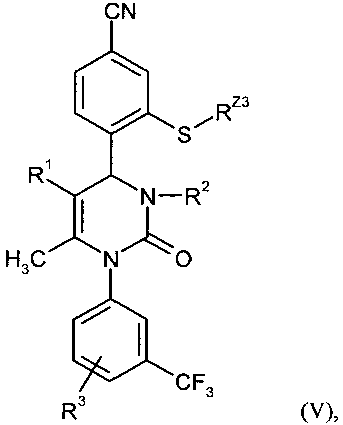 in welcher R¹, R², R³ und R^{Z3} die oben angegebenen Bedeutungen haben,
zunächst mit Wasserstoffperoxid, einer Persäure oder einem Periodat zum Sulfoxid der Formel (VI) 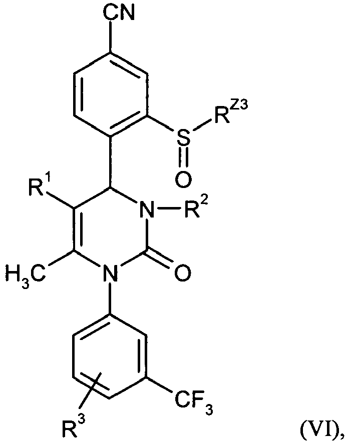 in welcher R¹, R², R³ und R^{Z3} die oben angegebenen Bedeutungen haben,
oxidiert, anschließend mit 2,2,2-Trifluoracetamid und (Diacetoxyiod)-benzol in Gegenwart von dimerem Rhodium(II)acetat als Katalysator und Magnesiumoxid als Base zu einem *N*-Acyl-Sulfoximin der Formel (VII) 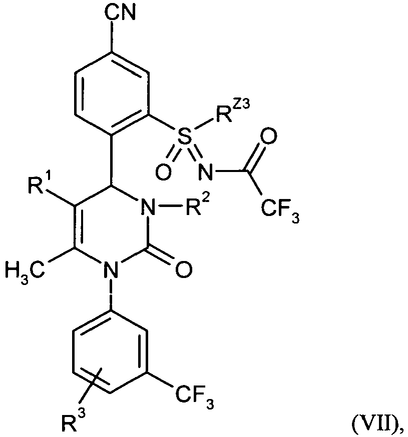 in welcher R¹, R², R³ und R^{Z3} die oben angegebenen Bedeutungen haben,
umsetzt und die Trifluoracetyl-Gruppe in (VII) dann unter basischen Bedingungen zum Sulfoximin der Formel (I-B) 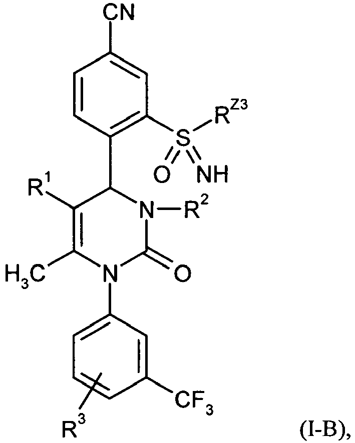 in welcher R¹, R², R³ und R^{Z3} die oben angegebenen Bedeutungen haben,
abspaltet
und gegebenenfalls die so erhaltenen Verbindungen der Formel (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Als Oxidationsmittel für den Verfahrensschritt (V) → (VI) sind insbesondere organische oder anorganische Peroxo-Verbindungen geeignet. Dazu zählen beispielsweise Wasserstoffperoxid, gegebenenfalls unter Mitwirkung eines Katalysators, Persäuren wie Peressigsäure oder *m*-Chlorperbenzoesäure, oder Salze solcher Verbindungen wie Natriumperiodat. Bevorzugt wird Wasserstoffperoxid, in Gegenwart von Methyltrioxorhenium als Katalysator, oder Natriumperiodat eingesetzt.

Die Oxidation (V) → (VI) wird vorzugsweise in alkoholischen Lösungsmitteln wie Methanol oder Ethanol, gegebenenfalls unter Zusatz von Wasser, in einem Temperaturbereich von -20°C bis +100°C, bevorzugt bei 0°C bis +60°C, durchgeführt.

Die Transformation des Sulfoxids (VI) zum *N*-Trifluoracetyl-Sulfoximin (VII) erfolgt nach einer literaturbeschriebenen Methode über eine Metall-katalysierte, oxidative Iminierungsreaktion mit 2,2,2-Trifluoracetamid und (Diacetoxyiod)-benzol in Gegenwart von dimerem Rhodium(II)acetat als Katalysator und Magnesiumoxid als Base [vgl. H. Okamura und C. Bolm, Org. Lett. 6 (8), 1305-1307 (2004)]. Die Reaktion wird vorzugsweise in Dichlormethan als Lösungsmittel in einem Temperaturbereich von 0°C bis +40°C durchgeführt.

Die Abspaltung der Trifluoracetyl-Gruppe im Verfahrensschritt (VII) → (I-B) geschieht auf übliche Weise durch Behandlung mit einem Alkalicarbonat oder -hydroxid in einem alkoholischen oder wässrigen Lösungsmittel. Bevorzugt wird Kaliumcarbonat in Methanol oder Acetonitril/ Methanol-Gemischen eingesetzt. Die Reaktion wird im Allgemeinen in einem Temperaturbereich von -10°C bis +30°C durchgeführt.

Erfindungsgemäße Sulfoximin-Derivate der Formel (I-C) 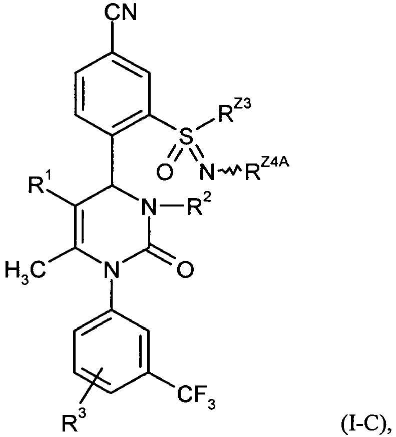 in welcher R¹, R², R³ und R^{Z3} die oben angegebenen Bedeutungen haben
und
- R^{Z4A}: für (C₁-C₄)-Alkyl oder (C₃-C₆)-Cycloalkyl steht,
können durch Reaktion der oben beschriebenen Verbindungen (I-B) mit einer Verbindung der Formel (VIII)

R^{Z4A}-X¹ (VIII),

in welcher R^{Z4A} die oben angegebene Bedeutung hat
und
- X¹: für eine Abgangsgruppe wie beispielsweise Halogen, Mesylat, Tosylat oder Triflat steht,
in Gegenwart einer starken Base, wie beispielsweise Natrium- oder Kalium-*tert*.-butylat oder Natrium- oder Kaliumhydrid, erhalten werden. Gegebenenfalls kann hierbei die Verwendung eines Phasen-Transfer-Katalysators, wie Tetrabutylammoniumbromid oder Benzyltriethylammoniumchlorid, von Vorteil sein [vgl. z.B. C.R. Johnson und O.M. Lavergne, J. Org. Chem. 58 (7), 1922-1923 (1993)].

Die Verbindungen der Formel (II) können in Analogie zu literaturbeschriebenen Verfahren beispielsweise hergestellt werden, indem man 4-Cyano-2-nitrobenzaldehyd der Formel (IX) 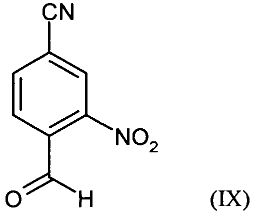 in Gegenwart einer Säure oder eines Säureanhydrids in einer 3-Komponenten-Eintopf-Reaktion oder sequentiell mit einer Keto-Verbindung der Formel (X) 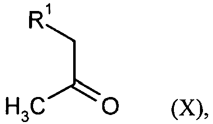 in welcher R¹ die oben angegebene Bedeutung hat,
und einem Phenylharnstoff-Derivat der Formel (XI) 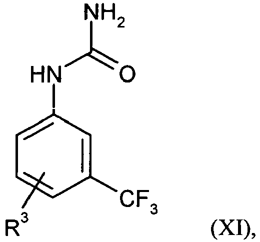 in welcher R³ die oben angegebene Bedeutung hat,
zu einer Verbindung der Formel (XII-A) 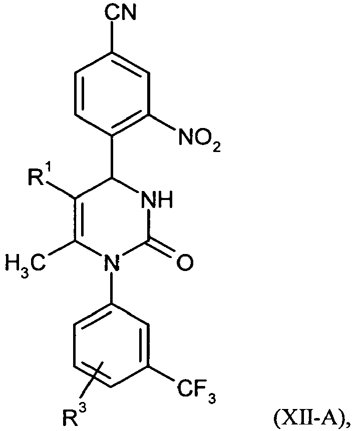 in welcher R¹ und R³ die oben angegebenen Bedeutungen haben,
kondensiert, diese im Falle, dass der Rest R² in Formel (I) nicht für Wasserstoff steht, in Gegenwart einer Base mit einer Verbindung der Formel (XIII)

R^{2A}-X² (XIII),

in welcher
- R^{2A}: die oben angegebene Bedeutung von R² hat, jedoch nicht für Wasserstoff steht,
und
- X²: für eine Abgangsgruppe wie beispielsweise Halogen, Mesylat, Tosylat oder Triflat steht,
zu einer Verbindung der Formel (XII-B) 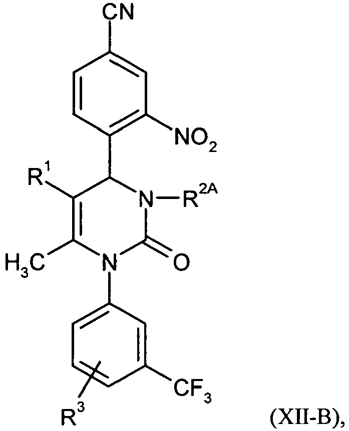 in welcher R¹, R^{2A} und R³ die oben angegebenen Bedeutungen haben,
umsetzt und die Nitro-Verbindung der Formel (XII-A) bzw. (XII-B) dann zum Anilin-Derivat der Formel (II) 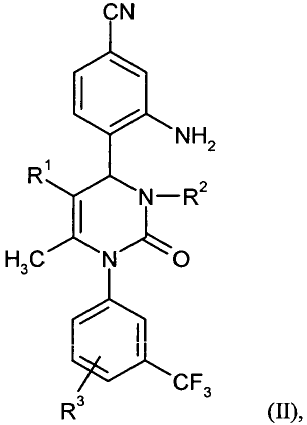 in welcher R¹, R² und R³ die oben angegebenen Bedeutungen haben,
reduziert.

Für den Verfahrensschritt (IX) + (X) + (XI) → (XII-A) geeignete Lösungsmittel sind übliche organische Lösungsmittel, die sich unter den Reaktionsbedingungen nicht verändern. Dazu zählen beispielsweise Ether wie Diethylether, Diisopropylether, Methyl-*tert*.-butylether, 1,2-Dimethoxyethan, 1,4-Dioxan oder Tetrahydrofuran, Alkohole wie Methanol, Ethanol, *n*-Propanol, Isopropanol, *n*-Butanol oder *tert*.-Butanol, Kohlenwasserstoffe wie Pentan, Hexan, Cyclohexan, Benzol, Toluol oder Xylol, Halogenkohlenwasserstoffe wie Dichlormethan, 1,2-Dichlorethan, Trichlormethan oder Chlorbenzol, oder andere Lösungsmittel wie Ethylacetat, Acetonitril, Dimethylsulfoxid oder *N,N*-Dimethylformamid. Ebenso ist es möglich, Gemische solcher Lösungsmittel einzusetzen. Bevorzugt wird Methyl-*tert*.-butylether, Tetrahydrofuran oder 1,4-Dioxan verwendet.

Als Säure für den Verfahrensschritt (IX) + (X) + (XI) → (XII-A) eignen sich übliche anorganische oder organische Säuren oder Säureanhydride. Hierzu gehören bevorzugt Carbonsäuren wie beispielsweise Essigsäure oder Trifluoressigsäure, Sulfonsäuren wie Methansulfonsäure, Trifluormethansulfonsäure oder *p*-Toluolsulfonsäure, Salzsäure, Schwefelsäure, Phosphorsäure, Phosphonsäuren, oder Phosphorsäure- oder Phosphonsäureanhydride oder -ester wie Polyphosphorsäure, Phosphorsäuretriethylester, Polyphosphorsäureethylester, Phosphorpentoxid oder Propanphosphonsäureanhydrid. Bevorzugt wird Phosphorsäuretriethylester in Kombination mit Phosphorpentoxid verwendet. Die Säure wird im Allgemeinen in einer Menge von 0.25 Mol bis 100 Mol, bezogen auf 1 Mol der Verbindung (X), eingesetzt.

Der Verfahrensschritt (IX) + (X) + (XI) → (XII-A) wird im Allgemeinen in einem Temperaturbereich von +20°C bis +150°C, bevorzugt bei +50°C bis +100°C durchgeführt. Die Umsetzung kann bei normalem, erhöhtem oder erniedrigtem Druck erfolgen (z.B. von 0.5 bis 5 bar); in der Regel arbeitet man bei Normaldruck.

Für den Verfahrensschritt (XII-A) + (XIII) → (XII-B) geeignete Lösungsmittel sind übliche organische Lösungsmittel, die sich unter den Reaktionsbedingungen nicht verändern. Dazu zählen beispielsweise Ether wie Diethylether, Diisopropylether, Methyl-*tert*.-butylether, 1,2-Dimethoxyethan, 1,4-Dioxan oder Tetrahydrofuran, Kohlenwasserstoffe wie Pentan, Hexan, Cyclohexan, Benzol, Toluol oder Xylol, Halogenkohlenwasserstoffe wie Dichlormethan, 1,2-Dichlorethan, Trichlormethan oder Chlorbenzol, oder andere Lösungsmittel wie Aceton, Methylethylketon, Methyl*tert*.-butylketon, Acetonitril, Dimethylsulfoxid, *N,N-*Dimethylformamid, *N,N*'-Dimethylpropylenharnstoff (DMPU) oder N-Methylpyrrolidinon (NMP). Ebenso ist es möglich, Gemische solcher Lösungsmittel einzusetzen. Bevorzugt wird Tetrahydrofuran, Acetonitril oder *N,N*-Dimethylformamid verwendet.

Als Base für den Verfahrensschritt (XII-A) + (XIII) → (XII-B) eignen sich übliche anorganische oder organische Basen. Hierzu gehören insbesondere Alkali- oder Erdalkalicarbonate wie Lithium-, Natrium-, Kalium-, Calcium- oder Cäsiumcarbonat, Alkali-Alkoholate wie Natrium- oder Kalium-*tert*.-butylat, Alkalihydride wie Natrium- oder Kaliumhydrid, Amide wie Lithium- oder Kalium-bis(trimethylsilyl)amid oder Lithiumdiisopropylamid (LDA), organische Amine wie Triethylamin, *N*-Methylmorpholin, *N*-Methylpiperidin, *N,N*-Diisopropylethylamin, 1,5-Diazabicyclo[4.3.0]non-5-en (DBN), 1,8-Diazabicyclo[5.4.0]undec-7-en (DBU), Pyridin oder 4-*N,N*-Dimethylaminopyridin, oder Phosphazen-Basen (so genannte "Schwesinger-Basen") wie beispielsweise P1-t-Bu, P2-t-Bu oder P4-t-Bu. Bevorzugt wird Kaliumcarbonat, Cäsiumcarbonat, Natriumhydrid, Triethylamin, *N,N*-Diisopropylethylamin oder Lithium-bis(trimethylsilyl)amid verwendet; besonders bevorzugt sind Natriumhydrid und Lithium-bis(trimethylsilyl)amid. Die Base wird im Allgemeinen in einer Menge von 0.1 Mol bis 10 Mol, bevorzugt von 1 Mol bis 3 Mol, bezogen auf 1 Mol der Verbindung (XII-A), eingesetzt.

Der Verfahrensschritt (XII-A) + (XIII) → (XII-B) wird im Allgemeinen in einem Temperaturbereich von -78°C bis +100°C, bevorzugt bei -78°C bis +80°C, besonders bevorzugt bei -78°C bis +25°C durchgeführt. Die Umsetzung kann bei normalem, erhöhtem oder erniedrigtem Druck erfolgen (z.B. von 0.5 bis 5 bar); in der Regel arbeitet man bei Normaldruck.

Die Reduktion der Nitro-Verbindung (XII-A) bzw. (XII-B) zum Anilin-Derivat (II) erfolgt nach Standardmethoden durch katalytische Hydrierung in Gegenwart eines üblichen Palladium- oder Platin-Katalysators; bevorzugt wird Palladium auf Aktivkohle verwendet. Die Hydrierung kann bei normalem oder bei erhöhtem Wasserstoff-Druck geschehen; in der Regel arbeitet man bei Normaldruck. Die Reaktion wird vorzugsweise bei Raumtemperatur in alkoholischen Lösungsmitteln wie Methanol oder Ethanol, gegebenenfalls unter Verwendung von inerten Ko-Solventien wie beispielsweise Tetrahydrofuran oder Ethylacetat, durchgeführt.

Im Fall, dass der Rest R¹ in Formel (I) für Cyano steht, kann gemäß einer Verfahrensvariante an Stelle der Verbindung (X) auch ein Acetessigester der Formel (XIV) 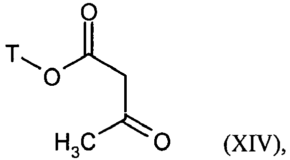 in welcher
- T: für (C₁-C₄)-Alkyl oder Allyl steht,
in der Kondensationsreaktion mit den Verbindungen (IX) und (XI) eingesetzt werden; das resultierende Produkt der Formel (XV) 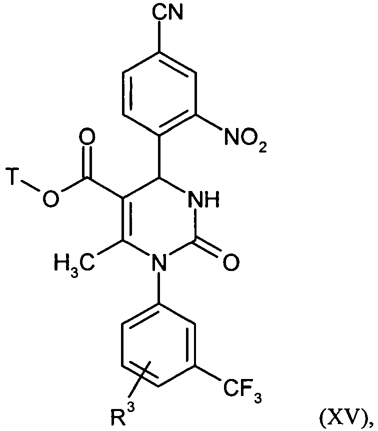 in welcher R³ und T die oben angegebenen Bedeutungen haben, kann dann nach Standardmethoden über eine Ester-Spaltung zur Carbonsäure der Formel (XVI) 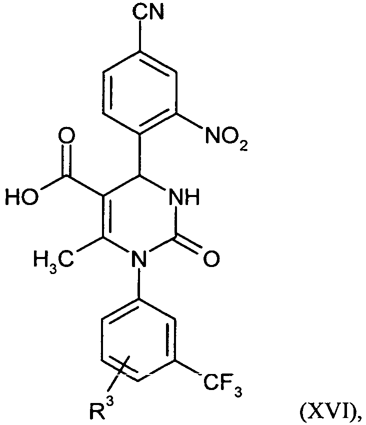 in welcher R³ die oben angegebene Bedeutung hat, anschließende Überführung in das primäre Carbonamid der Formel (XVII) 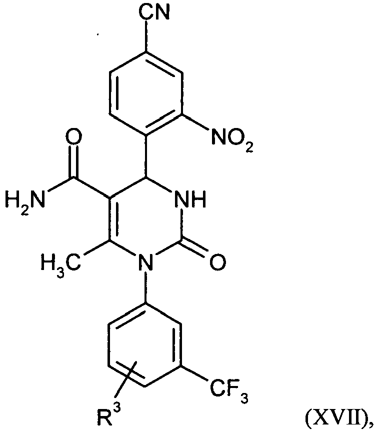 in welcher R³ die oben angegebene Bedeutung hat,
und nachfolgende Dehydratisierung der Amid-Gruppierung in das 5-Cyano-Dihydropyrimidinon der Formel (XII-A) [R' = CN] umgewandelt werden (vgl. das nachfolgende Reaktionsschema 1).

Die Verbindungen der Formel (V) können auf analoge Weise hergestellt werden, indem man zunächst 4-Cyano-2-fluorbenzaldehyd der Formel (XVIII) 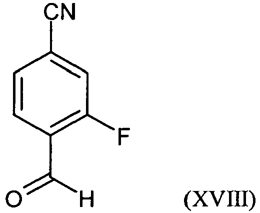 mit einem Thiol der Formel (XIX)

R^{Z3}-SH (XIX),

in welcher R^{Z3} die oben angegebene Bedeutung hat,
in Gegenwart einer Base zu einem 2-Sulfanyl-substituierten Benzaldehyd der Formel (XX) 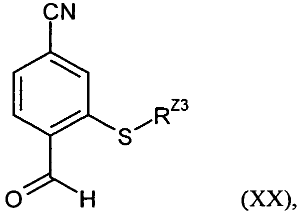 in welcher R^{Z3} die oben angegebene Bedeutung hat,
reagiert und diesen dann im Austausch gegen die Verbindung (IX) gemäß der zuvor beschriebenen Reaktionssequenz (IX) + (X) + (XI) → (XII-A) → (XII-B) oder (IX) + (XIV) + (XI) → (XV) → (XVI) → (XVII) → (XII-A) weiter umsetzt (vgl. das nachfolgende Reaktionsschema 2).

Weitere erfindungsgemäße Verbindungen der Formel (I) können, falls zweckmäßig, auch durch Umwandlungen von funktionellen Gruppen einzelner Substituenten, insbesondere den unter R^{Z1} und R^{Z2} aufgeführten, ausgehend von anderen, nach obigem Verfahren erhaltenen Verbindungen der Formel (I) hergestellt werden. Diese Umwandlungen werden nach üblichen, dem Fachmann bekannten Methoden durchgeführt und umfassen beispielsweise Reaktionen wie nukleophile oder elektrophile Substitutionsreaktionen, Übergangsmetall-vermittelte Kupplungsreaktionen (z.B. Suzuki-, Heck- oder Hartwig-Buchwald-Reaktion), Oxidation, Reduktion, Hydrierung, Alkylierung, Acylierung, Aminierung, Hydroxylierung, Veretherung, Veresterung, Esterspaltung und -hydrolyse, Bildung von Nitrilen, Carbonamiden und Carbamaten, sowie die Einführung und Entfernung temporärer Schutzgruppen.

Eine Trennung der erfindungsgemäßen Verbindungen in die entsprechenden Enantiomere und/ oder Diastereomere kann, je nach Zweckmäßigkeit, auf der Stufe der Verbindungen (I-A), (I-B) und (I-C) oder bereits auf der Stufe der Verbindungen (II), (V), (VI) oder (VII) oder auch der Intermediate (XII-A), (XII-B), (XV), (XVI) oder (XVII) bzw. deren R^{Z3}S-substituierten Analoga erfolgen, wobei diese Intermediate dann in separierter Form entsprechend den zuvor beschriebenen Verfahrensschritten weiter umgesetzt werden. Eine solche Auftrennung der Stereoisomeren läßt sich nach üblichen, dem Fachmann bekannten Methoden durchführen; vorzugsweise werden chromatographische Verfahren, insbesondere die HPLC-Chromatographie an chiraler Phase, verwendet.

Die Verbindungen der Formeln (IV), (VIII), (IX), (X), (XI), (XIII), (XIV), (XVIII) und (XIX) sind kommerziell erhältlich oder als solche literaturbekannt, oder sie können nach gängigen, in der Literatur beschriebenen Methoden hergestellt werden.

Die oben beschriebenen Verfahren können durch die folgenden Reaktionsschemata beispielhaft veranschaulicht werden:

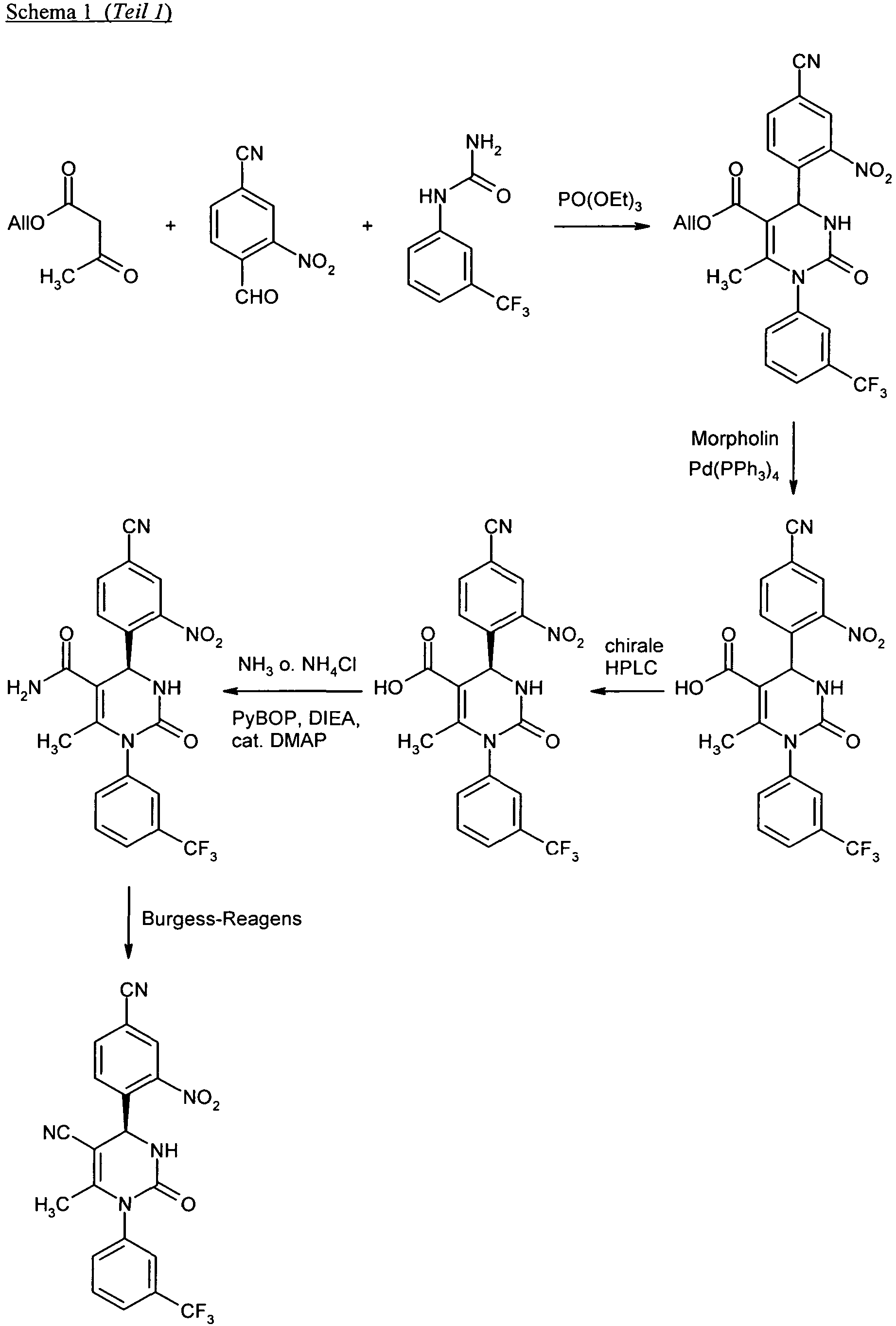

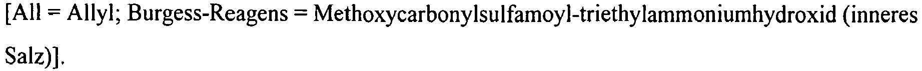

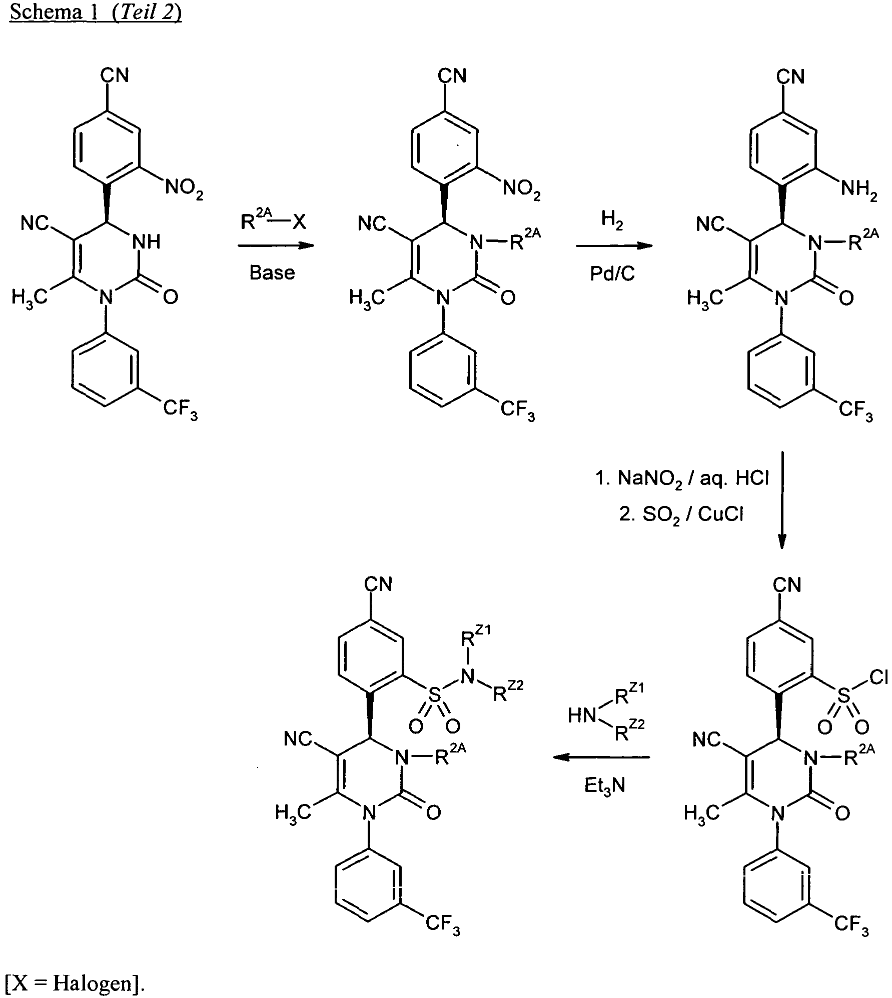

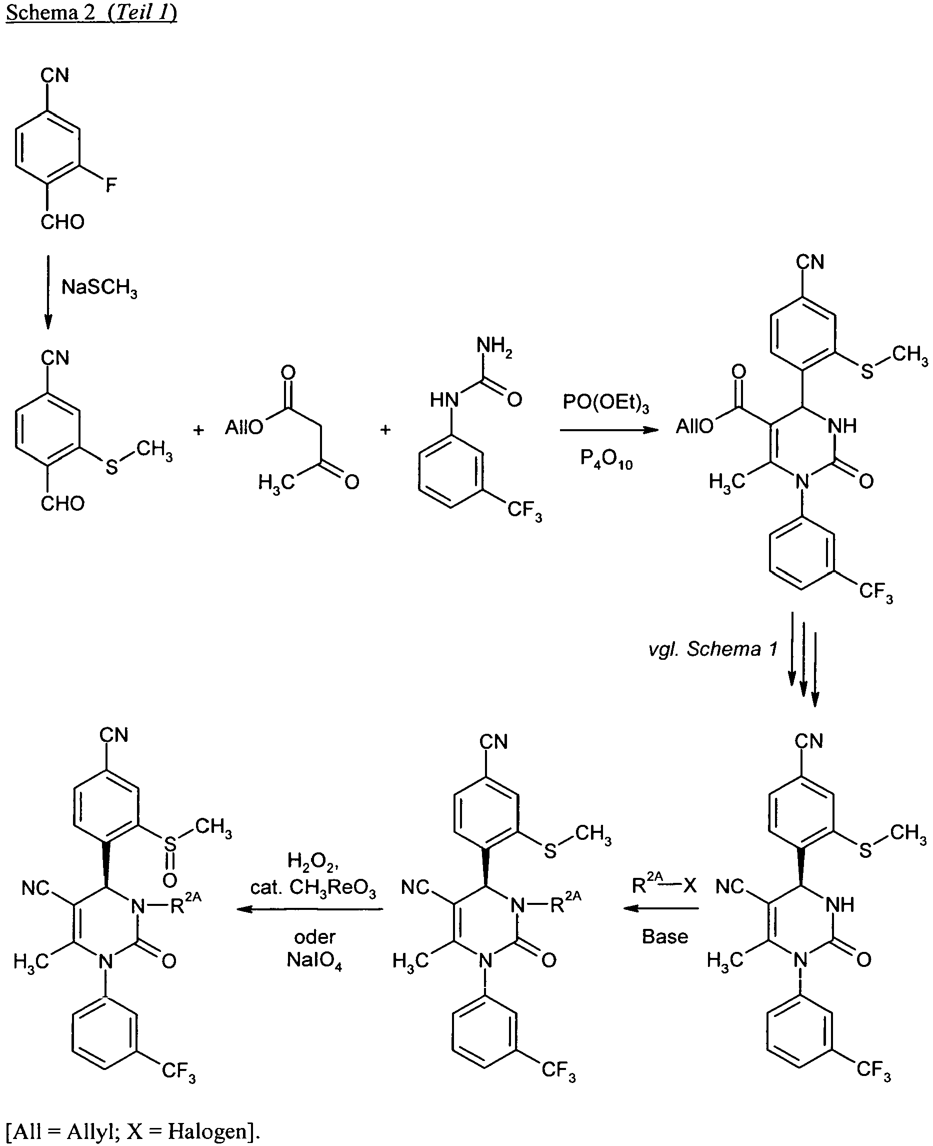

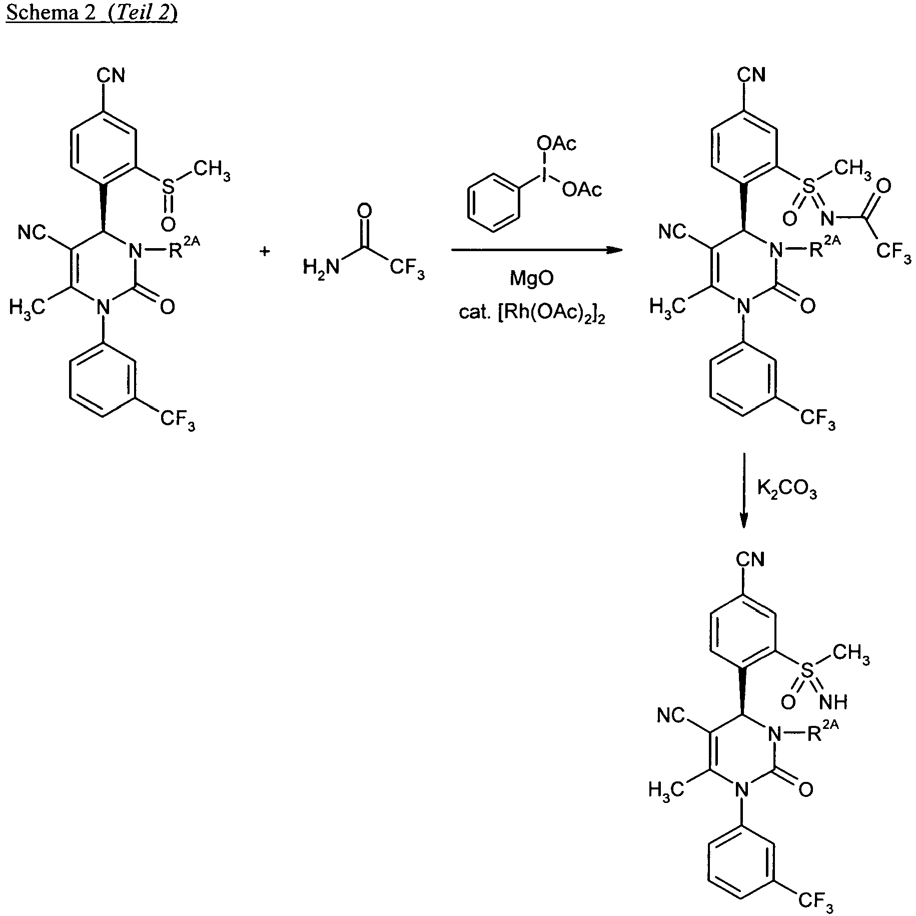

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen sind niedermolekulare, nicht-reaktive und selektive Inhibitoren der humanen neutrophilen Elastase, die überraschenderweise eine deutlich stärkere Inhibition dieser Protease im Vergleich zu den aus dem Stand der Technik bekannten Verbindungen bewirken. Darüber hinaus weisen die erfindungsgemäßen Verbindungen unerwartet eine niedrige *in vitro*-Clearance gegenüber Hepatozyten auf und verfügen damit über eine verbesserte metabolische Stabilität. Manche der erfindungsgemäßen Verbindungen besitzen zudem eine gute Löslichkeit in wässrigen Systemen, was vorteilhaft für ihre allgemeine Formulierbarkeit und/oder die intravenöse Anwendbarkeit ist.

Die erfindungsgemäßen Verbindungen eignen sich daher in besonderem Maße zur Behandlung und/oder Prävention von Erkrankungen und pathologischen Prozessen, insbesondere solchen, bei denen im Zuge eines Entzündungsgeschehens und/oder eines Gewebe- oder Gefäßumbaus die neutrophile Elastase (HNE) involviert ist.

Dazu zählen im Sinne der vorliegenden Erfindung insbesondere Erkrankungen wie die pulmonale arterielle Hypertonie (PAH) und andere Formen der pulmonalen Hypertonie (PH), die chronisch-obstruktive Lungenerkrankung (COPD), das akute Atemwegssyndrom (ARDS), akute Lungenschädigung (ALI), alpha-1-Antitrypsin-Defizienz (AATD), Lungenfibrose, Lungenemphysem (z.B. durch Zigarettenrauch induziertes Lungenemphysem), zystische Fibrose (CF), akutes Koronarsyndrom (ACS), Herzmuskelentzündungen (Myokarditis) und andere autoimmune Herzerkrankungen (Perikarditis, Endokarditis, Valvolitis, Aortitis, Kardiomyopathien), Myokardinfarkt, kardiogener Schock, Herzinsuffizienz, Aneurysmen, Sepsis (SIRS), multiples Organversagen (MODS, MOF), Arteriosklerose, entzündliche Erkrankungen der Niere, chronische Darmentzündungen (IBD, CD, UC), Pankreatitis, Peritonitis, rheumatoide Erkrankungen, entzündliche Hauterkrankungen sowie entzündliche Augenerkrankungen.

Die erfindungsgemäßen Verbindungen können weiterhin verwendet werden zur Behandlung und/ oder Prävention von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Darüber hinaus können die erfindungsgemäßen Verbindungen auch eingesetzt werden zur Behandlung und/oder Prävention von mikro- und makrovaskulären Schädigungen (Vasculitis), Reperfusionsschäden, arteriellen und venösen Thrombosen, Thrombosen im Zusammenhang mit orthopädischen Eingriffen bei Patienten mit rheumatoider Arthritis, von diabetischer und nicht-diabetischer Nephropathie, der Glomerulonephritis, der Glomerulosklerose, des nephrotischen Syndroms, der hypertensiven Nephrosklerose, der Mikroalbuminurie, der akuten und chronischen Niereninsuffizienz, des akuten und chronischen Nierenversagens, Cystitis, Urethritis, Prostatitis, Epidymititis, Oophoritis, Salpingitis, Vulvovaginitis, erektiler Dysfunktion, Hunner-Geschwür, Peyronie-Krankheit, arterieller Hypertonie, Schock, atrialen und ventrikulären Arrhythmien, transitorischen und ischämischen Attacken, Herzmuskelschwäche, Hirnschlag, endothelialer Dysfunktion, peripheren und kardialen Gefäßerkrankungen, peripheren Durchblutungsstörungen, Ödembildung wie zum Beispiel pulmonales Ödem, Hirnödem, renales Ödem und Herzinsuffizienz-bedingtes Ödem, Restenosen wie nach Thrombolysetherapien, percutan-transluminalen Angioplastien (PTA), transluminalen Koronarangioplastien (PTCA), Herztransplantationen und Bypass-Operationen, bei erhöhtem Spiegel von Fibrinogen und von LDL geringer Dichte sowie bei erhöhten Konzentrationen von Plasminogenaktivator-Inhibitor 1 (PAI-1), von Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien) sowie metabolischen Erkrankungen (Metabolisches Syndrom, Hyperglykämie, Insulin-abhängiger Diabetes, Nicht-Insulin-abhängiger Diabetes, Gestationsdiabetes, Hyperinsulinämie, Insulinresistenz, Glukose-Intoleranz, Fettsucht (Adipositas) und diabetische Spätfolgen wie Retinopathie, Nephropathie und Neuropathie), Krebserkrankungen (Hautkrebs, Hirntumore, Brustkrebs, Knochenmarktumore, Leukämien, Liposarcome, Karzinome des Magen-Darm-Traktes, der Leber, Bauchspeicheldrüse, Lunge, Niere, Harnleiter, Prostata und des Genitaltraktes sowie bösartige Tumore des lymphoproliferativen Systems wie z.B. Hodgkin's und Non-Hodgkin's Lymphom), von Erkrankungen des Gastrointestinaltraktes und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielfältige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres Eigenschaftsprofils eignen sich die erfindungsgemäßen Verbindungen insbesondere zur Behandlung und/oder Prävention von pulmonaler arterieller Hypertonie (PAH) und anderen Formen der pulmonalen Hypertonie (PH), chronisch-obstruktiven Lungenerkrankungen (COPD), akuter Lungenschädigung (ALI), akutem Atemwegssyndrom (ARDS), Bronchiektasien, Bronchiolitis obliterans, Lungenemphysem, alpha-1-Antitrypsin-Defizienz (AATD), zystischer Fibrose (CF), Sepsis und systemisch-inflammatorischem Response-Syndrom (SIRS), multiplem Organversagen (MOF, MODS), entzündlichen Darmerkrankungen (IBD, Morbus Crohn, Colitis), chronischer Bronchitis, Asthma, Rhinitis, rheumatoider Arthritis, entzündlichen Haut- und Augenkrankheiten, Arteriosklerose und Krebserkrankungen.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-HT_{2b}-Rezeptors;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- NO-unabhängige, jedoch Häm-abhängige Stimulatoren der löslichen Guanylatcyclase, wie insbesondere die in WO 00/06568, WO 00/06569, WO 02/42301 und WO 03/095451 beschriebenen Verbindungen;
- NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen;
- Prostacyclin-Analoga, wie beispielhaft und vorzugsweise Iloprost, Beraprost, Treprostinil oder Epoprostenol;
- Verbindungen, die die lösliche Epoxidhydrolase (sEH) inhibieren, wie beispielsweise *N,N*'-Di-cyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;
- den Energiestoffwechsel des Herzens beeinflussende Verbindungen, wie beispielhaft und vorzugsweise Etomoxir, Dichloracetat, Ranolazine oder Trimetazidine;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika;
- bronchodilatorisch wirkende Mittel, beispielhaft und vorzugsweise aus der Gruppe der betaadrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;
- anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason; und/ oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafarnib, Pegaptinib, Pelitinib, Semaxanib, Sorafenib, Sunitinib, Tandutinib, Tipifarnib, Vatalanib, Fasudil, Lonidamin, Leflunomid, BMS-3354825 oder Y-27632, eingesetzt.

Bei einer bevorzugten Ausführungsform der Erfindung werden die erfindungsgemäßen Verbindungen in Kombination mit einem Serotonin-Rezeptor-Antagonisten, wie beispielhaft und vorzugsweise PRX-08066, eingesetzt.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Aerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale, die intravenöse und die inhalative Applikation.

Die erfindungsgemäßen Verbindungen können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich, sofern nicht anders angegeben, jeweils auf das Volumen.

### A. Beispiele

### Abkürzungen:

- Ac: Acetyl
- aq.: wässrig, wässrige Lösung
- c: Konzentration
- cat.: katalytisch
- DC: Dünnschichtchromatographie
- DCI: direkte chemische Ionisation (bei MS)
- dest.: destilliert
- DIEA: *N,N*-Diisopropylethylamin
- DMAP: 4-*N,N*-Dimethylaminopyridin
- DMF: Dimethylformamid
- DMSO: Dimethylsulfoxid
- d. Th.: der Theorie (bei Ausbeute)
- ee: Enantiomerenüberschuss
- ent: enantiomerenrein, Enantiomer
- eq.: Äquivalent(e)
- ESI: Elektrospray-Ionisation (bei MS)
- Et: Ethyl
- GC-MS: Gaschromatographie-gekoppelte Massenspektrometrie
- h: Stunde(n)
- HATU: *O*-(7-Azabenzotriazol-1-yl)-*N,N,N;N'*-tetramethyluronium-Hexafluorophosphat
- HPLC: Hochdruck-, Hochieistungsflüssigchromatographie
- konz.: konzentriert
- LC-MS: Flüssigchromatographie-gekoppelte Massenspektrometrie
- Me: Methyl
- min: Minute(n)
- MPLC: Mitteldruckflüssigchromatographie
- MS: Massenspektrometrie
- MTBE: Methyl-*tert*.-butylether
- NMR: Kernresonanzspektrometrie
- Pd/C: Palladium auf Aktivkohle
- Ph: Phenyl
- PyBOP: Benzotriazol-1-yloxy-tris(pyrrolidino)phosphonium-hexafluorophosphat
- quant.: quantitativ (bei Ausbeute)
- rac: racemisch, Racemat
- RT: Raumtemperatur
- Rₜ: Retentionszeit (bei HPLC)
- Schmp.: Schmelzpunkt
- tBu: *tert*.-Butyl
- TFA: Trifluoressigsäure
- TFAA: Trifluoressigsäureanhydrid
- THF: Tetrahydrofuran
- UV: Ultraviolett-Spektrometrie
- vgl.: vergleiche
- v/v: Volumen zu Volumen-Verhältnis (einer Lösung)

### HPLC-, GC-MS- und LC-MS-Methoden:

### Methode 1 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 2 (analytische HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 9.0 min 90% B → 9.2 min 2% B → 10 min 2% B; Fluss: 0.75 ml/min; Säulentemperatur: 30°C; UV-Detektion: 210 nm.

### Methode 3 (analytische HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 4 (LC-MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 5 (LC-MS):

Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 6 (LC-MS):

Gerätetyp MS: Micromass ZQ; Gerätetyp HPLC: HP 1100 Series; UV DAD; Säule: Phenomenex Gemini 3µ 30 mm x 3.0 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 7 (LC-MS):

Gerätetyp MS: Waters ZQ; Gerätetyp HPLC: Waters Alliance 2795; Säule: Phenomenex Onyx Monolithic C18, 100 mm x 3 mm; Eluent A= 11 Wasser + 0.5 ml 50%-ige Ameisensäure, Eluent B; 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 8 (LC-MS):

Instrument: Waters Acquity SQD UPLC System; Säule: Waters Acquity UPLC HSS T3 1.8µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 9 (LC-MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 10 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 11 (LC-MS):

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1 % Ameisensäure, Eluent B: Acetonitril + 0.1% Ameisensäure; Gradient: 0.0 min 100% A → 0.2 min 95% A → 1.8 min 25% A → 1.9min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Ausgangsverbindungen und Intermediate:

### Beispiel 1A

### (rac)-Allyl 4-(4-cyano-2-nitrophenyl)-6-methyl-2-oxo-1-[3-(trifluormethyl)phenyl]-1,2,3,4-tetrahydropyrimidin-5-carboxylat

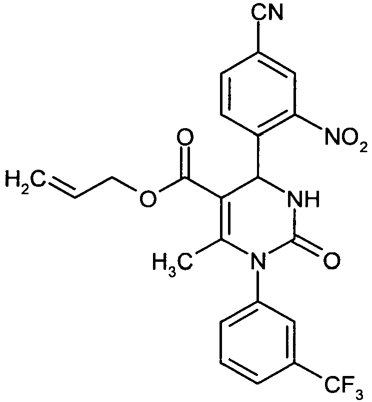

Die Reaktion wurde unter Argon durchgeführt. Allylacetoacetat (5.94 g, 41.5 mmol; 1.0 eq.) wurde in THF (117 ml) bei RT vorgelegt. Anschließend wurden 4-Cyano-2-nitrobenzaldehyd (10.45 g, 70% Reinheit, 41.5 mmol; 1.0 eq.), 1-[3-(Trifluormethyl)phenyl]harnstoff (8.48 g, 41.5 mmol) und Phosphorsäuretriethylester (17.7 g) zugegeben. Das Gemisch wurde 16 h unter Rückfluss gerührt. Zur Aufarbeitung wurde zunächst mit Eiswasser versetzt und dann in Essigsäureethylester (400 ml) aufgenommen. Die organische Phase wurde über festem Natriumsulfat getrocknet, filtriert und im Vakuum eingeengt. Das Rohprodukt wurde aus heißem Wasser/Isopropanol (2:1, ∼400 ml) umkristallisiert. Der erhaltene Feststoff wurde in Diethylether (60 mi) verrührt, erneut abgesaugt, mit wenig Diethylether nachgewaschen und im Hochvakuum getrocknet. Die Titelverbindung wurde als Feststoff erhalten (16.63 g, 82% d. Th.).
LC-MS (Methode 7): Rₜ = 3.70 min; MS (ESIpos): *m*/*z* (%) = 487.1 (100) [M+H]⁺
¹H-NMR (400 MHz, DMSO-d₆): δ = 2.10 (s, 3H), 4.40 (m, 2H), 4.95 (d, 1H), 5.05 (d, 1H), 5.70 (m, 1H), 6.15 (d, 1H), 6.05 (d, 1H), 7.70-7.90 (m, 4H), 8.10 (br. d, 1H), 8.25 (dd, 1H), 8.45 (d, 1H), 8.55 (d, 1H).

### Beispiel 2A

### (rac)-4-(4-Cyano-2-nitrophenyl)-6-methyl-2-oxo-1-[3-(trifluormethyl)phenyl]-1,2,3,4-tetrahydro-pyrimidin-5-carbonsäure

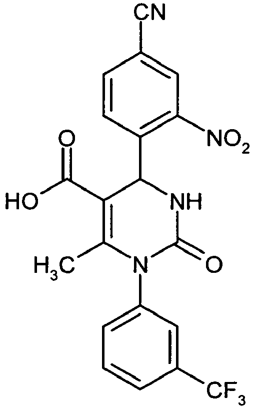

Die Reaktion wurde unter Argon durchgeführt. (*rac*)-Allyl 4-(4-cyano-2-nitrophenyl)-6-methyl-2-oxo-1-[3-(trifluormethyl)phenyl]-1,2,3,4-tetrahydropyrimidin-5-carboxylat (15.0 g, 30.8 mmol) und Morpholin (1.5 eq., 4.03 g, 46.3 mmol) wurden in trockenem THF (300 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(O) (0.05 eq., 1.78 g, 1.54 mmol) zugegeben und das Reaktionsgemisch 2 h bei RT gerührt (HPLC-Kontrolle). Das Gemisch wurde dann eingeengt und der Rückstand in Essigsäureethylester (700 ml) aufgenommen. Die organische Phase wurde mit 0.5 N Salzsäure (500 ml) und mit gesättigter Kochsalz-Lösung (300 ml) gewaschen, über Natriumsulfat getrocknet, filtriert und im Vakuum eingeengt. Das Rohprodukt wurde aus Essigsäureethylester umkristallisiert und im Hochvakuum getrocknet. Die Titelverbindung wurde als Feststoff erhalten (12.87 g, 93% d. Th.).
¹H-NMR (400 MHz, DMSO-d₆): δ = 2.05 (s, 3H), 6.00 (d, 1H), 7.65-7.90 (m, 4H), 8.10 (d, 1H), 8.25 (dd, 1H), 8.40 (d, 1H), 8.50 (d, 1H), 12.5 (br. s, 1H).

### Beispiel 3A

### (4R)-4-(4-Cyano-2-nitrophenyl)-6-methyl-2-oxo-1-[3-(trifluormethyl)phenyl]-1,2,3,4-tetrahydro-pyrimidin-5-carbonsäure

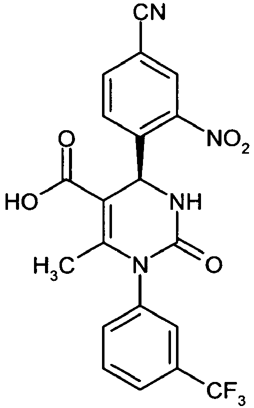

(*rac*)-4-(4-Cyano-2-nitrophenyl)-6-methyl-2-oxo-1-[3-(trifluormethyl)phenyl]-1,2,3,4-tetrahydro-pyrimidin-5-carbonsäure (590 g) wurde durch präparative HPLC-Chromatographie an chiraler Phase in die Enantiomere aufgetrennt [Säule: chirale Kieselgel-Phase basierend auf dem Selektor Poly(*N*-methacryloyl-L-leucin-*tert*.-butylamid); Säulendimension: 670 mm x 40 mm; Probenvorbereitung: 100 g Probe gelöst in 2000 ml THF; Injektionsvolumen: 70 ml; Eluent: Essigsäureethyl-ester/Methanol 100:1 → 1:100; Fluss: 80 ml/min; Temperatur: 24°C; Detektion: 260 nm]. Es wurden 280 g (95% d. Th.; 99.6% ee) des 4R-Enantiomeren erhalten.

Der Enantiomerenüberschuss (ee-Wert) wurde chromatographisch bestimmt [Säule: chirale Kieselgel-Phase basierend auf dem Selektor Poly(*N*-methacryloyl-L-leucin-*tert*.-butylamid); Säulendimension: 250 mm x 4.6 mm; Eluent: Essigsäureethylester/Methanol 10:1; Fluss: 2 ml/min; Detektion: 265 nm; Rₜ = 1.38 min].

### Beispiel 4A

### (4R)-4-(4-Cyano-2-nitrophenyl)-6-methyl-2-oxo-1-[3-(trifluormethyl)phenyl]-1,2,3,4-tetrahydro-pyrimidin-5-carboxamid

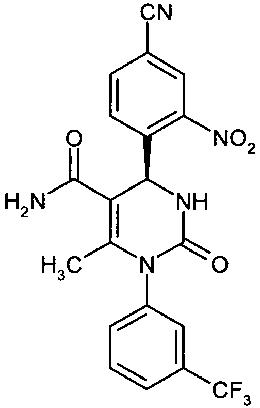

Die Reaktion wurde unter Argon durchgeführt. (4*R*)-4-(4-Cyano-2-nitrophenyl)-6-methyl-2-oxo-1-[3-(trifluormethyl)phenyl]-1,2,3,4-tetrahydropyrimidin-5-carbonsäure (6.0 g, 11.4 mmol, 85% Reinheit), DMAP (140 mg, 1.143 mmol; 0.1 eq.), DIEA (1.77 g, 13.7 mmol; 1.2 eq.) und PyBOP (7.14 g, 13.71 mmol; 1.2 eq.) wurden in trockenem THF (34 ml) bei RT vorgelegt, nach kurzem Rühren (15 min) mit 0.5 M Ammoniak-Lösung in THF (5 eq., 57.1 mmol) versetzt und anschließend 1 h bei RT gerührt. Das Reaktionsgemisch wurde dann mit Essigsäureethylester (250 ml) versetzt. Die organische Phase wurde nacheinander mit gesättigter Natriumhydrogencarbonat-Lösung, Wasser und gesättigter Kochsalz-Lösung gewaschen, über Natriumsulfat getrocknet, filtriert und im Vakuum eingeengt. Das Rohprodukt wurde an Kieselgel flash-chromatographiert (Laufmittel: Dichlormethan/Methanol 20: 1). Die Titelverbindung wurde als farbloser Feststoff erhalten (5.0 g, 98% d. Th.).
MS (ESIpos): *m*/*z* (%) = 446.2 (100) [M+H]⁺.

### Beispiel 5A

### (4R)-4-(4-Cyano-2-nitrophenyl)-6-methyl-2-oxo-1-[3-(trifluormethyl)phenyl]-1,2,3,4-tetrahydro-pyrimidin-5-carbonitril

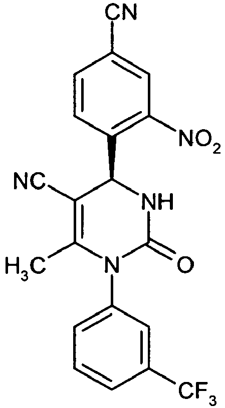

Die Reaktion wurde unter Argon durchgeführt. (4*R*)-4-(4-Cyano-2-nitrophenyl)-6-methyl-2-oxo-1-[3-(trifluormethyl)phenyl]-1,2,3,4-tetrahydropyrimidin-5-carboxamid (5.0g, 10.1 mmol; 90% Reinheit) wurde in trockenem THF (135 ml) vorgelegt, mit Methoxycarbonylsulfamoyl-triethylammoniumhydroxid (Burgess-Reagens; 3.85 g, 16.17 mmol; 1.6 eq.) versetzt und anschließend 2 h bei RT gerührt. Das Reaktionsgemisch wurde dann mit Essigsäureethylester (300 ml) versetzt. Die organische Phase wurde zweimal mit Wasser und einmal mit gesättigter Kochsalz-Lösung gewaschen, über Natriumsulfat getrocknet, filtriert und im Vakuum eingeengt. Das Rohprodukt wurde aus Cyclohexan/Essigsäureethylester umkristallisiert. Die erhaltenen Kristalle wurden im Hochvakuum getrocknet. Die Titelverbindung wurde als Feststoff erhalten (2.8 g, 65% d. Th.).
¹H-NMR (400 MHz, DMSO-d₆): δ = 1.80 (s, 3H), 5.95 (s, 1H), 7.75-8.25 (m, 6H), 8.35 (dd, 1H), 8.65 (s, 1H).

### Beispiel 6A

### (4R)-4-(4-Cyano-2-nitrophenyl)-3,6-dimethyl-2-oxo-1-[3-(trifluormethyl)phenyl]-1,2,3,4-tetra-hydropyrimidin-5-carbonitril

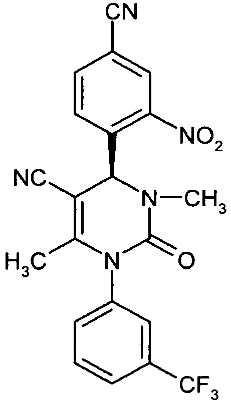

Die Reaktion wurde unter Argon durchgeführt. (4*R*)-4-(4-Cyano-2-nitrophenyl)-6-methyl-2-oxo-1-[3-(trifluormethyl)phenyl]-1,2,3,4-tetrahydropyrimidin-5-carbonitril (5.0 g, 11.7 mmol) wurde in absolutem THF (500 ml) vorgelegt und bei -78°C mit einer 1 M Lösung von Lithiumhexamethyldisilazid (LiHMDS) in THF (13.5 ml, 13.5 mmol; 1.15 eq.) versetzt. Nach 30 min Rühren wurde Iodmethan (8.30 g, 58.5 mmol; 5 eq.) in THF zugegeben und die Mischung für 16 h unter allmählicher Erwärmung von -78°C auf RT gerührt. Das Reaktionsgemisch wurde anschließend im Vakuum eingeengt und zunächst mit 1 N Salzsäure (14.0 ml), dann mit MTBE (500 ml) versetzt. Die organische Phase wurde nacheinander mit Wasser (2 x), gesättigter Natriumhydrogencarbonat-Lösung, gesättigter Ammoniumchlorid-Lösung und gesättigter Kochsalz-Lösung gewaschen, über Natriumsulfat getrocknet, filtriert und im Vakuum eingeengt. Die Titelverbindung wurde als Feststoff erhalten (4.3 g, 83% d. Th.).
LC-MS (Methode 4): Rₜ = 1.28 min; MS (ESIpos): *m*/*z* (%) = 442.2 (100) [M+H]⁺; MS (ESIneg): *m*/*z* (%) = 440.2 (50) [M-H]⁻
¹H-NMR (400 MHz, DMSO-d₆): δ = 1.80 (s, 3H), 2.70 (s, 3H), 5.95 (s, 1H), 7.75-8.25 (m, 5H), 8.35 (dd, 1H), 8.65 (s, 1H).

### Beispiel 7A

### (4R)-4-(2-Amino-4-cyanophenyl)-3,6-dimethyl-2-oxo-1-[3-(trifluormethyl)phenyl]-1,2,3,4-tetra-hydropyrimidin-5-carbonitril

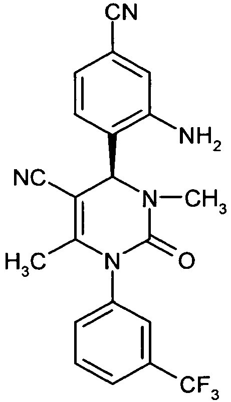

(4*R*)-4-(4-Cyano-2-nitrophenyl)-3,6-dimethyl-2-oxo-1-[3-(trifluormethyl)phenyl]-1,2,3,4-tetra-hydropyrimidin-5-carbonitril (6.0 g, 11.3 mmol) wurde unter Argon in Methanol (420 ml) gelöst. Anschließend wurde 10% Palladium auf Aktivkohle (5.5 g) zugegeben und für 5.5 h bei RT und Normaldruck hydriert (genaue HPLC-Kontrolle). Das Reaktionsgemisch wurde dann über Kieselgur filtriert und der Filter-Rückstand mit Methanol (1000 ml) nachgewaschen. Das Filtrat wurde eingeengt und das Rohprodukt an Kieselgel flash-chromatographiert (Laufmittel: Essigsäureethylester/Cyclohexan 2:1). Die Titelverbindung wurde als Feststoff erhalten (2.28 g, 40% d. Th.).
LC-MS (Methode 8): Rₜ = 1.06 min; MS (ESIpos): *m*/*z* (%) = 412.3 (80) [M+H]⁺; MS (ESIneg): *m*/*z* (%) = 410.3 (100) [M-H]⁻.

### Beispiel 8A

### 5-Cyano-2-{(4S)-5-cyano-3,6-dimethyl-2-oxo-1-[3-(trifluormethyl)phenyl]-1,2,3,4-tetrahydro-pyrimidin-4-yl}benzolsulfonylchlorid

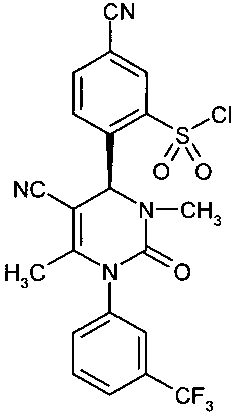

Unter Argon wurde (4*R*)-4-(2-Amino-4-cyanophenyl)-3,6-dimethyl-2-oxo-1-[3-(trifluormethyl)-phenyl]-1,2,3,4-tetrahydropyrimidin-5-carbonitril (2.1 g, 5.1 mmol) in einem 2:1:1-Gemisch aus Essigsäure/konz. Salzsäure/Wasser (insgesamt 50 ml) bei -10°C vorgelegt. Dazu wurde langsam eine Lösung von Natriumnitrit (371 mg, 5.38 mmol) in Wasser (2 ml) getropft und die Mischung für 40 min bei -10°C bis -5°C gerührt. Diese Lösung wurde dann zu 45 ml einer auf -10°C vorgekühlten, mit Schwefeldioxid gesättigten Suspension von Kupfer(I)chlorid (101.4 mg, 1.0 mmol) in Eisessig (44 ml) gegeben. Der Ansatz wurde ca. 30 min bei 0°C und dann 1 h bei +15°C gerührt (Reaktionskontrolle per HPLC und LC-MS). Die Reaktionsmischung wurde danach wieder auf 0°C gekühlt und anschließend pipettenweise in ca. 300 ml eiskaltes Wasser gegeben. Der Niederschlag wurde abfiltriert und in Essigsäureethylester (150 ml) aufgenommen. Die Lösung wurde zweimal mit gesättigter Kochsalz-Lösung gewaschen, über Natriumsulfat getrocknet, filtriert und im Vakuum eingeengt. Die Titelverbindung wurde als ein Feststoff erhalten (2.13 g, 77% d. Th., 92% Reinheit), welcher ohne weitere Aufreinigung in Folgereaktionen eingesetzt wurde.
LC-MS (Methode 4): Rₜ = 1.37 min; MS (ESIpos): m/z (%) = 495.1 (100) [M+H]⁺
¹H-NMR (400 MHz, DMSO-d₆): δ = 1.80 (s, 3H), 2.70 (s, 3H), 6.55 (s, 1H), 7.75-8.00 (m, 6H), 8.10 (s, 1H).

### Beispiel 9A

### (4R)-4-(2-Amino-4-cyanophenyl)-6-methyl-2-oxo-1-[3-(trifluormethyl)phenyl]-1,2,3,4-tetrahydro-pyrimidin-5-carbonitril

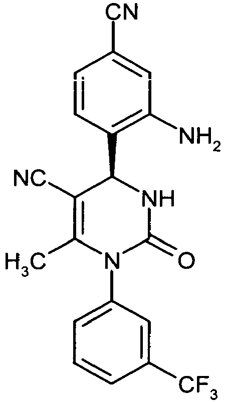

(4*R*)-4-(4-Cyano-2-nitrophenyl)-6-methyl-2-oxo-1-[3-(trifluormethyl)phenyl]-1,2,3,4-tetrahydro-pyrimidin-5-carbonitril (39.5 g, 92.4 mmol) wurde unter Argon in Ethanol (1975 ml) gelöst. Anschließend wurde 10% Palladium auf Aktivkohle (19.8 g) zugegeben und für 2 h bei RT und Normaldruck hydriert (genaue DC-Kontrolle). Das Reaktionsgemisch wurde dann über Kieselgur filtriert. Das Filtrat wurde eingeengt und das erhaltene Rohprodukt an Kieselgel flash-chromatographiert (Laufmittel: Essigsäureethylester/Cyclohexan 2:1). Die Titelverbindung wurde als Feststoff erhalten (25.5 g, 68% d. Th.).
LC-MS (Methode 10): Rₜ = 2.21 min; MS (ESIpos): *m*/*z* (%) = 398.2 (100) [M+H]⁺.

### Beispiel 10A

### 5-Cyano-2-{(4S)-5-cyano-6-methyl-2-oxo-1-[3-(trifluormethyl)phenyl]-1,2,3,4-tetrahydro-pyrimidin-4-yl}benzolsulfonylchlorid

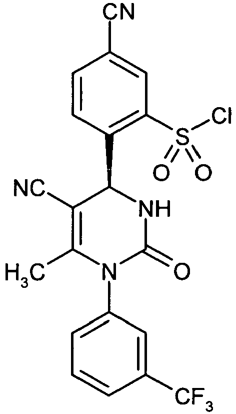

Unter Argon wurde (4*R*)-4-(2-Amino-4-cyanophenyl)-6-methyl-2-oxo-1-[3-(trifluormethyl)-phenyl]-1,2,3,4-tetrahydropyrimidin-5-carbonitril (3.0 g, 7.55 mmol) in einem 2:1:1-Gemisch aus Essigäure/konz. Salzsäure/Wasser (insgesamt 50 ml) bei -10°C vorgelegt. Dazu wurde eine Lösung von Natriumnitrit (547 mg, 7.93 mmol) in Wasser (6 ml) gegeben und die Mischung für 15 min bei -10°C gerührt. Diese Lösung wurde dann zu einer auf -10°C vorgekühlten, mit Schwefeldioxid gesättigten Suspension von Kupfer(I)chlorid (75 mg, 755 µmol; 0.1 eq.) in Eisessig (60 ml) gegeben. Der Ansatz wurde 60 min bei -10°C (Innentemperatur) gerührt und dann langsam innerhalb von 3 h auf +15°C erwärmt (Reaktionskontrolle per HPLC und LC-MS). Die Reaktionsmischung wurde danach wieder auf 0°C gekühlt und anschließend pipettenweise in ca. 300 ml eiskaltes Wasser gegeben. Die wässrige Phase wurde mehrfach mit MTBE extrahiert. Die vereinigten organischen Phasen wurden zweimal mit gesättigter Kochsalz-Lösung gewaschen, über Natriumsulfat getrocknet, filtriert und im Vakuum eingeengt. Die Titelverbindung wurde als ein Feststoff erhalten (1.67 g, 73% Reinheit laut LC-MS, 32% d. Th.) und ohne weitere Aufreinigung in Folgereaktionen eingesetzt.
LC-MS (Methode 6): Rₜ = 2.52 min; MS (ESIpos): *m*/*z* (%) = 481.0 (100) [M+H]⁺.

### Beispiel 11A

### 3-Fluor-4-formylbenzonitril

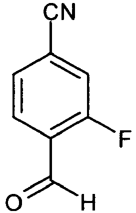

Die Reaktion wurde unter Argon durchgeführt. 3-Fluor-4-methylbenzonitril (121 g, 895 mmol) und *N,N*-Dimethylformamid-dimethylacetal (245 g, 2.06 mol) wurden in DMF (1.8 Liter) gelöst und über Nacht unter Rückfluss gerührt. Der Kolbeninhalt wurde danach auf Wasser (2 Liter) gegossen, und es wurde zweimal mit Essigsäureethylester extrahiert und die vereinigten organischen Phasen mit gesättigter Kochsalz-Lösung gewaschen. Die organische Phase wurde eingeengt und der Rückstand in THF/Wasser (1:1, 2.7 Liter) erneut gelöst. Es wurde mit Natriumperiodat (503 g, 2.35 mol) versetzt und eine Stunde bei Raumtemperatur gerührt. Dann wurde vom ausgefallenen Niederschlag abgetrennt, das Filtrat gewonnen und mehrfach mit Essigsäureethylester extrahiert. Die vereinigten organischen Phasen wurden einmal mit gesättigter Natriumhydrogencarbonat-Lösung und einmal mit gesättigter Natriumchlorid-Lösung gewaschen, getrocknet und zu einem Öl eingeengt. Dieses wurde durch Säulenchromatographie an Kieselgel gereinigt (Laufmittel: Petrol-ether/Dichlormethan 6:4, dann 4:6, zuletzt reines Dichlormethan). Die Produktfraktionen wurden eingeengt. Man erhielt 28.0 g (20% d. Th.) der Zielverbindung als weißen kristallinen Feststoff.
GC-MS (Methode 1): Rₜ = 3.63 min; MS (ESIpos): *m*/*z* (%) = 149.0 (48) [M]⁺, 150.0 (5) [M+H]⁺
¹H-NMR (400 MHz, DMSO-d₆): δ = 7.89 (d, 1H), 8.00 (t, 1H), 8.11 (d, 1 H), 10.24 (s, 1H).

### Beispiel 12A

### 4-Formyl-3-(methylsulfanyl)benzonitril

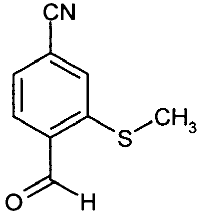

3-Fluor-4-formylbenzonitril (2.00 g, 13.4 mmol) wurde in DMSO (27 ml) gelöst und unter Eisbad-Kühlung mit Natriummethanthiolat (1.50 g, 21.5 mmol) versetzt. Es wurde 45 min nachgerührt und dann mit Wasser (100 ml) verdünnt. Das dabei ausfallende Produkt wurde abgesaugt, mit Wasser gewaschen und im Vakuum getrocknet. Man erhielt 1.36 g (51% d. Th.) der Zielverbindung als gelben kristallinen Feststoff.
GC-MS (Methode 1): Rₜ = 5.90 min; MS (ESIpos): *m*/*z* (%) = 177.0 (100) [M]⁺, 178.0 (11) [M+H]⁺.

### Beispiel 13A

### (rac)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure-allylester

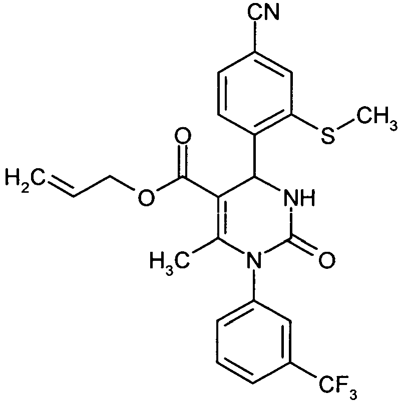

Die Reaktion wurde unter Argon durchgeführt. Phosphorsäuretriethylester (1.46 g, 8.04 mmol) und Diphosphorpentoxid (761 mg, 5.36 mmol) wurden über Nacht bei 50°C gerührt. Dann wurde mit MTBE (27 ml) verdünnt, und 4-Formyl-3-(methylsulfanyl)benzonitril (1.18 g, 6.70 mmol), 1-[3-(Trifluormethyl)phenyl]harnstoff (1.37 g, 6.70 mmol) sowie Allylacetoacetat (1.43 g, 10.1 mmol) wurden hinzugefügt. Das Gemisch wurde über Nacht unter Rückfluss gerührt. Zur Aufarbeitung wurde das Lösungsmittel im Vakuum entfernt und der Rückstand mit Diethylether aufgeschlämmt und dann abgesaugt. Man erhielt 978 mg (19% d. Th.) der Titelverbindung.
LC-MS (Methode 4): Rₜ = 1.37 min; MS (ESIpos): *m*/*z* (%) = 488.3 (100) [M+H]⁺; MS (ESIneg): *m*/*z* (%) = 486.2 (65) [M-H]⁻.

### Beispiel 14A

### (rac)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure

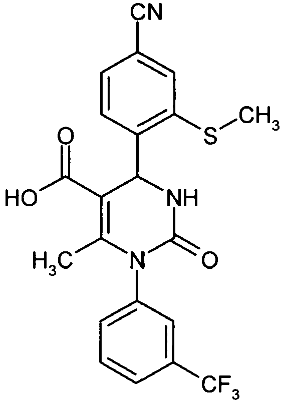

(*rac*)-4-[4-Cyano-2-(methylsulfanyl)phenyl]-6-methyl-2-oxo-l-[3-(trifluormethyl)phenyl]-1,2,3,4-tetrahydropyrimidin-5-carbonsäure-allylester (750 mg, 1.54 mmol) wurde in THF (10 ml) gelöst und mit Morpholin (201 mg, 2.308 mmol) versetzt. Die Reaktionslösung wurde mit Argon gesättigt (30 min Durchleiten von Argon). Anschließend wurde Tetrakis(triphenylphosphin)palladium(0) (7.47 mg, 0.006 mmol) zugegeben und die Mischung über Nacht bei RT gerührt. Da nach HPLC-Kontrolle nur wenig Umsatz zu beobachten war, wurde erneut Tetrakis(triphenylphosphin)-palladium(0) (7.47 mg), 0.006 mmol) zugesetzt und weitere 3 h bei RT gerührt. Der Koibeninhait wurde dann über Kieselgur filtriert und der Rückstand mit THF nachgewaschen. Das Filtrat wurde im Vakuum eingeengt und der Rückstand aus Diethylether (15 ml) umkristallisiert. Die Kristalle wurden abgesaugt und im Hochvakuum getrocknet. Es wurden 663 mg (96% d. Th.) der Zielverbindung erhalten.
LC-MS (Methode 4): Rₜ = 1.10 min; MS (ESIpos): *m*/*z* (%) = 448.0 (100) [M+H]⁺; MS (ESIneg): *m*/*z* (%) = 446.3 (100) [M-H]⁻.

### Beispiel 15A

### (4S)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure

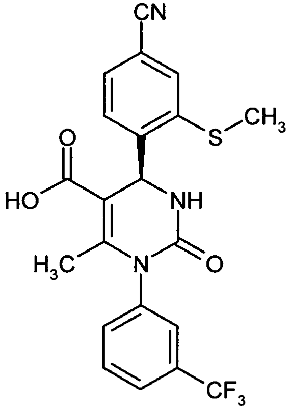

(*rac*)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure (663 mg, 1.48 mmol) wurde durch präparative HPLC-Chromatographie an chiraler Phase in die Enantiomere aufgetrennt [Säule: chirale Kieselgel-Phase basierend auf dem Selektor Poly(*N*-methacryloyl-D-leucin-dicyclopropylmethylamid); Säulendimension: 670 mm x 40 mm; Probenvorbereitung: Probe wurde in 20 ml Methanol/Ethylacetat 1:3 gelöst; Injektionsvolumen: 15 ml; Gradienten-Elution: Ethylacetat (100%) → Methanol (100%); Fluss: 80 ml/min; Temperatur: 25°C; Detektion: 260 nm]. Es wurden 279 mg (84% d. Th., 96% ee) des 4S-Enantiomeren als farbloser, amorpher Feststoff erhalten.
HPLC (Methode 2): Rₜ = 4.15 min.
MS (DCI / NH₃): *m*/*z* = 448.1 [M+H]⁺
¹H-NMR (400 MHz, DMSO-d₆): δ = 2.07 (s, 3H), 2.57 (s, 3H), 5.80 (d, 1H), 7.62-7.83 (m, 7H), 8.02 (d, 1H).
Drehwert: [α]²⁰_{Na} = +14.0° (*c* = 0.210 in DMF).

### Beispiel 16A

### (4S)-4-[4-Cyano-2-(methylsulfanyl)phenyl]-6-methyl-2-oxo-1-[3-(trifluormethyl)phenyl]-1,2,3,4-tetrahydropyrimidin-5-carboxamid

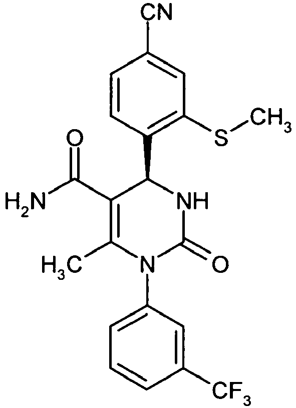

Die Reaktion wurde unter Argon durchgeführt. (4*S*)-4-[4-Cyano-2-(methylsulfanyl)phenyll-6-methyl-2-oxo-1-[3-(trifluorinethyl)phenyl]-1,2,3,4-tetrahydropyrimidin-5-carbonsäure (240 mg, 0.536 mmol) wurde in THF (5 ml) gelöst und mit PyBOP (419 mg, 0.805 mmol) und Triethylamin (380 mg, 3.76 mmol) versetzt. Nach kurzem Rühren wurde auf 0°C abgekühlt und mit Ammonium-chlorid (143 mg, 2.68 mmol) versetzt. Die Reaktionsmischung wurde über Nacht bei RT gerührt und der Kolbeninhalt dann in 1 N Salzsäure gegeben. Es wurde zweimal mit Essigsäureethylester extrahiert, und die vereinigten organischen Phasen wurden mit 1 N Salzsäure und mit gesättigter Kochsalz-Lösung gewaschen, getrocknet und eingeengt. Der Rückstand wurde durch präparative HPLC gereinigt. Man erhielt 161 mg (67% d. Th.) der Titelverbindung.
LC-MS (Methode 4): Rₜ = 0.99 min; MS (ESIpos): *m*/*z* (%) = 447.1 (100) [M+H]⁺; MS (ESIneg): *m*/*z* (%) = 445.3 (100) [M-H]⁻.

### Beispiel 17A

### (4S)-4-[4-Cyano-2-(methylsulfanyl)phenyl]-6-methyl-2-oxo-1-[3-(trifluormethyl)phenyl]-1,2,3,4-tetrahydropyrimidin-5-carbonitril

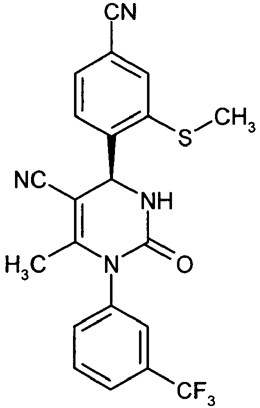

Die Reaktion wurde unter Argon durchgeführt. (4*S*)-4-[4-Cyano-2-(methylsulfanyl)phenyl]-6-methyl-2-oxo-1-[3-(trifluormethyl)phenyl]-1,2,3,4-tetrahydropyrimidin-5-carboxamid (95.0 mg, 0.213 mmol) wurde in THF (4 ml) gelöst und mit Methoxycarbonylsulfamoyl-triethylammoniumhydroxid (Burgess-Reagens; 101 mg, 0.426 mmol) versetzt. Nach 30 min Rühren bei Raumtemperatur zeigte die HPLC-Kontrolle vollständigen Umsatz an. Es wurde mit Essigsäureethylester (4 ml) verdünnt und mit Wasser (1 ml) versetzt. Die Mischung wurde dann über eine Merck Extrelut^{®} NT3-Säule gegeben und das Filtrat durch präparative HPLC gereinigt. Nach dem Einengen der Produktfraktionen erhielt man 96.0 mg (quant.) der Titelverbindung.
HPLC (Methode 3): Rₜ = 4.61 min.
MS (DCI / NH₃): *m*/*z* = 429.1 [M+H]⁺, 446.1 [M+NH₄]⁺
¹H-NMR (400 MHz, DMSO-d₆): δ = 1.80 (s, 3H), 2.61 (s, 3H), 5.76 (s, 1H), 7.67-7.89 (m, 7H), 8.28 (s, 1H).

### Beispiel 18A

### (R_{S}, 4S)-4-[4-Cyano-2-(methylsulfinyl)phenyl]-6-methyl-2-oxo-1-[3-(trifluormethyl)phenyl]-1,2,3,4-tetrahydropyrimidin-5-carbonitril und (S_{S}, 4S)-4-[4-Cyano-2-(methylsulfinyl)phenyl]-6-methyl-2-oxo-1-[3-(trifluormethyl)phenyl]-1,2,3,4-tetrahydropyrimidin-5-carbonitril (Diastereomerengemisch)

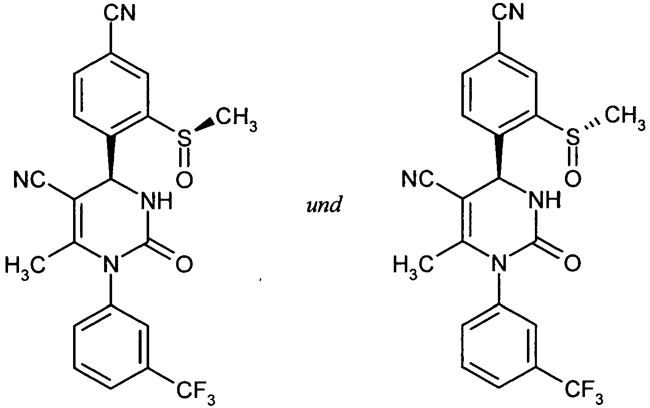

### Methode A:

(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonitril (55 mg, 0.13 mmol) wurde in Ethanol (5.5 ml) gelöst und mit Methyltrioxorhenium (3.20 mg, 0.013 mmol) sowie Wasserstoffperoxid (16.0 mg, 0.14 mmol) versetzt. Das Reaktionsgemisch wurde 60 min bei RT gerührt, dann im Vakuum eingeengt und der Rückstand mittels präparativer HPLC gereinigt. Es wurden 27 mg (47% d. Th.) der Zielverbindung als Diastereomerengemisch erhalten.
LC-MS (Methode 4): Rₜ = 1.05 min; MS (ESIpos): *m*/*z* (%) = 445.0 (100) [M+H]⁺.

### Methode B:

(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonitril (2.00 g, 4.67 mmol) wurde in Methanol/Wasser (4.4:1, ∼40 ml) vorgelegt, mit Natriumperiodat (1.90 g, 8.87 mmol; 1.9 eq.) versetzt und 16 h bei 30°C gerührt. Anschließend wurde erneut Natriumperiodat (0.45 g, 2.10 mmol; 0.45 eq.) hinzugefügt und der Ansatz weitere 4 h bei 50°C gerührt (HPLC-Kontrolle). Das Reaktionsgemisch wurde danach auf gesättigte wässrige Natriumhydrogencarbonat-Lösung (∼200 ml) gegeben und mit Essigsäureethyl-ester (4 x 50 ml) extrahiert. Die vereinigten organischen Phasen wurden über Natriumsulfat getrocknet, filtriert und im Vakuum eingeengt. Das Rohprodukt wurde an Kieselgel flash-chromatographiert (Gradient Cyclohexan → Essigsäureethylester). Die Zielverbindung wurde als Diastereomerengemisch in Form eines farblosen Feststoffs erhalten (2.18 g, quant.).
LC-MS (Methode 9): Rₜ = 1.98 min; MS (ESIpos): *m*/*z* (%) = 402.0 (100), 445.0 (60) [M+H]⁺; MS (ESIneg): *m*/*z* (%) = 400.1 (100), 443.1 (40) [M-H]⁻.
¹H-NMR (400 MHz, DMSO-d₆): δ = 1.85 (2 s, 3H), 2.85 (2 s, 3H), 5.75 (2 s, 1H), 7.70-8.50 (m, 8H).

### Beispiel 19A

### (R_{S}, 4S)-4-[4-Cyano-2-(methylsulfinyl)phenyl]-3,6-dimethyl-2-oxo-1-[3-(trifluormethyl)phenyl]-1,2,3,4-tetrahydropyrimidin-5-carbonitril und (S_{S}, 4S)-4-[4-Cyano-2-(methylsulfinyl)phenyl]-3,6-dimethyl-2-oxo-1-[3-(trifluormethyl)phenyl]-1,2,3,4-tetrahydropyrimidin-5-carbonitril (Diastereomerengemisch)

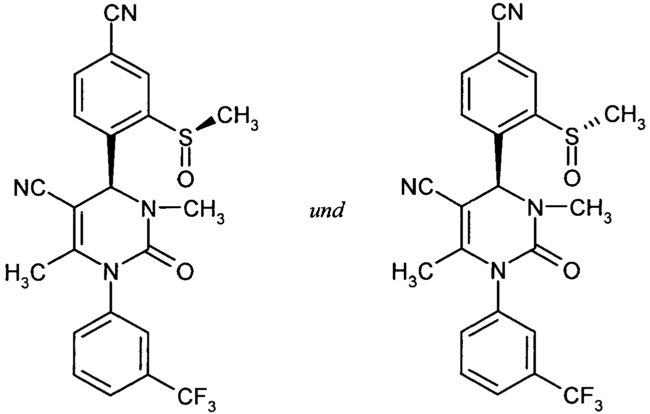

Die Reaktion wurde unter Argon durchgeführt. Das Diastereomerengemisch von (*R_{S},* 4*S*)-4-[4-Cyano-2-(methylsulfinyl)phenyl]-6-methyl-2-oxo-1-[3-(trifluormethyl)phenyl]-1,2,3,4-tetrahydro-pyrimidin-5-carbonitril und (*S_{S}*, 4*S*)-4-[4-Cyano-2-(methylsulfinyl)phenyl]-6-methyl-2-oxo-1-[3-(trifluormethyl)phenyl]-1,2,3,4-tetrahydropyrimidin-5-carbonitril (535 mg, 1.2 mmol) wurde in absolutem THF (12 ml) vorgelegt und bei -78°C mit einer 1 M Lösung von Lithiumhexamethyldisilazid (LiHMDS) in THF (1.45 ml; 1.2 eq.) versetzt. Nach 20 min Rühren bei -78°C wurde Iodmethan (854 mg; 5 eq.) zugegeben und die Mischung für 16 h unter allmählicher Erwärmung von -78°C auf RT gerührt. Das Reaktionsgemisch wurde dann im Vakuum eingeengt, mit gesättigter Ammoniumchlorid-Lösung (50 ml) versetzt und anschließend mit Essigsäureethylester (3 x 30 ml) extrahiert. Die vereinigten organischen Phasen wurden über festem Natriumsulfat getrocknet, filtriert, im Vakuum eingeengt und der Rückstand mittels präparativer HPLC gereinigt (Säule: Gromsil C-18 10 µm; Eluent: Acetonitril/Wasser + 0.1 % TFA 10:90 → 90:10). Man erhielt die Titelverbindung als einen Feststoff (488 mg, 88% d. Th.).
LC-MS (Methode 6): Rₜ = 2.12 min; MS (ESIpos): *m*/*z* (%) = 459.0 (100) [M+H]⁺; MS (ESIneg): *m*/*z* (%) = 456.9 (100) [M-H]⁻.
¹H-NMR (400 MHz, DMSO-d₆): δ = 1.80 (2 s, 3H), 2.65 (2 s, 3H), 2.90 (2 s, 3H), 5.80 (2 s, 1H), 7.70-8.20 (m, 6H), 8.45 (2 s, 1H).

### Beispiel 20A

### (R_{S})-N-[(5-Cyano-2-{(4S)-5-cyano-3,6-dimethyl-2-oxo-1-[3-(trifluormethyl)phenyl]-1,2,3,4-tetra-hydropyrimidin-4-yl}phenyl)(methyl)oxido-λ⁶-sulfanyliden]-2,2,2-trifluoracetamid und (S_{S})-N-[(5-Cyano-2-{(4S)-5-cyano-3,6-dimethyl-2-oxo-1-[3-(trifluormethyl)phenyl]-1,2,3,4-tetra-hydropyrimidin-4-yl}phenyl)(methyl)oxido-λ⁶-sulfanyliden]-2,2,2-trifluoracetamid (Diastereomerengemisch)

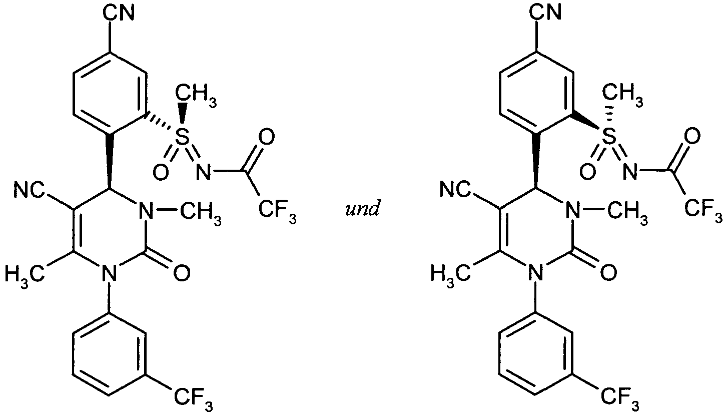

Die Reaktion wurde unter Argon durchgeführt. Das Diastereomerengemisch von (*R_{S}*, 4*S*)-4-[4-Cyano-2-(methylsulfinyl)phenyl]-3,6-dimethyl-2-oxo-1-[3-(trifluormethyl)phenyl]-1,2,3,4-tetra-hydropyrimidin-5-carbonitril und (*S_{S}*, 4*S*)-4-[4-Cyano-2-(methylsulfinyl)phenyl]-3,6-dimethyl-2-oxo-1-[3-(trifluormethyl)phenyl]-1,2,3,4-tetrahydropyrimidin-5-carbonitril (488 mg, 1.1 mmol) wurde in Dichlormethan (10 ml) vorgelegt und nacheinander mit 2,2,2-Trifluoracetamid (241 mg, 2.13 mmol; 2.0 eq.), Magnesiumoxid (172 mg, 4.26 mmol; 4.0 eq.), Rhodium(II)acetat-Dimer (24 mg, 53 µmol; 0.05 eq.) sowie (Diacetoxyiod)-benzol (514 mg, 1.60 mmol; 1.5 eq.) versetzt. Die Mischung wurde 16 h bei Raumtemperatur gerührt. Anschließend wurden erneut 2,2,2-Trifluoracetamid (120 mg, 1.06 mmol; 1.0 eq.), Magnesiumoxid (86 mg, 2.13 mmol; 2.0 eq.), Rhodium(II)acetat-Dimer (12 mg, 27 µmol; 0.025 eq.) sowie (Diacetoxyiod)-benzol (257 mg, 798 µmol; 0.75 eq.) zugesetzt und weitere 24 h bei Raumtemperatur gerührt. Das Reaktionsgemisch wurde dann über Kieselgur filtriert, das Filtrat im Vakuum eingeengt und der Rückstand mittels präparativer HPLC gereinigt (Säule: Gromsil C-18 10 µm; Eluent: Acetonitril/Wasser + 0.1% TFA 10:90 → 80:20). Man erhielt die Titelverbindung als Diastereomerengemisch in Form eines Feststoffs (160 mg, 25% d. Th.).
LC-MS (Methode 4): Rₜ = 1.35 min und 1.37 min; MS (ESIpos): *m*/*z* (%) = 570.1 (100) [M+H]⁺; MS (ESIneg): *m*/*z* (%) = 567.9 (100) [M-H]⁻.

### Beispiel 21A

### (R_{S})-N-[(5-Cyano-2-{(4S)-5-cyano-3,6-dimethyl-2-oxo-1-[3-(trifluormethyl)phenyl]-1,2,3,4-tetra-hydropyrimidin-4-yl}phenyl)(methyl)oxido-λ⁶-sulfanyliden]-2,2,2-trifluoracetamid oder (S_{S})-N-[(5-Cyano-2-{(4S)-5-cyano-3,6-dimethyl-2-oxo-1-[3-(trifluormethyl)phenyl]-1,2,3,4-tetra-hydropyrimidin-4-yl}phenyl)(methyl)oxido-λ⁶-sulfanyliden]-2,2,2-trifluoracetamid (Diastereomer 1)

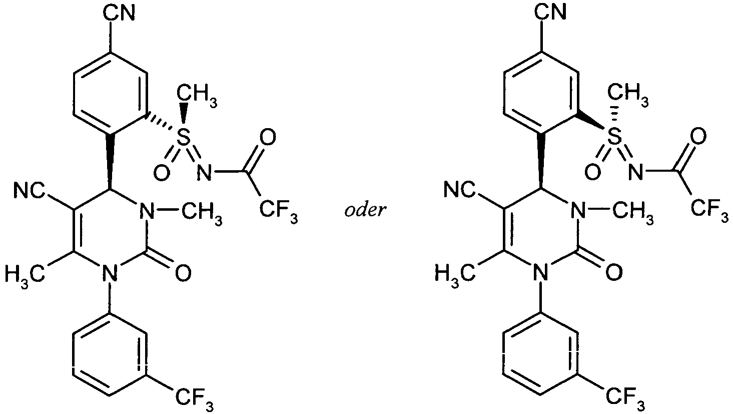

Das Diastereomerengemisch von (*R_{S}*)-*N*-[(5-Cyano-2-{(4*S*)-5-cyano-3,6-dimethyl-2-oxo-1-[3-(trifluormethyl)phenyl]-1,2,3,4-tetrahydropyrimidin-4-yl}phenyl)(methyl)oxido-λ⁶-sulfanyliden]-2,2,2-trifluoracetamid und (*S_{S}*)-*N*-[(5-Cyano-2-{(4*S*)-5-cyano-3,6-dimethyl-2-oxo-1-[3-(trifluormethyl)phenyl]-1,2,3,4-tetrahydropyrimidin-4-yl}phenyl)(methyl)oxido-λ⁶-sulfanyliden]-2,2,2-trifluoracetamid (160 mg) wurde durch Flash-Chromatographie an Kieselgel aufgetrennt (Laufmittel-Gradient Cyclohexan → Cyclohexan/Essigsäureethylester 45:55). Das Diastereomer 1 wurde als früher eluierende Fraktion erhalten (Ausbeute: 52 mg).
LC-MS (Methode 4): Rₜ = 1.38 min; MS (ESIpos): *m*/*z* (%) = 570.1 (100) [M+H]⁺; MS (ESIneg): *m*/*z* (%) = 568.3 (100) [M-H]⁻.

### Beispiel 22A

### (S_{S})-N-[(5-Cyano-2-{(4S)-5-cyano-3,6-dimethyl-2-oxo-1-[3-(trifluormethyl)phenyl]-1,2,3,4-tetra-hydropyrimidin-4-yl}phenyl)(methyl)oxido-λ⁶-sulfanyliden]-2,2,2-trifluoracetamid oder (R_{S})-N-[(5-Cyano-2-{(4S)-5-cyano-3,6-dimethyl-2-oxo-1-[3-(trifluormethyl)phenyl]-1,2,3,4-tetra-hydropyrimidin-4-yl}phenyl)(methyl)oxido-λ⁶-sulfanyliden]-2,2,2-trifluoracetamid (Diastereomer 2)

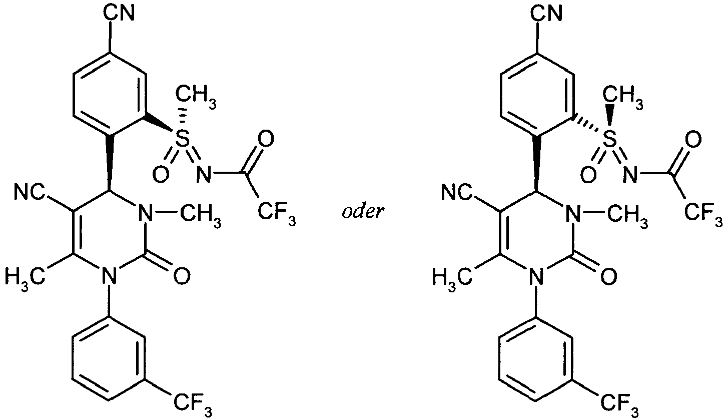

Das Diastereomerengemisch von (*R_{S}*)-*N*-[(5-Cyano-2-{(4*S*)-5-cyano-3,6-dimethyl-2-oxo-1-[3-(trifluormethyl)phenyl]-1,2,3,4-tetrahydropyrimidin-4-yl}phenyl)(methyl)oxido-λ⁶-sulfanyliden]-2,2,2-trifluoracetamid und (*S_{S}*)-*N*-[(5-Cyano-2-{(4*S*)-5-cyano-3,6-dimethyl-2-oxo-1-[3-(trifluormethyl)phenyl]-1,2,3,4-tetrahydropyrimidin-4-yl}phenyl)(methyl)oxido-λ⁶-sulfanyliden]-2,2,2-trifluoracetamid (160 mg) wurde durch Flash-Chromatographie an Kieselgel aufgetrennt (Laufmittel-Gradient Cyclohexan → Cyclohexan/Essigsäureethylester 45:55). Das Diastereomer 2 wurde als später eluierende Fraktion erhalten (Ausbeute: 68 mg).

LC-MS (Methode 4): Rₜ = 1.35 min; MS (ESIpos): *m*/*z* (%) = 570.1 (100) [M+H]⁺; MS (ESIneg): *m*/*z* (%) = 568.4 (100) [M-H]⁻.

### Beispiel 23A

### (R_{S})-N-[(5-Cyano-2-{(4S)-5-cyano-6-methyl-2-oxo-1-[3-(trifluormethyl)phenyl]-1,2,3,4-tetrahydro-pyrimidin-4-yl}phenyl)(methyl)oxido-λ⁶-sulfanyliden]-2,2,2-trifluoracetamid und (S_{S})-N-[(5-Cyano-2-{(4S)-5-cyano-6-methyl-2-oxo-1-[3-(trifluormethyl)phenyl]-1,2,3,4-tetrahydro-pyrimidin-4-yl}phenyl)(methyl)oxido-λ⁶-sulfanyliden]-2,2,2-trifluoracetamid (Diastereomerengemisch)

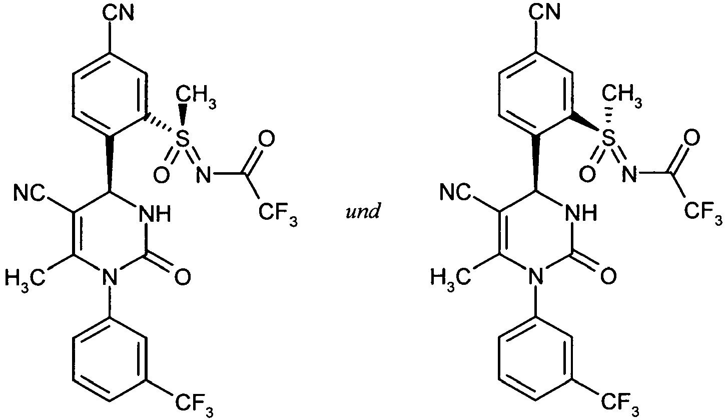

Die Reaktion wurde unter Argon durchgeführt. Das Diastereomerengemisch von (*R_{S}*, 4*S*)-4-[4-Cyano-2-(methylsulfinyl)phenyl]-6-methyl-2-oxo-1-[3-(trifluormethyl)phenyl]-1,2,3,4-tetrahydro-pyrimidin-5-carbonitril und (*S_{S}*, 4*S*)-4-[4-Cyano-2-(methylsulfinyl)phenyl]-6-methyl-2-oxo-1-[3-(trifluormethyl)phenyl]-1,2,3,4-tetrahydropyrimidin-5-carbonitril (600 mg, 1.35 mmol) wurde in Dichlormethan (13.5 ml) vorgelegt und nacheinander mit 2,2,2-Trifluoracetamid (305 mg, 2.7 mmol; 2.0 eq.), Magnesiumoxid (217 mg, 5.4 mmol; 4.0 eq.), Rhodium(II)acetat-Dimer (29.8 mg, 68 µmol; 0.05 eq.) sowie (Diacetoxyiod)-benzol (652 mg, 2.03 mmol; 1.5 eq.) versetzt. Die Mischung wurde 16 h bei Raumtemperatur gerührt. Anschließend wurden erneut 2,2,2-Trifluoracetamid (152.6 mg, 1.35 mmol; 1.0 eq.), Magnesiumoxid (109 mg, 2.7 mmol; 2.0 eq.), Rhodium(II)acetat-Dimer (15 mg, 34 µmol; 0.025 eq.) sowie (Diacetoxyiod)-benzol (326 mg, 1013 µmol; 0.75 eq.) zugesetzt und weitere 3 h bei Raumtemperatur gerührt. Das Reaktionsgemisch wurde dann über Kieselgur filtriert, das Filtrat im Vakuum eingeengt und der Rückstand an Kieselgel flash-chromatographiert (Gradient Cyclohexan → Essigsäureethylester). Man erhielt die Titelverbindung als Diastereomerengemisch in Form eines Feststoffs (485 mg, 65% d. Th.).
LC-MS (Methode 4): Rₜ = 1.28 min; MS (ESIpos): *m*/*z* (%) = 556.0 (100) [M+H]⁺.
¹H-NMR (400 MHz, DMSO-d₆): δ = 1.85 (2 s, 3H), 4.00 (2 s, 3H), 6.50 (2 s, 1H), 7.70-8.55 (m, 8H).

### Beispiel 24A

### (R_{S}, 4S)-4-[4-Cyano-2-(methylsulfinyl)phenyl]-6-methyl-3-(methylsulfonyl)-2-oxo-1-[3-(trifluor-methyl)phenyl]-1,2,3,4-tetrahydropyrimidin-5-carbonitril und (S_{S}, 4S)-4-[4-Cyano-2-(methylsulfinyl)phenyl]-6-methyl-3-(methylsulfonyl)-2-oxo-1-[3-(trifluormethyl)phenyl]-1,2,3,4-tetrahydropyrimidin-5-carbonitril (Diastereomerengemisch)

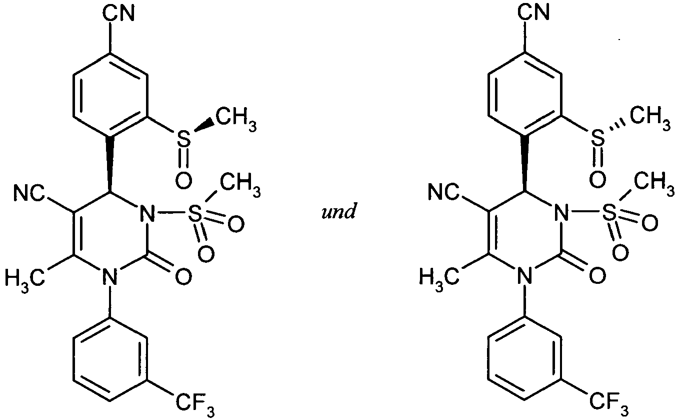

Die Reaktion wurde unter Argon durchgeführt. Das Diastereomerengemisch von (*R_{S}*, 4*S*)-4-[4-Cyano-2-(methylsulfinyl)phenyl]-6-methyl-2-oxo-1-[3-(trifluormethyl)phenyl]-1,2,3,4-tetrahydro-pyrimidin-5-carbonitril und (*S_{S}*, 4*S*)-4-[4-Cyano-2-(methylsulfinyl)phenyl]-6-methyl-2-oxo-1-[3-(trifluormethyl)phenyl]-1,2,3,4-tetrahydropyrimidin-5-carbonitril (444.4 mg, 1000 µmol) wurde in THF (10 ml) vorgelegt und bei 0°C mit Natriumhydrid (60% in Mineralöl; 56 mg, 1400 µmol) versetzt. Es wurde auf RT erwärmt und 20 min gerührt. Anschließend wurde eine Lösung von Methansulfonylchlorid (160.4 mg, 1400 µmol; 1.4 eq.) in THF (5 ml) langsam zugetropft. Nach 16 h Reaktionszeit wurde nochmals Methansulfonylchlorid (54 mg, 467 µmol; 0.47 eq.) zugesetzt und erneut für 60 min bei RT gerührt. Das Reaktionsgemisch wurde dann mit gesättigter Ammoniumchlorid-Lösung (50 ml) versetzt und anschließend mit Essigsäureethylester (3 x 30 ml) extrahiert. Die vereinigten organischen Phasen wurden über festem Natriumsulfat getrocknet, filtriert und im Vakuum eingeengt. Das Rohprodukt wurde mittels präparativer HPLC aufgereinigt (Säule: Gromsil C-18 10 µm; Eluent: Acetonitril/Wasser + 0.1% TFA 10:90 → 80:20). Man erhielt die Titelverbindung als einen farblosen Feststoff (245 mg, 47% d. Th.).
LC-MS (Methode 6): Rₜ = 2.20 min; MS (ESIpos): *m*/*z* (%) = 522.9 (100) [M+H]⁺; MS (ESIneg): *m*/*z* (%) = 440.9 (100), 520.9 (100) [M-H]⁻.
¹H-NMR (400 MHz, DMSO-d₆): δ = 1.80 (2 s, 3H), 2.90 (2 s, 3H), ∼3.40 (2 s, 3H), 6.40 (2 s, 1H), 7.75-8.20 (m, 6H), 8.50 (2 s, 1 H).

### Beispiel 25A

### (R_{S})-N-[(5-Cyano-2-{(4S)-5-cyano-6-methyl-3-(methylsulfonyl)-2-oxo-1-[3-(trifluormethyl)-phenyl]-1,2,3,4-tetrahydropyrimidin-4-yl}phenyl)(methyl)oxido-λ⁶-sulfanyliden]-2,2,2-trifluoracetamid und (S_{S})-N-[(5-Cyano-2-{(4S)-5-cyano-6-methyl-3-(methylsulfonyl)-2-oxo-1-[3-(trifluormethyl)-phenyl]-1,2,3,4-tetrahydropyrimidin-4-yl}phenyl)(methyl)oxido-λ⁶-sulfanyliden]-2,2,2-trifluoracetamid (Diastereomerengemisch)

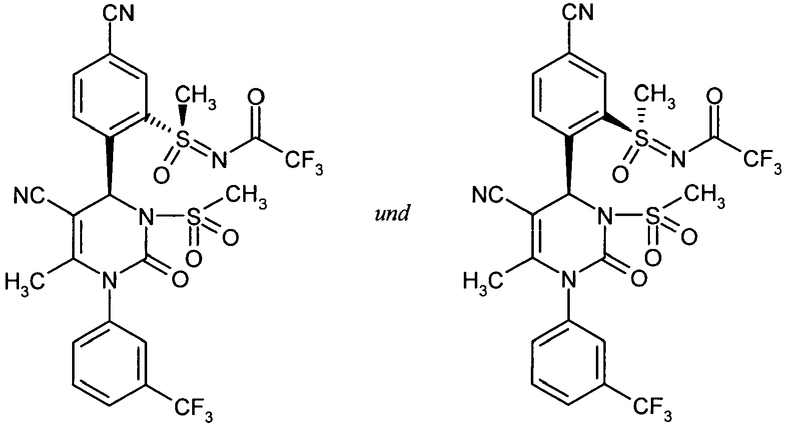

Die Reaktion wurde unter Argon durchgeführt. Das Diastereomerengemisch von (*R_{S}*, 4*S*)-4-[4-Cyano-2-(methylsulfinyl)phenyl]-6-methyl-3-(methylsulfonyl)-2-oxo-1-[3-(trifluormethyl)phenyl]-1,2,3,4-tetrahydropyrimidin-5-carbonitril und (*S_{S}*, 4*S*)-4-[4-Cyano-2-(methylsulfinyl)phenyl]-6-methyl-3-(methylsulfonyl)-2-oxo-1-[3-(trifluormethyl)phenyl]-1,2,3,4-tetrahydropyrimidin-5-carbonitril (160 mg, 0.306 mmol) wurde in Dichlormethan (3 ml) vorgelegt und nacheinander mit 2,2,2-Trifluoracetamid (69 mg, 0.612 mmol; 2.0 eq.), Magnesiumoxid (49.4 mg, 1.225 mmol; 4.0 eq.), Rhodium(II)acetat-Dimer (6.8 mg, 15 µmol; 0.05 eq.) sowie (Diacetoxyiod)-benzol (147.9 mg, 0.459 mmol; 1.5 eq.) versetzt. Die Mischung wurde 16 h bei Raumtemperatur gerührt. Anschließend wurden erneut 2,2,2-Trifluoracetamid (34.6 mg, 0.306 mmol; 1.0 eq.), Magnesiumoxid (24.7 mg, 0.612 mmol; 2.0 eq.), Rhodium(II)acetat-Dimer (3.4 mg, 8 µmol; 0.025 eq.) sowie (Diacetoxyiod)-benzol (74 mg, 230 µmol; 0.75 eq.) zugesetzt und weitere 24 h bei Raumtemperatur gerührt. Das Reaktionsgemisch wurde dann über Kieselgur filtriert, das Filtrat im Vakuum eingeengt und der Rückstand an Kieselgel flash-chromatographiert (Gradient Cyclohexan → Cyclohexan/Essigsäureethylester 1:2 → Essigsäureethylester). Man erhielt die Titelverbindung als Diastereomerengemisch in Form eines Feststoffs (25 mg, 8% d. Th., 61% Reinheit). Dieses Produkt wurde ohne weitere Aufreinigung in der Folgereaktion eingesetzt.
LC-MS (Methode 5): Rₜ = 2.27 min; MS (ESIpos): *m*/*z* (%) = 634.1 (100) [M+H]⁺; MS (ESIneg): *m*/*z* (%) = 632.1 (100) [M-H]⁻.

### Ausführungsbeispiele:

### Beispiel 1

### 5-Cyano-2-{(4S)-5-cyano-6-methyl-2-oxo-1-[3-(trifluormethyl)phenyl]-1,2,3,4-tetrahydro-pyrimidin-4-yl}benzolsulfonamid

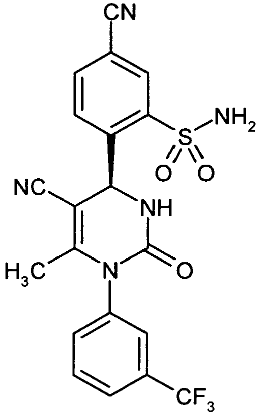

5-Cyano-2-{(4*S*)-5-cyano-6-methyl-2-oxo-1-[3-(trifluormethyl)phenyl]-1,2,3,4-tetrahydropyrimidin-4-yl}benzolsulfonylchlorid (3.10 g, Gehalt 20%, 1.29 mmol) wurde bei Raumtemperatur mit 0.5 M Ammoniak-Lösung in Dioxan (25.79 ml, 12.9 mmol; 10 eq.) sowie Triethylamin (130 mg, 1.3 mmol; 1 eq.) versetzt und die Mischung über Nacht gerührt. Das Reaktionsgemisch wurde danach im Vakuum eingeengt, der Rückstand mit Wasser/Acetonitril (∼10:1) versetzt und die Lösung lyophilisiert. Die erhaltene Substanz wurde in Acetonitril gelöst und dann mittels präparativer HPLC gereinigt (Säule: Waters Sunfire C18, 5 µm; Säulendimension: 250 mm x 20 mm; Detektion: 240 nm; Temperatur: 28°C; Fluss: 25 ml/min; Injektionsvolumen: 500 µl; Eluent: Acetonitril/0.2% Trifluoressigsäure 45:55). Die Titelverbindung wurde als ein Feststoff erhalten (0.155 g, 26% d. Th.).
LC-MS (Methode 4): Rₜ = 1.10 min; MS (ESIpos): *m*/*z* (%) = 462.0 (100) [M+H]⁺.
¹H-NMR (400 MHz, DMSO-d₆): δ = 1.85 (s, 3H), 6.30 (s, 1H), 7.73-7.90 (m, 6H), 7.99 (d, 1H), 8.20-8.30 (m, 3H).

### Beispiel 2

### 5-Cyano-2-{(4S)-5-cyano-6-methyl-2-oxo-1-[3-(trifluormethyl)phenyl]-1,2,3,4-tetrahydro-pyrimidin-4-yl}-N-methylbenzolsulfonamid

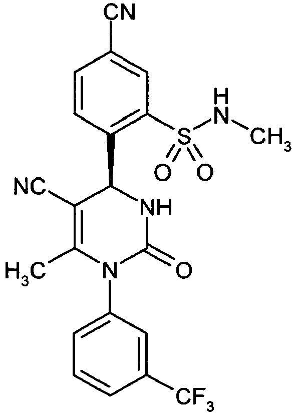

5-Cyano-2-{(4*S*)-5-cyano-6-methyl-2-oxo-1-[3-(trifluormethyl)phenyl]-1,2,3,4-tetrahydropyrimidin-4-yl}benzolsulfonylchlorid (40 mg, 83 µmol) wurde in THF (5 ml) gelöst, bei Raumtemperatur mit 2 M Methylamin-Lösung in THF (208 µl, 415 µmol; 5 eq.) versetzt und über Nacht gerührt. Das Reaktionsgemisch wurde danach im Vakuum eingeengt und der Rückstand mittels präparativer HPLC gereinigt (Säule: Gromsil C-18, 10 µm; Eluent: Acetonitril/Wasser + 0.1% TFA 10:90 → 90:10). Es wurde ein farbloser, amorpher Feststoff erhalten (14.3 mg, 36% d. Th.).
LC-MS (Methode 6): Rₜ = 2.29 min; MS (ESIpos): *m*/*z* (%) = 476.0 (100) [M+H]⁺.
¹H-NMR (400 MHz, DMSO-d₆): δ = 1.85 (s, 3H), 2.55 (s, 3H), 6.30 (s, 1H), 7.70-8.00 (m, 5H), 8.20-8.30 (m, 4H).

### Beispiel 3

### 5-Cyano-2-{(4S)-5-cyano-6-methyl-2-oxo-1-[3-(trifluormethyl)phenyl]-1,2,3,4-tetrahydro-pyrimidin-4-yl}-N,N-dimethylbenzolsulfonamid

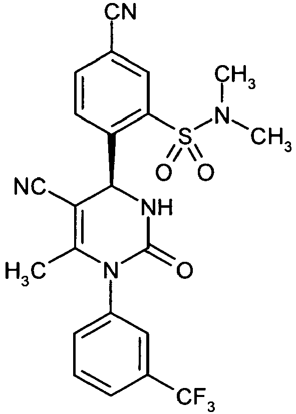

5-Cyano-2-{(4*S*)-5-cyano-6-methyl-2-oxo-1-[3-(trifluormethyl)phenyl]-1,2,3,4-tetrahydropyrimidin-4-yl}benzolsulfonylchlorid (40 mg, 83 µmol) wurde in THF (5 ml) gelöst, bei Raumtemperatur mit 33%-iger Dimethylamin-Lösung in Ethanol (37 µl, 208 µmol; 2.5 eq.) versetzt und über Nacht gerührt. Das Reaktionsgemisch wurde danach im Vakuum eingeengt und der Rückstand mittels präparativer HPLC gereinigt (Säule: Gromsil C-18, 10 µm; Eluent: Acetonitril/Wasser + 0.1% TFA 10:90 → 90:10). Es wurde ein farbloser, amorpher Feststoff erhalten (13.8 mg, 34% d. Th.).
LC-MS (Methode 6): Rₜ = 2.41 min; MS (ESIpos): *m*/*z* (%) = 489.9 (100) [M+H]⁺.
¹H-NMR (400 MHz, DMSO-d₆): δ = 1.80 (s, 3H), 2.80 (s, 6H), 6.20 (s, 1H), 7.70-8.00 (m, 4H), 8.25-8.40 (m, 4H).

### Beispiel 4

### (4S)-4-[4-Cyano-2-(morpholin-4-ylsulfonyl)phenyl]-6-methyl-2-oxo-1-[3-(trifluormethyl)phenyl]-1,2,3,4-tetrahydropyrimidin-5-carbonitril

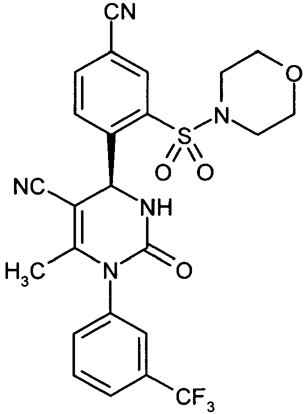

5-Cyano-2-{(4*S*)-5-cyano-6-methyl-2-oxo-1-[3-(trifluormethyl)phenyl]-1,2,3,4-tetrahydropyrimidin-4-yl}benzolsulfonylchlorid (20 mg, 42 µmol) wurde unter Argonschutzgasatmosphäre in absolutem Dichlormethan (2.5 ml) gelöst, bei Raumtemperatur mit Morpholin (7.3 µl, 83 µmol; 2 eq.) versetzt und über Nacht gerührt. Das Reaktionsgemisch wurde danach im Vakuum eingeengt und der Rückstand mittels präparativer HPLC gereinigt (Säule: Gromsil C-18, 10 µm; Eluent: Acetonitril/Wasser + 0.1% TFA 10:90 → 90:10). Es wurde ein farbloser, amorpher Feststoff erhalten (8.6 mg, 35% d. Th.).
LC-MS (Methode 6): Kₜ = 2.39 min; MS (ESIpos): *m*/*z* (%) = 532.0 (100) [M+H]⁺.
¹H-NMR (400 MHz, DMSO-d₆): δ = 1.80 (s, 3H), 3.10 (m, 2H), 3.15 (m, 2H), 3.70 (m, 4H), 6.20 (s, 1H), 7.70-8.00 (m, 4H), 8.25-8.40 (m, 4H).

### Beispiel 5

### 5-Cyano-2-{(4S)-5-cyano-3,6-dimethyl-2-oxo-1-[3-(trifluormethyl)phenyl]-1,2,3,4-tetrahydro-pyrimidin-4-yl}-N,N-bis(2-hydroxyethyl)benzolsulfonamid

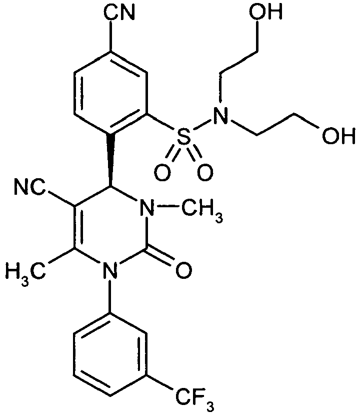

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (50 mg, 101 µmol) wurde unter Argonschutzgasatmosphäre in absolutem THF (2.5 ml) gelöst, bei Raumtemperatur mit Diethanolamin (29 µl, 303 µmol; 3 eq.) sowie Triethylamin (10.2 mg, 101 µmol; 1 eq.) versetzt und über Nacht gerührt. Das Reaktionsgemisch wurde danach im Vakuum eingeengt und der Rückstand mittels präparativer HPLC gereinigt (Säule: Gromsil C-18, 10 µm; Eluent: Acetonitril/Wasser + 0.1% TFA 10:90 → 90:10). Es wurde ein farbloser, amorpher Feststoff erhalten (4.9 mg, 9% d. Th.).
LC-MS (Methode 4): Rₜ = 1.11 min; MS (ESIpos): *m*/*z* (%) = 564.0 (100) [M+H]⁺; MS (ESIneg): *m*/*z* (%) = 562.8 (100) [M-H]⁻.
¹H-NMR (400 MHz, DMSO-d₆): δ = 1.80 (s, 3H), 2.65 (s, 3H), 3.45 (m, 4H), 3.65 (m, 4H), 5.00 (br. s, 2H), 6.20 (s, 1H), 7.70-8.00 (m, 4H), 8.25-8.40 (m, 3H).

### Beispiel 6

### (4S)-4-[4-Cyano-2-(morpholin-4-ylsulfonyl)phenyl]-3,6-dimethyl-2-oxo-1-[3-(trifluormethyl)-phenyl]-1,2,3,4-tetrahydropyrimidin-5-carbonitril

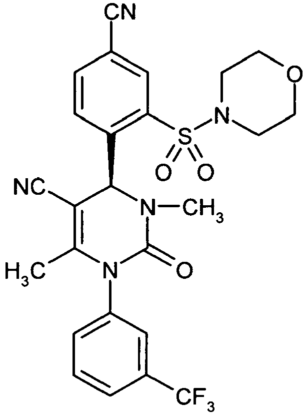

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (50 mg, 101 µmol) wurde unter Argonschutzgasatmosphäre in absolutem THF (2.5 ml) gelöst, bei Raumtemperatur mit Morpholin (26 µl, 303 µmol; 3 eq.) sowie Triethylamin (10.2 mg, 101 µmol; 1 eq.) versetzt und über Nacht gerührt. Das Reaktionsgemisch wurde danach im Vakuum eingeengt und der Rückstand mittels präparativer HPLC gereinigt (Säule: Kromasil C-18, 5 µm; Eluent: Acetonitril/Wasser + 0.1% TFA 10:90 → 90:10). Es wurde ein farbloser, amorpher Feststoff erhalten (44 mg, 80% d. Th.).
LC-MS (Methode 4): Rₜ = 1.27 min; MS (ESIpos): *m*/*z* (%) = 545.9 (100) [M+H]⁺; MS (ESIneg): *m*/*z* (%) = 544.0 (100) [M-H]⁻.
¹H-NMR (400 MHz, DMSO-d₆): δ = 1.80 (s, 3H), 2.70 (s, 3H), 3.30 (m, 4H), 3.70 (m, 4H), 6.20 (s, 1H), 7.70-8.05 (m, 4H), 8.25-8.40 (m, 3H).

### Beispiel 7

### 5-Cyano-2-{(4S)-5-cyano-3,6-dimethyl-2-oxo-1-[3-(trifluormethyl)phenyl]-1,2,3,4-tetrahydro-pyrimidin-4-yl}-N-(morpholin-4-yl)benzolsulfonamid

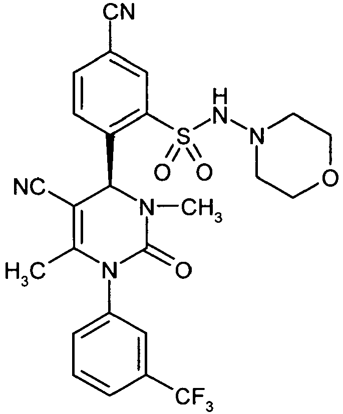

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (50 mg, 101 µmol) wurde unter Argonschutzgasatmosphäre in absolutem THF (2.5 ml) gelöst, bei Raumtemperatur mit *N*-Aminomorpholin (29 µl, 303 µmol; 3 eq.) sowie Triethylamin (10.2 mg, 101 µmol; 1 eq.) versetzt und über Nacht gerührt. Das Reaktionsgemisch wurde danach im Vakuum eingeengt und der Rückstand mittels präparativer HPLC gereinigt (Säule: Kromasil C-18, 5 µm; Eluent: Acetonitril/Wasser + 0.1% TFA 10:90 → 90:10). Es wurde ein farbloser, amorpher Feststoff erhalten (38 mg, 67% d. Th.).
LC-MS (Methode 5): Rₜ = 1.98 min; MS (ESIpos): *m*/*z* (%) = 101.0 (100), 561.2 (15) [M+H]⁺; MS (ESIneg): *m*/*z* (%) = 459.1 (100), 475.1 (60), 559.2 (30) [M-H]⁻.
¹H-NMR (400 MHz, DMSO-d₆): δ = 1.80 (s, 3H), 2.60 (m, 4H), 2.75 (s, 3H), 3.50 (br. s, 4H), 6.50 (s, 1H), 7.70-8.00 (m, 4H), 8.10-8.40 (m, 4H).

### Beispiel 8

### 5-Cyano-2-{(4S)-5-cyano-3,6-dimethyl-2-oxo-1-[3-(trifluormethyl)phenyl]-1,2,3,4-tetrahydro-pyrimidin-4-yl}-N,N-dimethylbenzolsulfonamid

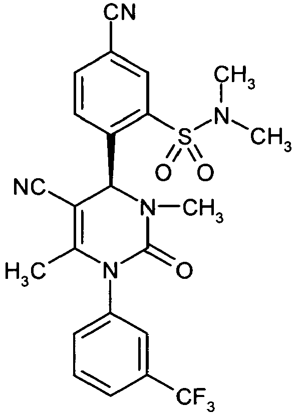

5-Cyano-2-{(4*S*)-5-cyano-6-methyl-2-oxo-1-[3-(trifluormethyl)phenyl]-1,2,3,4-tetrahydropyrimidin-4-yl}-*N,N*-dimethylbenzolsulfonamid (50 mg, 108 µmol) wurde unter Argonschutzgasatmosphäre in absolutem THF (4.5 ml) vorgelegt und bei -78°C mit einer 1 M Lösung von Lithiumhexamethyldisilazid (LiHMDS) in THF (130 µl, 130 µmol; 1.2 eq.) versetzt. Nach 30 min Rühren wurde Iodmethan (77 mg, 542 µmol; 5 eq.) in THF (1 ml) zugegeben und die Mischung für 16 h unter allmählicher Erwärmung von -78°C auf RT gerührt. Das Reaktionsgemisch wurde danach mit wenig Essigsäure versetzt und im Vakuum eingeengt. Der Rückstand wurde mittels präparativer HPLC gereinigt (Säule: Gromsil C-18; Eluent: Acetonitril/Wasser + 0.1% TFA 10:90 → 90:10). Es wurde ein farbloser, amorpher Feststoff erhalten (7.6 mg, 14% d. Th.).
LC-MS (Methode 4): Rₜ = 1.29 min; MS (ESIpos): *m*/*z* (%) = 504.1 (100) [M+H]⁺; MS (ESIneg): *m*/*z* (%) = 502.2 (100) [M-H]⁻.

### Beispiel 9

### 5-Cyano-2-{(4S)-5-cyano-3,6-dimethyl-2-oxo-1-[3-(trifluormethyl)phenyl]-1,2,3,4-tetrahydro-pyrimidin-4-yl}-N-methylbenzolsulfonamid

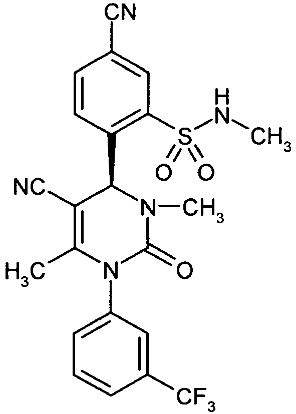

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (30 mg, 61 µmol) wurde unter Argonschutzgasatmosphäre in absolutem THF (2 ml) gelöst, bei Raumtemperatur mit einer 2 M Lösung von Methylamin in THF (91 µl, 182 µmol; 3 eq.) sowie Triethylamin (6.1 mg, 61 µmol; 1 eq.) versetzt und 3 h lang gerührt. Das Reaktionsgemisch wurde danach im Vakuum eingeengt und der Rückstand mittels präparativer HPLC gereinigt (Säule: Gromsil C-18; Eluent: Acetonitril/Wasser + 0.1% TFA 10:90 → 90:10). Es wurde ein farbloser, amorpher Feststoff erhalten (25 mg, 83% d. Th.).
LC-MS (Methode 4): Rₜ = 1.21 min; MS (ESIpos): *m*/*z* (%) = 490.1 (100) [M+H]⁺; MS (ESIneg): *m*/*z* (%) = 488.1 (100) [M-H]⁻.
¹H-NMR (400 MHz, DMSO-d₆): δ = 1.80 (s, 3H), 2.60 (d, 3H), 2.65 (s, 3H), 6.25 (s, 1H), 7.70-8.30 (m, 8H).

### Beispiel 10

### 5-Cyano-2-{(4S)-5-cyano-3,6-dimethyl-2-oxo-1-[3-(trifluormethyl)phenyl]-1,2,3,4-tetrahydro-pyrimidin-4-yl}benzolsulfonamid

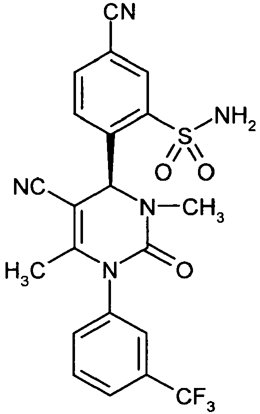

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (100 mg, 202 µmol) wurde unter Argonschutzgasatmosphäre bei Raumtemperatur mit einer 0.5 M Lösung von Ammoniak in Dioxan (4000 µl, 2021 µmol; 10 eq.) sowie Triethylamin (20.4 mg, 202 µmol; 1 eq.) versetzt und 3 h lang gerührt. Das Reaktionsgemisch wurde danach im Vakuum eingeengt und der Rückstand mittels präparativer HPLC gereinigt (Säule: Gromsil C-18; Eluent: Acetonitril/Wasser + 0.1% TFA 10:90 → 90:10). Es wurde ein farbloser, amorpher Feststoff erhalten (56 mg, 58% d. Th.).
LC-MS (Methode 4): Rₜ = 1.14 min; MS (ESIpos): *m*/*z* (%) = 476.0 (100) [M+H]⁺; MS (ESIneg): *m*/*z* (%) = 473.9 (100) [M-H]⁻.
¹H-NMR (400 MHz, DMSO-d₆): δ = 1.80 (s, 3H), 2.70 (s, 3H), 6.25 (s, 1H), 7.60-8.30 (m, 9H).

### Beispiel 11

### 5-Cyano-2-{(4S)-5-cyano-3,6-dimethyl-2-oxo-1-[3-(trifluormethyl)phenyl]-1,2,3,4-tetrahydro-pyrimidin-4-yl}-N-(2-hydroxyethyl)benzolsulfonamid

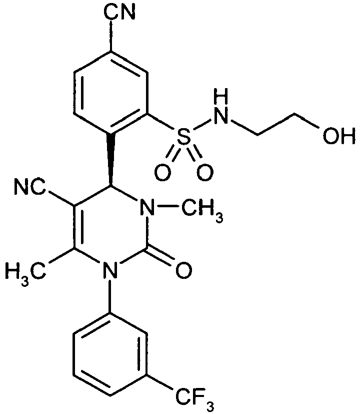

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (50 mg, 101 µmol) wurde unter Argonschutzgasatmosphäre in absolutem THF (2.5 ml) gelöst, bei Raumtemperatur mit Ethanolamin (18 µl, 303 µmol; 3 eq.) sowie Triethylamin (10.2 mg, 101 µmol; 1 eq.) versetzt und über Nacht gerührt. Das Reaktionsgemisch wurde danach im Vakuum eingeengt und der Rückstand mittels präparativer HPLC gereinigt (Säule: Kromasil C-18, 5 µm; Eluent: Acetonitril/Wasser + 0.1% TFA 10:90 → 90:10). Es wurde ein farbloser, amorpher Feststoff erhalten (43 mg, 81 % d. Th.).
LC-MS (Methode 4): Rₜ = 1.13 min; MS (ESIpos): *m*/*z* (%) = 520.0 (100) [M+H]⁺.
¹H-NMR (400 MHz, DMSO-d₆): δ = 1.80 (s, 3H), 2.65 (s, 3H), 3.00 (m, 2H), 3.45 (m, 2H), 6.30 (s, 1H), 7.70-8.30 (m, 7H), 8.35 (s, 1H), 8.45 (t, 1H).

### Beispiel 12

### 5-Cyano-2-{(4S)-5-cyano-3,6-dimethyl-2-oxo-1-[3-(trifluormethyl)phenyl]-1,2,3,4-tetrahydro-pyrimidin-4-yl}-N-(3-hydroxypropyl)benzolsulfonamid

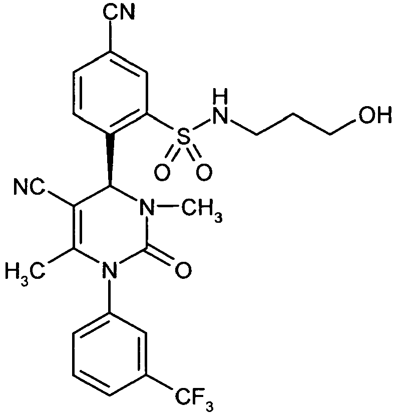

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (25 mg, 51 µmol) wurde unter Argonschutzgasatmosphäre in absolutem THF (1.5 ml) gelöst, bei Raumtemperatur mit 3-Aminopropanol (11 µl, 152 µmol; 3 eq.) sowie Triethylamin (5.1 mg, 51 µmol; 1 eq.) versetzt und über Nacht gerührt. Das Reaktionsgemisch wurde danach im Vakuum eingeengt und der Rückstand mittels präparativer HPLC gereinigt (Säule: Kromasil C-18, 5 µm; Eluent: Acetonitril/Wasser + 0.1% TFA 10:90 → 90:10). Es wurde ein farbloser, amorpher Feststoff erhalten (18 mg, 67% d. Th.).
LC-MS (Methode 4): Rₜ = 1.14 min; MS (ESIpos): *m*/*z* (%) = 534.1 (100) [M+H]⁺; MS (ESIneg): *m*/*z* (%) = 532.1 (100) [M-H]⁻.

### Beispiel 13

### N²-[(5-Cyano-2-{(4S)-5-cyano-3,6-dimethyl-2-oxo-1-[3-(trifluormethyl)phenyl]-1,2,3,4-tetrahydro-pyrimidin-4-yl}phenyl)sulfonyl]glycinamid

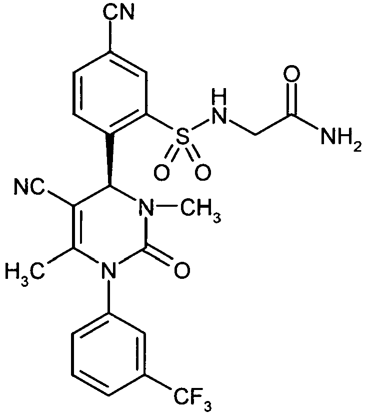

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (50 mg, 101 µmol) wurde unter Argonschutzgasatmosphäre in absolutem THF (2.5 ml) gelöst, bei Raumtemperatur mit Glycinamid-Hydrochlorid (57 mg, 505 µmol; 5 eq.) sowie Triethylamin (102 mg, 1010 µmol; 10 eq.) versetzt und über Nacht gerührt. Das Reaktionsgemisch wurde danach im Vakuum eingeengt und der Rückstand mittels präparativer HPLC gereinigt (Säule: Gromsil C-18, 10 µm; Eluent: Acetonitril/Wasser + 0.1% TFA 10:90 → 90:10). Es wurde ein farbloser, amorpher Feststoff erhalten (39.2 mg, 73% d. Th.).
LC-MS (Methode 4): Rₜ = 1.09 min; MS (ESIpos): *m*/*z* (%) = 532.9 (100) [M+H]⁺.
¹H-NMR (400 MHz, DMSO-d₆): δ = 1.80 (s, 3H), 2.65 (s, 3H), 3.60 (m, 2H), 6.30 (s, 1H), 7.15 (s, 1H), 7.40 (s, 1H), 7.70-8.30 (m, 6H), 8.40 (s, 1H), 8.70 (t, 1H).

### Allgemeine Vorschrift zur Herstellung weiterer Sulfonamid-Derivate:

Die betreffende Amin-Komponente (0.1 mmol) wurde in 1,2-Dichlorethan (0.2 ml) vorgelegt. Anschließend wurden *N,N*-Diisopropylethylamin (25.8 mg, 0.2 mmol) sowie 5-Cyano-2-{(4*S*)-5-cyano-6-methyl-2-oxo-1-[3-(trifluormethyl)phenyl]-1,2,3,4-tetrahydropyrimidin-4-yl}benzolsulfo-nylchlorid (48.1 mg, 0.1 mmol), gelöst in 1,2-Dichlorethan (0.3 ml), zugegeben. Das Gemisch wurde über Nacht bei Raumtemperatur gerührt. Danach wurde das Dichlorethan in einer Vakuumzentrifuge abgedampft. Der Rückstand wurde in Dimethylsulfoxid (0.5 ml) gelöst und mittels präparativer HPLC/MS gereinigt.

Nach dieser Vorschrift wurden die in der folgenden Tabelle aufgeführten Verbindungen erhalten:

| **Beispiel** | **Struktur / Name** | **Analytische Daten** |
|---|---|---|
| **14** | 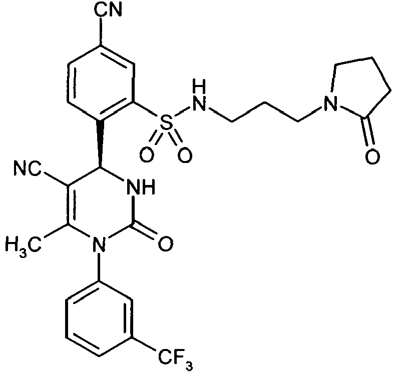 | MS (ESIpos): m/z = 587 (M+H)⁺ LC-MS (Methode 11): Rₜ = 1.97 min. |
| | | ¹H-NMR (400 MHz, DMSO-*d*₆): δ = 1.64 (quin, *J* = 7.09 Hz, 2H), 1.84 (s, 3H), 1.86-1.94 (m, 2H), 2.13-2.22 (m, 2H), 2.81-2.90 (m, 2H), 3.10-3.24 (m, 2H), 6.29 (s, 1H), 7.74 (br. s, 2H), 7.79-7.86 (m, 1H), 7.90-7.98 (m, 1H), 8.11-8.19 (m, 2H), 8.25 (s, 1H), 8.29 (s, 4H). |
| | 5-Cyano-2-{(*4*S)-5-cyano-6-methyl-2-oxo-1-[3-(trifluormethyl)phenyl]-1,2,3,4-tetrahydropyrimidin-4-yl}-*N*-[3-(2-oxopyrrolidin-1-yl)propyl]benzolsulfonamid | |
| **15** | 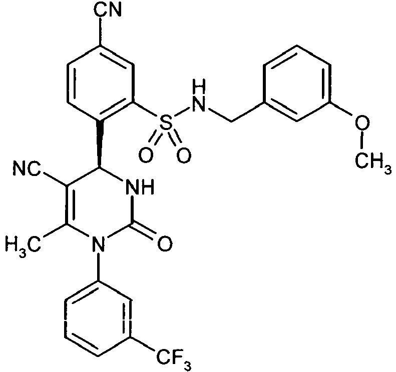 | MS (ESIpos): m/z = 582 (M+H)⁺ LC-MS (Methode 11): Rₜ = 2.17 min. |
| | | ¹H-NMR (400 MHz, DMSO-d₆): δ = 1.85 (s, 3H), 3.72 (s, 3H), 4.13 (br. s, 2H), 6.34 (s, 1H), 6.72-6.93 (m, 3H), 7.22 (t, *J* = 7.83 Hz, 1H), 7.74 (d, *J* = 4.89 7.83 Hz, 1H), 7.74 (d, *J* = 4.89 Hz, 2H), 7.78-7.87 (m, 1H), 7.87-7.98 (m, 1H), 8.17 (s, 1H), 8.25 (s, 3H), 8.67 (br. s, 1H). |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(3-methoxybenzyl)benzolsulfonamid | |
| **16** | 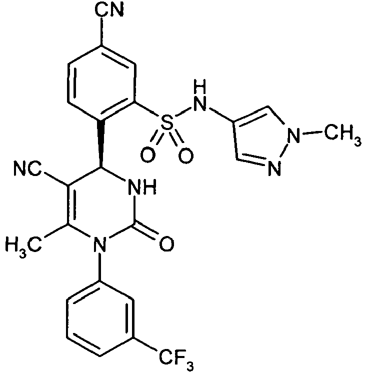 | MS (ESIpos): m/z = 542 (M+H)⁺ LC-MS (Methode 11): Rₜ = 1.94 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(1-methyl-1*H*-pyrazol-4-yl)benzolsulfonamid | |
| **17** | 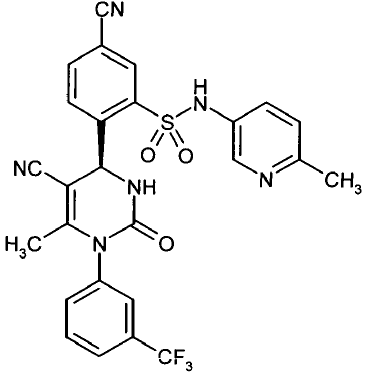 | MS (ESIpos): m/z = 553 (M+H)⁺ LC-MS (Methode 11): Rₜ = 1.79 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(6-methylpyridin-3-yl)benzolsulfonamid | |
| **18** | 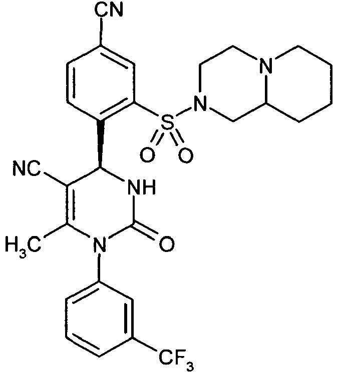 | MS (ESIpos): m/z = 585 (M+H)⁺ LC-MS (Methode 11): Rₜ = 1.55 min. |
| | | ¹H-NMR (400 MHz, DMSO-*d*₆): δ = 0.93-1.14 (m, 1H), 1.22 (dd, *J* = 9.05, 4.65 Hz, 1H), 1.31-1.46 (m, 1H), 1.56 (d, *J* = 11.74 Hz, 2H), 1.65 (br. s, 1H), 1.82 (s, 3H), 1.88-2.04 (m, 2H), 2.08-2.30 (m, 2H), 2.63-2.90 (m, 3H), 3.52 (t, *J* = 11.00 Hz, 1H), 3.63 (t, *J* = 11.74 Hz, 1H), 6.21 (d, *J* = 6.85 Hz, 1H), 7.66-7.78 (m, 2H), 7.82 (d, *J* = 6.85 Hz, 1H), 7.94 (br. s, 1H), 8.20-8.43 (m, 4H). |
| | (4*S*)-4-[4-Cyano-2-(octahydro-2*H*-pyrido[1,2-a]-pyrazin-2-ylsulfonyl)phenyl]-6-methyl-2-oxo-1-[3-(trifluormethyl)phenyl]-1,2,3,4-tetrahydro-pyrimidin-5-carbonitril | |
| **19** | 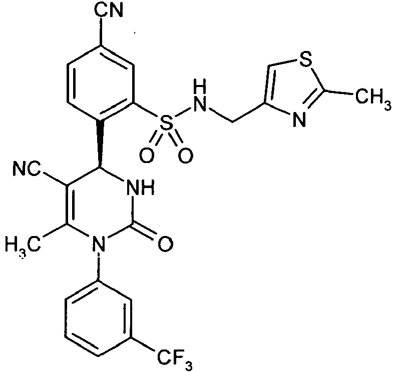 | MS (ESIpos): m/z = 573 (M+H)⁺ LC-MS (Methode 11): Rₜ = 2.09 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[(2-methyl-1,3-thiazol-4-yl)methyl]-benzolsulfonamid | |
| **20** | 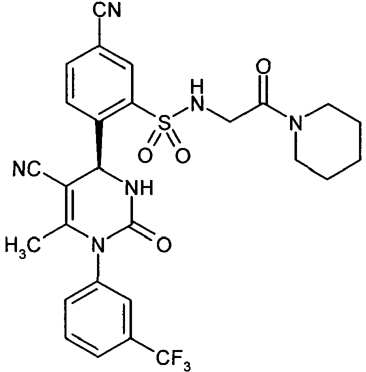 | MS (ESIpos): m/z = 587 (M+H)⁺ LC-MS (Methode 11): Rₜ = 2.13 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[2-oxo-2-(piperidin-1-yl)ethyl]benzolsulfonamid | |
| **21** | 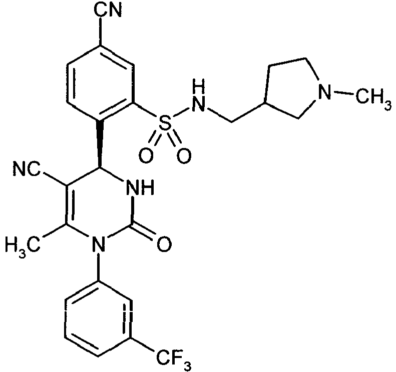 | MS (ESIpos): m/z = 559 (M+H)⁺ LC-MS (Methode 11): Rₜ = 1.51 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[(1-methylpyrrolidin-3-yl)methyl]benzolsulfonamid | |
| **22** | 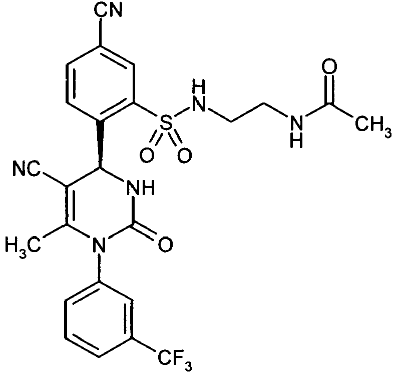 | MS (ESIpos): m/z = 547 (M+H)⁺ LC-MS (Methode 11): Rₜ = 1.86 min. |
| | *N*-(2-{[(5-Cyano-2-{(4*S*)-5-cyano-6-methyl-2-oxo-1-[3-(trifluormethyl)phenyl]-1,2,3,4-tetrahydro-pyrimidin-4-yl}phenyl)sulfonyl]amino}ethyl)-acetamid | |
| **23** | 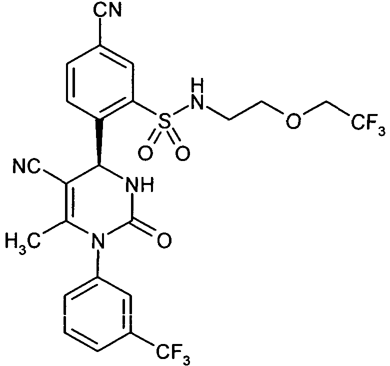 | MS (ESIpos): m/z = 588 (M+H)⁺ LC-MS (Methode 11): Rₜ = 2.15 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[2-(2,2,2-trifluorethoxy)ethyl]benzolsulfonamid | |
| **24** | 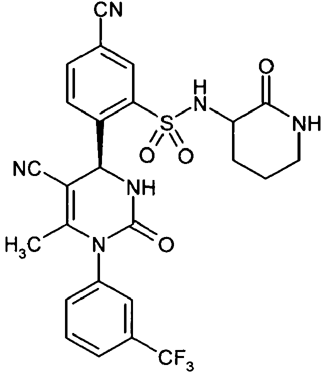 | MS (ESIpos): m/z = 559 (M+H)⁺ LC-MS (Methode 11): Rₜ = 1.99 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(2-oxopiperidin-3-yl)benzolsulfonamid | |
| **25** | 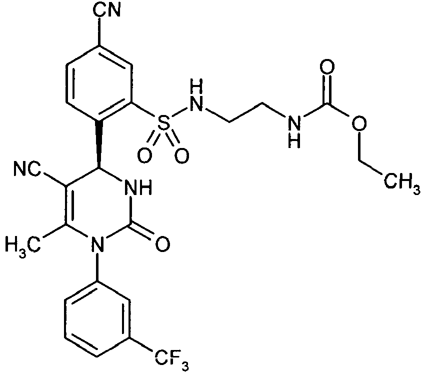 | MS (ESIpos): m/z = 577 (M+H)⁺ LC-MS (Methode 11): Rₜ = 2.01 min. |
| | Ethyl-(2-{[(5-cyano-2-{(4*S*)-5-cyano-6-methyl-2-oxo-1-[3-(trifluormethyl)phenyl]-1,2,3,4-tetra-hydropyrimidin-4-yl}phenyl)sulfonyl]amino}-ethyl)carbamat | |
| **26** | 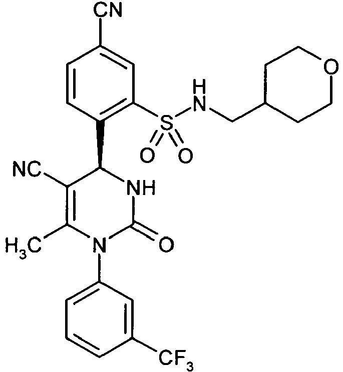 | MS (ESIpos): m/z = 560 (M+H)⁺ LC-MS (Methode 11): Rₜ = 2.03 min. |
| | | ¹H-NMR (400 MHz, DMSO-*d*₆): δ = 1.00-1.22 (m, 2H), 1.59 (d, *J* = 13.21 Hz, 2H), 1.62-1.74 (m, 1H), 1.84 (s, 3H), 2.71-2.84 (m, 2H), 3.18-3.27 (m, 2H), 3.67-3.92 (m, 2H), 6.31 (s, 1H), 7.74 (d, *J* = 4.40 Hz, 2H), 7.82 (d, *J* = 3.42 Hz, 1H), 7.93 (br. s, 1H), 8.07-8.21 (m, 2H), 8.26 (d, *J* = 8.31 Hz, 3H). |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(tetrahydro-2*H*-pyran-4-ylmethyl)benzolsulfonamid | |
| **27** | 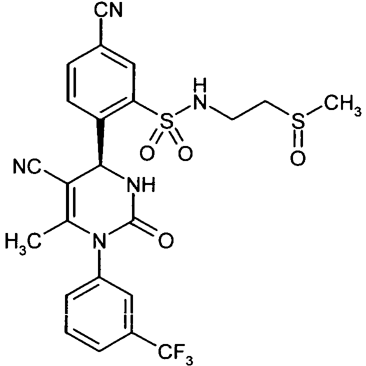 | MS (ESIpos): m/z = 552 (M+H)⁺ LC-MS (Methode 11): Rₜ = 1.83 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[2-(methylsulfinyl)ethyl]benzolsulfonamid | |
| **28** | 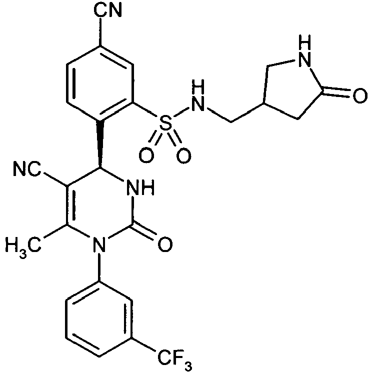 | MS (ESIpos): m/z = 559 (M+H)⁺ LC-MS (Methode 11): Rₜ = 1.83 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[(5-oxopyrrolidin-3-yl)methyl]benzol-sulfonamid | |
| **29** | 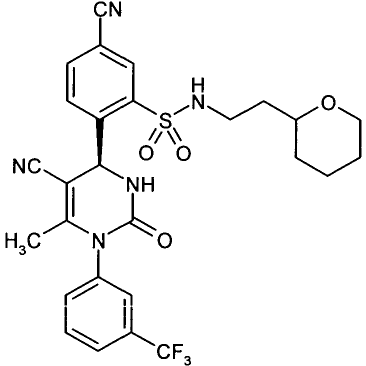 | MS (ESIpos): m/z = 574 (M+H)⁺ LC-MS (Methode 11): Rₜ = 2.18 min. |
| | | ¹H-NMR (400 MHz, DMSO-*d*₆): δ = 1.13 (br. s, 1H), 1.41 (br. s, 3H), 1.46-1.64 (m, 3H), 1.72 (br. s, 1H), 1.84 (s, 3H), 2.94 (br. s, 2H), 3.25 (br. s, 3H), 3.68-3.89 (m, 1 H), 6.29 (s, 1 H), 7.74 (d, *J* = 4.40 Hz, 2H), 7.82 (d, *J* = 2.93 Hz, 1H), 7.88-8.00 (m, 1H), 8.04-8.17 (m, 1H), 8.26 (d, *J* = 15.16 Hz, 3H). |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[2-(tetrahydro-2*H*-pyran-2-yl)ethyl]benzolsulfonamid | |
| **30** | 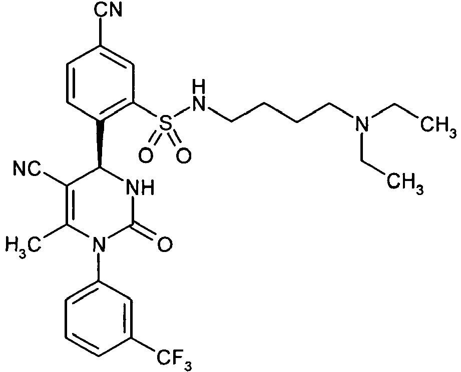 | MS (ESIpos): m/z = 589 (M+H)⁺ LC-MS (Methode 11): Rₜ = 1.55 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[4-(diethylamino)butyl]benzolsulfonamid | |
| **31** | 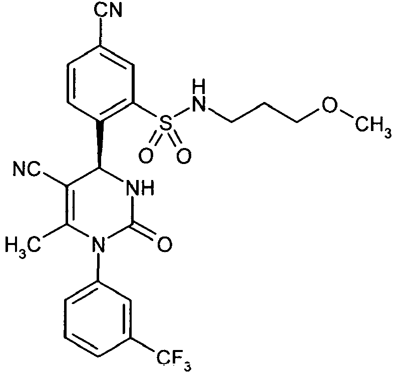 | MS (ESIpos): m/z = 533 (M+H)⁺ LC-MS (Methode 11): Rₜ = 2.05 min. |
| | | ¹H-NMR (400 MHz, DMSO-*d*₆): δ = 1.68 (quin, *J* = 6.60 Hz, 2H), 1.84 (s, 3H), 2.93 (q, J = 6.36 Hz, 2H), 3.20 (s, 3H), 6.29 (s, 1H), 7.74 (d, *J* = 4.40 Hz, 2H), 7.82 (d, *J* = 3.42 Hz, 1H), 7.92 (br. s, 1H), 8.08-8.16 (m, 2H), 8.24 (s, 2H), 8.28 (s, 3H). 2H), 8.28 s, 3H). |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(3-methoxypropyl)benzolsulfonamid | |
| **32** | 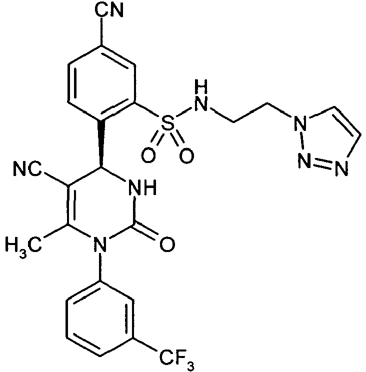 | MS (ESIpos): m/z = 557 (M+H)⁺ LC-MS (Methode 11): Rₜ = 1.91 min. |
| | | ¹H-NMR (400 MHz, DMSO-*d*₆): δ = 1.70-1.89 (m, 3H), 1.83 (s, 3H), 3.36-3.43 (m, 2H), 4.50 (t, *J* = 6.11 Hz, 2H), 6.25 (s, 1H), 7.74 (s, 7H), 7.83 (br. s, 3H), 7.88-8.00 (m, 2H), 8.12 (s, 3H), 8.16 (d, *J* = 0.98 Hz, 2H), 8.23 (s, 2H), 8.29 (s, 3H), 8.41-8.56 (m, 1H). |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[2-(1*H*-1,2,3-triazol-1-yl)ethyl]benzolsulfonamid | |
| 33 | 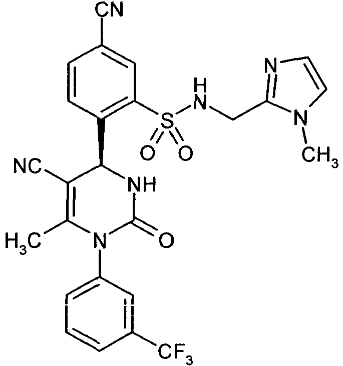 | MS (ESIpos): m/z = 556 (M+H)⁺ LC-MS (Methode 11): Rₜ = 1.51 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[(1-methyl-1*H*-imidazol-2-yl)methyl]benzolsulfonamid | |
| **34** | 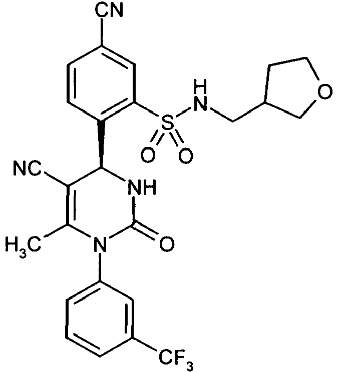 | MS (ESIpos): m/z = 546 (M+H)⁺ LC-MS (Methode 11): Rₜ = 2.00 min. |
| | | ¹H-NMR (400 MHz, DMSO-*d*₆): δ = 1.43-1.64 (m, 1H), 1.84 (s, 3H), 1.87-1.99 (m, 1H), 2.25-2.41 (m, 1H), 2.87 (d, *J* = 5.38 Hz, 2H), 3.37-3.48 (m, 1H), 3.51-3.63 (m, 1H), 3.62-3.78 (m, 3H), 6.30 (s, 1H), 7.74 (d, *J* = 3.91 Hz, 2H), 7.77-7.87 (m, 1H), 7.93 (br. s, 1H), 8.27 (d, *J* = 10.76 Hz, 4H). |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(tetrahydrofuran-3-ylmethyl)benzol-sulfonamid | |
| 35 | 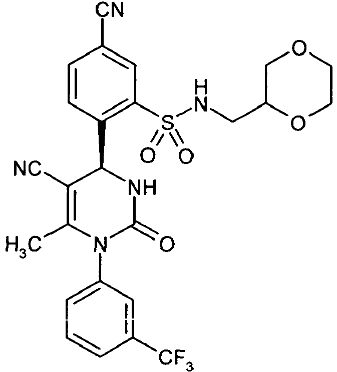 | MS (ESIpos): m/z = 562 (M+H)⁺ LC-MS (Methode 11): Rₜ = 2.01 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-(1,4-dioxan-2-ylmethyl)benzolsulfonamid | |
| **36** | 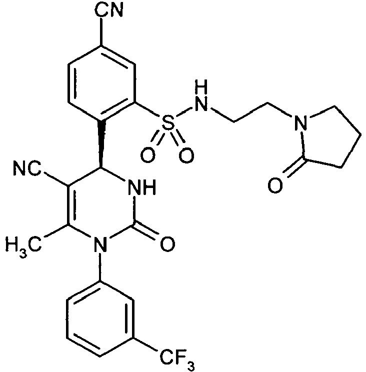 | MS (ESIpos): m/z = 573 (M+H)⁺ LC-MS (Methode 11): Rₜ = 1.93 min. |
| | 5-Cyano-2-{(4*S*)-5-cyano-6-methyl-2-oxo-1-[3-(trifluormethyl)phenyl]-1,2,3,4-tetrahydropyrimidin-4-yl}-*N*-[2-(2-oxopyrrolidin-1-yl)ethyl]benzol-sulfonamid | |

### Beispiel 37

### (R_{S})-(4S)-4-[4-Cyano-2-(S-methylsulfonimidoyl)phenyl]-3,6-dimethyl-2-oxo-1-[3-(trifluormethyl)-phenyl]-1,2,3,4-tetrahydropyrimidin-5-carbonitril oder (S_{S})-(4S)-4-[4-Cyano-2-(S-methylsulfonimidoyl)phenyl]-3,6-dimethyl-2-oxo-1-[3-(trifluor-methyl)phenyl]-1,2,3,4-tetrahydropyrimidin-5-carbonitril (Diastereomer 1)

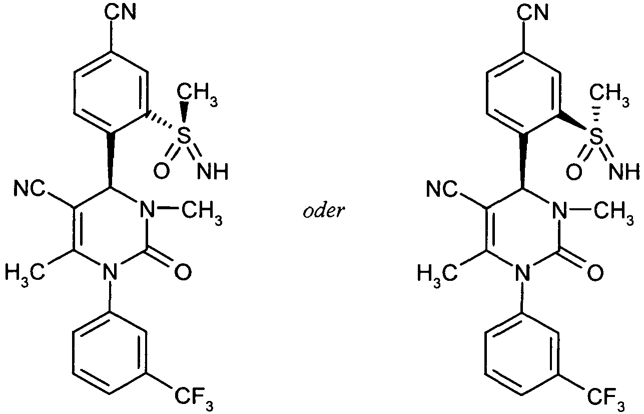

Die Reaktion wurde unter Argon durchgeführt. Die Verbindung aus Beispiel 21A ("Diastereomer 1 "; 63 mg, 111 µmol) wurde in einem Acetonitril/Methanol-Gemisch (10:1, 6 ml) vorgelegt. Bei 0°C wurde festes Kaliumcarbonat (7.6 mg, 55 µmol; 0.5 eq.) hinzugegeben und der Ansatz 15 min lang gerührt. Anschließend wurde mit Trifluoressigsäure (6.3 mg, 55 µmol; 0.5 eq.) neutralisiert, das Gemisch im Vakuum eingeengt und der Rückstand mittels präparativer HPLC gereinigt (Säule: Gromsil C-18, 10 µm; Eluent: Acetonitril/Wasser + 0.1% TFA 10:90 → 80:20). Die Titelverbindung wurde als ein Feststoff isoliert (48 mg, 91% d. Th.).
LC-MS (Methode 8): Rₜ = 0.99 min; MS (ESIpos): *m*/*z* (%) = 474.3 (100) [M+H]⁺; MS (ESIneg): *m*/*z* (%) = 472.4 (100) [M-H]⁻.
LC-MS (Methode 4): Rₜ = 1.13 min; MS (ESIpos): *m*/*z* (%) = 474.1 (100) [M+H]⁺; MS (ESIneg): *m*/*z* (%) = 472.4 (100) [M-H]⁻.
¹H-NMR (400 MHz, DMSO-d₆): δ = 1.80 (s, 3H), 2.70 (s, 3H), 3.30 (s, 3H), 4.85 (s, 1H), 6.75 (s, 1H), 7.70-8.30 (m, 6H), 8.50 (s, 1H).

### Beispiel 38

### (S_{S})-(4S)-4-[4-Cyano-2-(S-methylsulfonimidoyl)phenyl]-3,6-dimethyl-2-oxo-1-[3-(trifluormethyl)phenyl]-1,2,3,4-tetrahydropyrimidin-5-carbonitril oder (R_{S})-(4S)-4-[4-Cyano-2-(S-methylsulfonimidoyl)phenyl]-3,6-dimethyl-2-oxo-1-[3-(trifluormethyl)-phenyl]-1,2,3,4-tetrahydropyrimidin-5-carbonitril (Diastereomer 2)

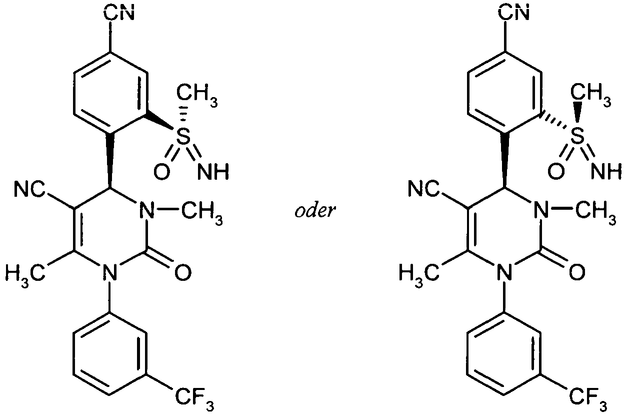

Die Reaktion wurde unter Argon durchgeführt. Die Verbindung aus Beispiel 22A ("Diastereomer 2"; 78 mg, 137 µmol) wurde in einem Acetonitril/Methanol-Gemisch (10:1, 7.7 ml) vorgelegt. Bei 0°C wurde festes Kaliumcarbonat (9.5 mg, 68 µmol; 0.5 eq.) hinzugegeben und der Ansatz 15 min lang gerührt. Anschließend wurde mit Trifluoressigsäure (7.8 mg, 68 µmol; 0.5 eq.) neutralisiert, das Gemisch im Vakuum eingeengt und der Rückstand mittels präparativer HPLC gereinigt (Säule: Gromsil C-18, 10 µm; Eluent: Acetonitril/Wasser + 0.1% TFA 10:90 → 80:20). Die Titelverbindung wurde als ein Feststoff isoliert (60 mg, 93% d. Th.).
LC-MS (Methode 8): Rₜ = 0.98 min; MS (ESIpos): *m*/*z* (%) = 474.3 (100) [M+H]⁺; MS (ESIneg): *m*/*z* (%) = 472.4 (100) [M-H]⁻.
LC-MS (Methode 5): Rₜ = 1.76 min; MS (ESIpos): *m*/*z* (%) = 474.1 (100) [M+H]⁺; MS (ESIneg): *m*/*z* (%) = 472.2 (100) [M-H]⁻.
Chirale analytische HPLC [Säule: Chiralpak AD-H, 5 µm, 250 mm x 4.6 mm; Eluent: *iso*-Hexan/ Ethanol 50:50; Fluss: 1 ml/min; Injektionsvolumen: 10 µl; Temperatur: 40°C; Detektion: 220 nm]: Rₜ = 4.40 min.
¹H-NMR (400 MHz, DMSO-d₆): δ = 1.80 (s, 3H), 2.70 (s, 3H), 3.30 (s, 3H), 6.80 (s, 1H), 7.70-8.30 (m, 6H), 8.45 (s, 1H).
[α]_{D}²⁰ = -286.9° (c = 0.49, Chloroform).

### Beispiel 39

### (R_{S})-(4S)-4-[4-Cyano-2-(S-methylsulfonimidoyl)phenyl]-6-methyl-2-oxo-1-[3-(trifluormethyl)-phenyl]-1,2,3,4-tetrahydropyrimidin-5-carbonitril und (S_{S})-(4S)-4-[4-Cyano-2-(S-methylsulfonimidoyl)phenyl]-6-methyl-2-oxo-1-[3-(trifluormethyl)-phenyl]-1,2,3,4-tetrahydropyrimidin-5-carbonitril (Diastereomerengemisch)

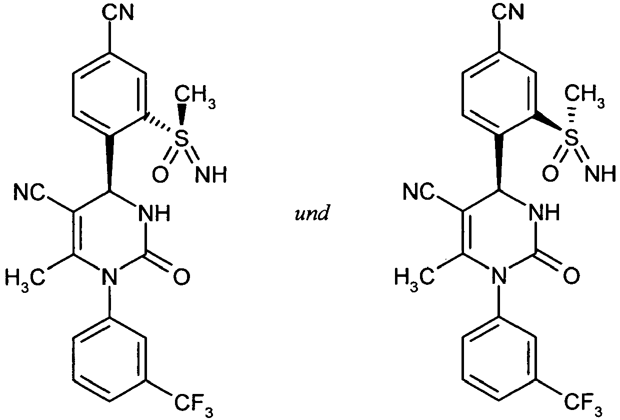

Die Reaktion wurde unter Argon durchgeführt. Das Diastereomerengemisch aus Beispiel 23A (485 mg, 873 µmol) wurde in einem Acetonitril/Methanol-Gemisch (10:1, 44 ml) vorgelegt. Bei 0°C wurde festes Kaliumcarbonat (60.3 mg, 437 µmol; 0.5 eq.) hinzugegeben und der Ansatz 15 min lang gerührt. Anschließend wurde mit Trifluoressigsäure (49.8 mg, 437 µmol ; 0.5 eq.) neutralisiert, das Gemisch im Vakuum eingeengt und der Rückstand in Essigsäureethylester (50 ml) aufgenommen. Die organische Phase wurde mit gesättigter wässriger Natriumchlorid-Lösung (2 x 15 ml) gewaschen, über Natriumsulfat getrocknet, filtriert und im Vakuum eingeengt. Die Titelverbindung wurde als ein Feststoff isoliert (400 mg, quant.).
LC-MS (Methode 6): Rₜ = 2.03 min; MS (ESIpos): *m*/*z* (%) = 417.0 (50), 460.0 (100) [M+H]⁺.
¹H-NMR (400 MHz, DMSO-d₆): δ = 1.80 (2 s, 3H), 3.30 (2 s, 3H), 4.75 (2 s, 1H), 6.65 (2 s, 1H), 7.70-8.40 (m, 8H).

### Beispiel 40

### (R_{S})-(4S)-4-[4-Cyano-2-(S-methylsulfonimidoyl)phenyl]-6-methyl-2-oxo-1-[3-(trifluormethyl)-phenyl]-1,2,3,4-tetrahydropyrimidin-5-carbonitril oder (S_{S})-(4S)-4-[4-Cyano-2-(S-methylsulfonimidoyl)phenyl]-6-methyl-2-oxo-1-[3-(trifluormethyl)-phenyl]-1,2,3,4-tetrahydropyrimidin-5-carbonitril (Diastereomer 1)

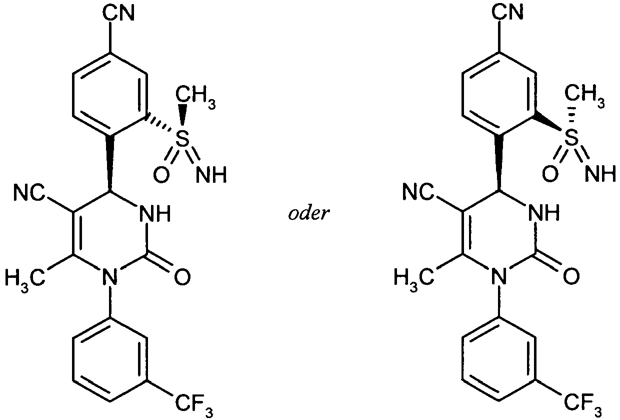

Das Diastereomerengemisch von (*R_{S}*)-(4*S*)-4-[4-Cyano-2-(*S*-methylsulfonimidoyl)phenyl]-6-methyl-2-oxo-1-[3-(trifluormethyl)phenyl]-1,2,3,4-tetrahydropyrimidin-5-carbonitril und (*S_{S}*)-(4*S*)-4-[4-Cyano-2-(*S*-methylsulfonimidoyl)phenyl]-6-methyl-2-oxo-1-[3-(trifluormethyl)phenyl]-1,2,3,4-tetrahydropyrimidin-5-carbonitril (400 mg) wurde durch präparative HPLC-Chromatographie an chiraler Phase aufgetrennt [Säule: Daicel Chiralpak AD-H, 250 mm x 20 mm; Probenvorbereitung: Probe in 20 ml Ethanol gelöst; Injektionsvolumen: 0.750 ml; Eluent: *iso*-Hexan/Ethanol 3:7; Fluss: 15 ml/min; Temperatur: 40°C; Detektion: 220 nm]. Das Diastereomer 1 wurde als früher eluierende Fraktion in Form eines Feststoffs erhalten (296 mg, 74% d. Th., Gehalt >99%).
LC-MS (Methode 6): Rₜ = 2.04 min; MS (ESIpos): *m*/*z* (%) = 417.0 (40), 460.0 (100) [M+H]⁺.
¹H-NMR (400 MHz, DMSO-d₆): δ = 1.85 (s, 3H), 3.25 (s, 3H), 4.85 (s, 1H), 6.65 (s, 1H), 7.70-8.40 (m, 8H).
Chirale analytische HPLC [Säule: Chiralpak AD-H, 5 µm, 250 mm x 4.6 mm; Eluent: *iso*-Hexan/ Ethanol 3:7; Fluss: 1 ml/min; Injektionsvolumen: 10 µl; Temperatur: 40°C; Detektion: 220 nm]: Rₜ = 4.16 min.

### Beispiel 41

### (S_{S})-(4S)-4-[4-Cyano-2-(S-methylsulfonimidoyl)phenyl]-6-methyl-2-oxo-1-[3-(trifluormethyl)-phenyl]-1,2,3,4-tetrahydropyrimidin-5-carbonitril oder (R_{S})-(4S)-4-[4-Cyano-2-(S-methylsulfonimidoyl)phenyl]-6-methyl-2-oxo-1-[3-(trifluormethyl)-phenyl]-1,2,3,4-tetrahydropyrimidin-5-carbonitril (Diastereomer 2)

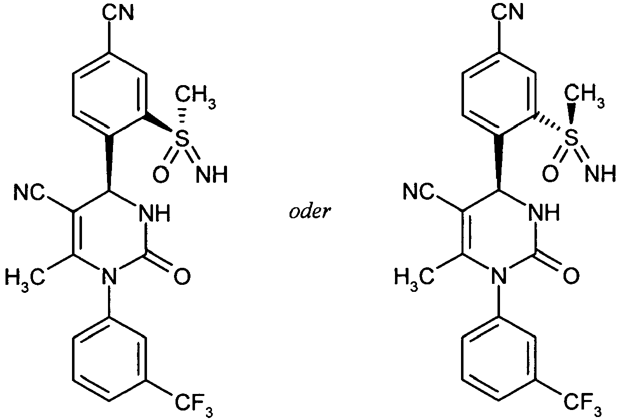

Das Diastereomerengemisch von (*R_{S}*)-(4*S*)-4-[4-Cyano-2-(*S*-methylsulfonimidoyl)phenyl]-6-methyl-2-oxo-1-[3-(trifluormethyl)phenyl]-1,2,3,4-tetrahydropyrimidin-5-carbonitril und (*S_{S}*)-(4*S*)-4-[4-Cyano-2-(*S*-methylsulfonimidoyl)phenyl]-6-methyl-2-oxo-1-[3-(trifluormethyl)phenyl]-1,2,3,4-tetrahydropyrimidin-5-carbonitril (400 mg) wurde durch präparative HPLC-Chromatographie an chiraler Phase aufgetrennt [Säule: Daicel Chiralpak AD-H, 250 mm x 20 mm; Probenvorbereitung: Probe in 20 ml Ethanol gelöst; Injektionsvolumen: 0.750 ml; Eluent: *iso*-Hexan/Ethanol 3:7; Fluss: 15 ml/min; Temperatur: 40°C; Detektion: 220 nm]. Das Diastereomer 2 wurde als später eluierende Fraktion in Form eines Feststoffs erhalten (103 mg, 26% d. Th., Gehalt >98.5%).
LC-MS (Methode 6): Rₜ = 2.04 min; MS (ESIpos): *m*/*z* (%) = 417.0 (40), 460.0 (100) [M+H]⁺.
¹H-NMR (400 MHz, DMSO-d₆): δ = 1.85 (s, 3H), 3.30 (s, 3H), 4.55 (s, 1H), 6.70 (s, 1H), 7.70-8.30 (m, 7H), 8.40 (s, 1H).
Chirale analytische HPLC [Säule: Chiralpak AD-H, 5 µm, 250 mm x 4.6 mm; Eluent: *iso*-Hexan/ Ethanol 3:7; Fluss: 1 ml/min; Injektionsvolumen: 10 µl; Temperatur: 40°C; Detektion: 220 nm]: Rₜ = 4.94 min.

### Beispiel 42

### (R_{S})-(4S)-4-[4-Cyano-2-(S-methylsulfonimidoyl)phenyl]-6-methyl-3-(methylsulfonyl)-2-oxo-1-[3-(trifluormethyl)phenyl]-1,2,3,4-tetrahydropyrimidin-5-carbonitril und (S_{S})-(4S)-4-[4-Cyano-2-(S-methylsulfonimidoyl)phenyl]-6-methyl-3-(methylsulfonyl)-2-oxo-1-[3-(trifluormethyl)phenyl]-1,2,3,4-tetrahydropyrimidin-5-carbonitril (Diastereomerengemisch)

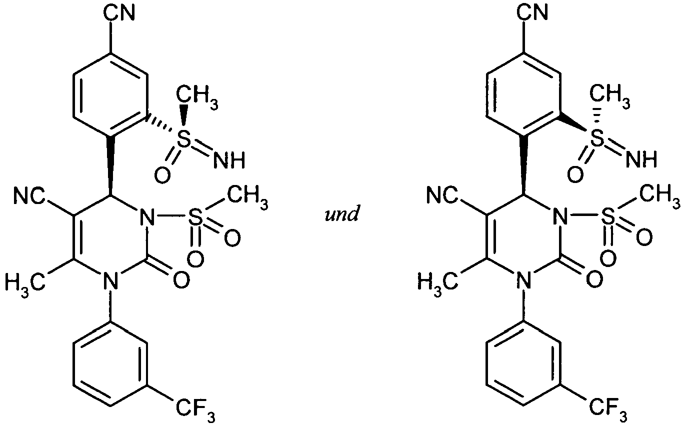

Die Reaktion wurde unter Argon durchgeführt. Das Diastereomerengemisch aus Beispiel 25A (25 mg, 39 µmol) wurde in einem Acetonitril/Methanol-Gemisch (10:1, 2.2 ml) vorgelegt. Bei 0°C wurde festes Kaliumcarbonat (2.7 mg, 20 µmol; 0.5 eq.) hinzugegeben und der Ansatz 15 min lang gerührt. Anschließend wurde mit Trifluoressigsäure (2.3 mg, 20 µmol; 0.5 eq.) neutralisiert, das Gemisch im Vakuum eingeengt und der Rückstand mittels präparativer HPLC gereinigt (Säule: Gromsil C-18, 10 µm; Eluent: Acetonitril/Wasser + 0.1% TFA 10:90 → 75:25). Die Titelverbindung wurde als ein farbloser Feststoff isoliert (4.3 mg, 20% d. Th.).
LC-MS (Methode 5): Rₜ = 1.84 min; MS (ESIpos): *m*/*z* (%) = 538.3 (100) [M+H]⁺; MS (ESIneg): *m*/*z* (%) = 415.3 (100), 536.3 (100) [M-H]⁻.

### B. Bewertung der pharmakologischen Wirksamkeit

Die pharmakologische Wirkung der erfindungsgemäßen Verbindungen kann in den nachstehend beschriebenen Assays gezeigt werden:

### Abkürzungen:

- AMC: 7-Amido-4-methylcumarin
- BNP: brain natriuretic peptide
- BSA: bovine serum albumin
- HEPES: *N*-(2-Hydroxyethyl)piperazin-*N*'-2-ethansulfonsäure
- HNE: humane neutrophile Elastase
- IC: Inhibitionskonzentration
- MeOSuc: Methoxysuccinyl
- NADP: Nikotinamid-Adenin-Dinukleotid-Phosphat
- PBS: Phosphat-gepufferte Kochsalz-Lösung
- PEG: Polyethylenglykol
- v/v: Volumen zu Volumen-Verhältnis (einer Lösung)
- w/v: Gewicht zu Volumen-Verhältnis (einer Lösung)

### B-1. In vitro HNE-Inhibitionstest

Die Wirkstärke der erfindungsgemäßen Verbindungen wird in einem *in vitro*-Hemmtest ermittelt. Die HNE-vermittelte amidolytische Spaltung eines geeigneten Peptidsubstrates führt hierbei zu einer Fluoreszenzlichtzunahme. Die Signalintensität des Fluoreszenzlichtes ist direkt proportional zur Enzymaktivität. Die Wirkkonzentration einer Testverbindung, bei der die Hälfte des Enzyms inhibiert ist (50% Signalintensität des Fluoreszenzlichtes), wird als IC₅₀-Wert angegeben.

### Ausführung:

In einer 384 Loch-Mikrotiterplatte werden in einem Testvolumen von insgesamt 50 µl Testpuffer (0.1 M HEPES pH 7.4, 0.5 M NaCl, 0.1% w/v BSA, 1% v/v DMSO), Enzym (80 pM HNE; Fa. Serva, Heidelberg) und Substrat (20 µM MeOSuc-Ala-Ala-Pro-Val-AMC; Fa. Bachem, Weil am Rhein) bei An- und Abwesenheit der Testsubstanz zwei Stunden bei 37°C inkubiert. Die Fluoreszenzlichtintensität der Testansätze wird gemessen (Ex. 380 nm, Em. 460 nm). Die IC₅₀-Werte werden durch eine Auftragung der Fluoreszenzlichtintensität gegenüber der Wirkstoffkonzentration ermittelt.

Für die erfindungsgemäßen Verbindungen repräsentative IC₅₀-Werte (bei einer HNE-Konzentration von 80 pM) sind in der folgenden Tabelle A wiedergegeben:

**Tabelle A: Hemmung der humanen neutrophilen Elastase (HNE)**

| **Ausführungsbeispiel Nr.** | **IC₅₀ [nM]** |
|---|---|
| 1 | 0.4 |
| 2 | < 0.3 |
| 3 | < 0.3 |
| 4 | < 0.3 |
| 10 | < 0.3 |
| 11 | < 0.3 |
| 13 | < 0.3 |
| 14 | 4.5 |
| 21 | 0.7 |
| 37 | < 0.3 |
| 38 | < 0.3 |
| 41 | 0.9 |

### B-2. Tiermodell der pulmonalen arteriellen Hypertonie

Die Monocrotalin-induzierte pulmonale Hypertonie der Ratte ist ein weit verbreitetes Tiermodell für die pulmonale arterielle Hypertonie. Das Pyrrolizidin-Alkaloid Monocrotalin wird nach subkutaner Injektion in der Leber zum toxischen Monocrotalinpyrrol metabolisiert und führt innerhalb weniger Tage zu einer Endothelschädigung im Lungenkreislauf, gefolgt von einem Remodeling der kleinen pulmonalen Arterien (Mediahypertrophie, *de novo*-Muskularisierung). Eine einmalige subkutane Injektion ist ausreichend, um bei Ratten innerhalb von 4 Wochen eine ausgeprägte pulmonale Hypertonie zu induzieren [Cowan et al., Nature Med. 6, 698-702 (2000)].

Für das Modell werden männliche Sprague-Dawley-Ratten verwendet. An Tag 0 erhalten die Tiere eine subkutane Injektion von 60 mg/kg Monocrotalin. Die Behandlung der Tiere beginnt erst frühestens 14 Tage nach der Monocrotalin-Injektion und erstreckt sich über einen Zeitraum von mindestens 14 Tagen. Am Studienende erfolgen hämodynamische Untersuchungen der Tiere sowie eine Ermittlung der arteriellen und zentralvenösen Sauerstoffsättigung. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver endexspiratorischer Druck: 1 cm H₂O; Atemzugvolumen: 10 ml/kg Körpergewicht; FIO₂: 0.5). Die Narkose wird durch Isofluran-Inhalationsnarkose aufrechterhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldruckes. Das Herzminutenvolumen wird mittels Thermodilution ermittelt. Im Anschluß an die Hämodynamik wird das Herz entnommen und das Verhältnis rechter zu linker Ventrikel inklusive Septum bestimmt. Weiterhin werden Plasmaproben zur Bestimmung von Biomarkern (zum Beispiel proBNP) und Plasma-Substanzspiegeln gewonnen.

### B-3. Tiermodell des akuten Lungenversagens

Elastase-induziertes Lungenversagen an Maus, Ratte oder Hamster ist ein weit verbreitetes Tiermodell für akutes Lungenversagen (auch: "acute lung injury", "acute respiratory distress syndrome") [Tremblay et al., Chest 121, 582-588 (2002); Kuraki et al., Am. J. Resp. Crit. Care Med. 166, 596-500 (2002)]. Die Behandlung der Tiere erfolgt 1 Stunde vor orotrachealer Instillation der humanen neutrophilen Elastase (HNE). 2 Stunden nach der orotrachealen HNE-Instillation wird eine bronchoalveolare Lavage durchgeführt und der Hämoglobingehalt sowie das Differentialzellbild in der Lavage bestimmt.

### B-4. Tiermodell des Lungenemphysems

Elastase-induziertes Lungenemphysem an Maus, Ratte oder Hamster ist ein weit verbreitetes Tiermodell für Lungenemphysem [Sawada et al., Exp. Lung Res. 33, 277-288 (2007)]. Die Tiere erhalten eine orotracheale Instillation porciner Pankreas-Elastase. Die Behandlung der Tiere beginnt am Tag der Instillation der porcinen Pankreas-Elastase und erstreckt sich über einen Zeitraum von 3 Wochen. Am Studienende wird die Lungen-Compliance bestimmt und eine Alveolarmorphometrie durchgeführt.

### B-5. CYP-Inhibitionstest

Die Fähigkeit von Substanzen, CYP1A2, CYP2C9, CYP2D6 und CYP3A4 im Menschen inhibieren zu können, wird untersucht mit gepoolten Human-Lebermikrosomen als Enzymquelle in Gegenwart von Standardsubstraten (s.u.), die CYP-spezifische Metaboliten bilden. Die Inhibitionseffekte werden bei sechs verschiedenen Konzentrationen der Testverbindungen untersucht [2.8, 5.6, 8.3, 16.7, 20 (oder 25) sowie 50 µM], mit dem Ausmaß der CYP-spezifischen Metabolitenbildung der Standardsubstrate in Abwesenheit der Testverbindungen verglichen und die entsprechenden IC₅₀-Werte berechnet. Ein Standard-Inhibitor, der eine einzelne CYP-Isoform spezifisch inhibiert, wird immer mit inkubiert, um Ergebnisse zwischen verschiedenen Serien vergleichbar zu machen.

### Durchführung:

Die Inkubation von Phenacetin, Diclofenac, Tolbutamid, Dextromethorphan oder Midazolam mit Human-Lebermikrosomen in Gegenwart von jeweils sechs verschiedenen Konzentrationen einer Testverbindung (als potentiellem Inhibitor) wird auf einer Workstation durchgeführt (Tecan, Genesis, Crailsheim, Deutschland). Standard-Inkubationsgemische enthalten 1.3 mM NADP, 3.3 mM MgCl₂ x 6 H₂O, 3.3 mM Glukose-6-phosphat, Glukose-6-phosphat-Dehydrogenase (0.4 U/ml) und 100 mM Phosphat-Puffer (pH 7.4) in einem Gesamtvolumen von 200 µl. Testverbindungen werden bevorzugt in Acetonitril gelöst. 96-Lochplatten werden eine definierte Zeit bei 37°C mit gepoolten Human-Lebermikrosomen inkubiert. Die Reaktionen werden durch Zugabe von 100 µl Acetonitril, worin sich ein geeigneter interner Standard befindet, abgestoppt. Gefällte Proteine werden durch Zentrifugation abgetrennt, die Überstände werden vereinigt und mittels LC-MS/MS analysiert.

### B-6. Hepatozytenassay zur Bestimmung der metabolischen Stabilität

Die metabolische Stabilität von Testverbindungen gegenüber Hepatozyten wird bestimmt, indem die Verbindungen bei niedrigen Konzentrationen (bevorzugt unter oder um 1 µM) und bei niedrigen Zellzahlen (bevorzugt bei 1 * 10⁶ Zellen/ml) inkubiert werden, um möglichst lineare kinetische Bedingungen im Versuch sicherzustellen. Sieben Proben aus der Inkubationslösung werden in einem festgelegten Zeitraster für die LC-MS-Analytik entnommen, um die Halbwertszeit (d.h. den Abbau) der jeweiligen Verbindung zu bestimmen. Aus dieser Halbwertszeit werden unterschiedliche "Clearance"-Parameter (CL) und "Fₘₐₓ"- Werte berechnet (s.u.).

Die CL- und Fₘₐₓ-Werte stellen ein Maß für den Phase 1- und Phase 2-Metabolismus der Verbindungen in den Hepatozyten dar. Um den Einfluss des organischen Lösungsmittels auf die Enzyme in den Inkubationsansätzen möglichst klein zu halten, wird dessen Konzentration im Allgemeinen auf 1% (Acetonitril) bzw. 0.1% (DMSO) begrenzt.

Für alle Spezies und Rassen wird mit einer Hepatozyten-Zellzahl in der Leber von 1.1 * 10⁸ Zellen/g Leber gerechnet. CL-Parameter, deren Berechnung auf Halbwertszeiten beruhen, die wesentlich über die Inkubationszeit hinausgehen (üblicherweise 90 Minuten), können nur als grobe Richtwerte angesehen werden.

Die berechneten Parameter und deren Bedeutung sind:

| | |
|---|---|
| **Fₘₐₓ well-stirred [%]** | maximal mögliche Bioverfügbarkeit nach oraler Applikation |
| *Berechnung:* | (1-CL_{blood} well-stirred/QH) * 100 |
| **CL_{blood} well-stirred [L/(h*kg)]** | berechnete Blut-Clearance (well stirred-Modell) |
| *Berechnung* | (QH * CL'_{intrinsic}) / (QH + CL'_{intrinsic}) |
| **CL'_{intrinsic} [ml/(min*kg)]** | maximale Fähigkeit der Leber (der Hepatozyten), eine Verbindung zu metabolisieren (unter der Annahme, dass der Leberblutfluss nicht geschwindigkeitslimitierend ist) |
| *Berechnung:* | CL'_{intrinsic,apparent} * speziesspezifische Hepatozytenzahl [1.1 * 10⁸/g Leber] * speziesspezifisches Lebergewicht [g/kg] |
| **CL'_{intrinsic, apparent} [ml/(min*mg)]** | normiert die Eliminationskonstante, indem diese durch die eingesetzte Hepatozyten-Zellzahl x (x * 10⁶/ml) dividiert wird |
| *Berechnung:* | kₑₗ [1/min] / (Zellzahl [x * 10⁶] / Inkubationsvolumen [ml]) |

| | |
|---|---|
| (QH = speziesspezifischer Leberblutfluss). | |

Für die erfindungsgemäßen Verbindungen repräsentative Werte aus diesem Assay nach Inkubation der Verbindungen mit Ratten-Hepatozyten sind in der folgenden Tabelle B wiedergegeben:

**Tabelle B: berechnete Blut-Clearance und Bioverfügbarkeit nach Inkubation mit Ratten-Hepatozyten**

| **Ausführungsbeispiel Nr.** | **CL_{blood} [L/(h*kg)]** | **Fₘₐₓ [%]** |
|---|---|---|
| 1 | 0.1 | 97 |
| 2 | 1.9 | 54 |
| 13 | 1.9 | 54 |
| 38 | 0.0 | 99 |
| 41 | 0.0 | 100 |

### B-7. Bestimmung der Löslichkeit

### Benötigte Reagenzien:

- PBS-Puffer pH 6.5: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1 N Natronlauge (z.B. Fa. Bernd Kraft GmbH, Art.-Nr. 01030.4000) werden in einen 1 Liter-Messkolben eingewogen, mit destilliertem Wasser auf 1 Liter aufgefüllt und für 1 Stunde gerührt. Danach wird mit 1 N Salzsäure (z.B. Fa. Merck, Art.-Nr. 1.09057.1000) der pH-Wert auf 6.5 eingestellt.
- PEG/Wasser-Lösung (70:30 v/v): 70 ml Polyethylenglykol 400 (z.B. Fa. Merck, Art.-Nr. 8.17003.1000) und 30 ml destilliertes Wasser werden in einem 100 ml-Messkolben homogenisiert.
- PEG/PBS-Puffer pH 6.5 (20:80 v/v): 20 ml Polyethylenglykol 400 (z.B. Fa. Merck, Art.-Nr. 8.17003.1000) und 80 ml PBS-Puffer pH 6.5 werden in einem 100 ml-Messkolben homogenisiert.
- Dimethylsulfoxid (z.B. Fa. Baker, Art.-Nr. 7157.2500)
- destilliertes Wasser.

### Herstellung der Ausgangslösung (Urlösung):

Mindestens 4 mg der Testsubstanz werden in ein Weithals-10 mm Screw V-Vial (Fa. Glastechnik Gräfenroda GmbH, Art.-Nr. 8004-WM-H/V15µ) mit passender Schraubkappe und Septum genau eingewogen, in einem Pipettierroboter mit DMSO bis zu einer Konzentration von 50 mg/ml versetzt und 10 Minuten geschüttelt.

### Herstellung der Kalibrierlösungen:

*Herstellung der Ausgangslösung für Kalibrierlösungen (Stammlösung):* In eine Mikrotiterplatte werden 10 µl der Urlösung mit Hilfe eines Pipettierroboters überführt und mit DMSO bis zu einer Konzentration von 600 µg/ml aufgefüllt. Die Probe wird bis zu ihrer vollständigen Lösung geschüttelt.

*Kalibrierlösung 1 (20 µglml):* 34.4 µl der Stammlösung werden mit 1000 µl DMSO versetzt und homogenisiert.

*Kalibrierlösung 2 (2.5 µg*/*ml):* 100 µl der Kalibrierlösung 1 werden mit 700 µl DMSO versetzt und homogenisiert.

### Herstellung der Probenlösungen:

*Probenlösung für Löslichkeit bis 5 g*/*Liter in PBS-Puffer pH 6.5:* In eine Mikrotiterplatte werden 10 µl Urlösung transferiert und mit 1000 µl PBS-Puffer pH 6.5 versetzt.

*Probenlösung für Löslichkeit bis 5 g*/*Liter in PEGlWasser (70:30):* In eine Mikrotiterplatte werden 10 µl Urlösung transferiert und mit 1000 µl PEG/Wasser (70:30) versetzt.

*Probenlösung für Löslichkeit bis 5 g*/*Liter in PEG*/*PBS-Puffer pH 6.5 (20:80):* In eine Mikrotiterplatte werden 10 µl Urlösung transferiert und mit 1000 µl PEG/PBS-Puffer pH 6.5 (20:80) versetzt.

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und mit DMSO zu 1:5 und 1:100 verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/Liter ausgedrückt. Analysensequenz: 1) Kalibrierlösung 2.5 mg/ml; 2) Kalibrierlösung 20 µg/ml; 3) Probenlösung 1:5; 4) Probenlösung 1:100.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 50 mm x 2 mm, 5 µ; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 60 mm x 2.1 mm, 3.5 µ; Temperatur: 30°C; Eluent A: Wasser + 5 ml Perchlorsäure/Liter; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

In der folgenden Tabelle C ist die nach diesem Verfahren bestimmte Löslichkeit erfindungsgemäßer Verbindungen in PBS-Puffer bei pH 6.5 wiedergegeben:

**Tabelle C: Löslichkeit in PBS-Puffer pH 6.5**

| **Ausführungsbeispiel Nr.** | **Löslichkeit [mg/Liter]** |
|---|---|
| 11 | 300 |
| 38 | 300 |

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 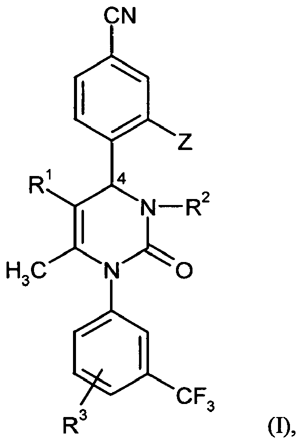 in welcher
Z für eine Sulfonamid-Gruppierung der Formel 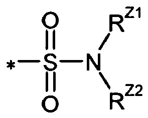 oder für eine Sulfoximin-Gruppierung der Formel 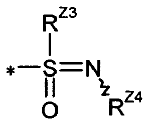 steht, worin
* die Verknüpfungsstelle mit dem Phenyl-Ring kennzeichnet,
R^{Z1} Wasserstoff oder (C₁-C₆)-Alkyl, das mit Hydroxy, (C₁-C₄)-Alkoxy, Amino, Mono- oder Di-(C₁-C₄)-alkylamino sowie bis zu dreifach mit Fluor substituiert sein kann, bedeutet,
R^{Z2} Wasserstoff, (C₃-C₆)-Cycloalkyl, 4- bis 6-gliedriges Heterocyclyl oder 5- oder 6-gliedriges Heteroaryl bedeutet
oder
(C₁-C₆)-Alkyl bedeutet, das mit Hydroxy, (C₁-C₄)-Alkoxy, Amino, Mono- oder Di-(C₁-C₄)-alkylamino, (C₁-C₄)-Alkylcarbonylamino, (C₁-C₄)-Alkoxycarbonylamino, (C₁-C₄)-Alkylsulfinyl, (C₁-C₄)-Alkylsulfonyl, (C₃-C₆)-Cycloalkyl, Phenyl, 4- bis 6-gliedrigem Heterocyclyl, 5- oder 6-gliedrigem Heteroaryl oder einer Gruppe der Formel -C(=O)-NR^{Z5}R^{Z6} sowie bis zu dreifach mit Fluor substituiert sein kann,
wobei der genannte Alkoxy-Substituent seinerseits bis zu dreifach mit Fluor substituiert sein kann,
und wobei
die genannten Heterocyclyl-Gruppen bis zu zweifach, gleich oder verschieden, mit Fluor, (C₁-C₄)-Alkyl, Oxo, Hydroxy, (C₁-C₄)-Alkoxy, Amino, Mono- und Di-(C₁-C₄)-alkylamino substituiert sein können
sowie
die genannte Phenyl-Gruppe und die genannten Heteroaryl-Gruppen bis zu zweifach, gleich oder verschieden, mit Fluor, Chlor, Cyano, (C₁-C₄)-Alkyl, Difluormethyl, Trifluormethyl und (C₁-C₄)-Alkoxy substituiert sein können,
und worin
R^{Z5} und R^{Z6} gleich oder verschieden sind und unabhängig voneinander Wasserstoff oder (C₁-C₄)-Alkyl darstellen
oder
R^{Z5} und R^{Z6} zusammen mit dem Stickstoffatom, an das sie gebunden sind, einen 4- bis 6-gliedrigen Aza-Heterocyclus bilden, der ein weiteres Ring-Heteroatom aus der Reihe N, O oder S enthalten und mit (C₁-C₄)-Alkyl, Oxo, Hydroxy, (C₁-C₄)-Alkoxy, Amino, Mono- oder Di-(C₁-C₄)-alkylamino substituiert sein kann,
oder
R^{Z1} und R^{Z2} zusammen mit dem Stickstoffatom, an das sie gebunden sind, einen 4- bis 10-gliedrigen Aza-Heterocyclus bilden, der ein weiteres Ring-Heteroatom aus der Reihe N, O oder S enthalten und bis zu zweifach, gleich oder verschieden, mit Fluor, (C₁-C₄)-Alkyl, Oxo, Hydroxy, (C₁-C₄)-Alkoxy, Amino, Mono- und Di-(C₁-C₄)-alkylamino substituiert sein kann,
R^{Z3} (C₁-C₆)-Alkyl, das mit (C₃-C₆)-Cycloalkyl oder bis zu dreifach mit Fluor substituiert sein kann, oder Phenyl, das bis zu zweifach, gleich oder verschieden, mit Fluor, Chlor, Cyano, (C₁-C₄)-Alkyl, Difluormethyl und Trifluormethyl substituiert sein kann, oder (C₃-C₆)-Cycloalkyl bedeutet,
und
R^{Z4} Wasserstoff, (C₁-C₄)-Alkyl oder (C₃-C₆)-Cycloalkyl bedeutet,
R¹ für Cyano oder Acetyl steht,
R² für Wasserstoff, (C₁-C₄)-Alkyl oder (C₁-C₄)-Alkylsulfonyl, welche jeweils bis zu dreifach mit Fluor substituiert sein können, oder eine Gruppe der Formel -CH₂-C(=O)-NH-R⁴ steht, worin
R⁴ Wasserstoff, (C₁-C₄)-Alkyl, das mit (C₃-C₆)-Cycloalkyl oder bis zu dreifach mit Fluor substituiert sein kann, oder (C₃-C₆)-Cycloalkyl bedeutet,
und
R³ für Wasserstoff, Fluor oder Chlor steht,
sowie ihre Salze, Solvate und Solvate der Salze.

2. Verbindung der Formel (I) nach Anspruch 1, in welcher
Z für eine Sulfonamid-Gruppierung der Formel 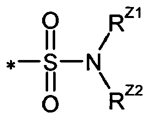 oder für eine Sulfoximin-Gruppierung der Formel 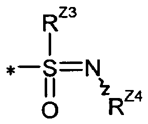 steht, worin
* die Verknüpfungsstelle mit dem Phenyl-Ring kennzeichnet,
R^{Z1} Wasserstoff oder (C₁-C₄)-Alkyl, das mit Hydroxy, Methoxy oder Ethoxy substituiert sein kann, bedeutet,
R^{Z2} Wasserstoff, (C₃-C₆)-Cycloalkyl, 5- oder 6-gliedriges Heterocyclyl oder 5- oder 6-gliedriges Heteroaryl bedeutet
oder
(C₁-C₄)-Alkyl bedeutet, das mit Hydroxy, (C₁-C₄)-Alkoxy, Amino, Mono- oder Di-(C₁-C₄)-alkylamino, (C₁-C₄)-Alkylcarbonylamino, (C₁-C₄)-Alkoxycarbonylamino, (C₁-C₄)-Alkylsulfinyl, (C₁-C₄)-Alkylsulfonyl, (C₃-C₆)-Cycloalkyl, Phenyl, 5- oder 6-gliedrigem Heterocyclyl, 5- oder 6-gliedrigem Heteroaryl oder einer Gruppe der Formel -C(=O)-NR^{Z5}R^{Z6} sowie bis zu dreifach mit Fluor substituiert sein kann,
wobei der genannte Alkoxy-Substituent seinerseits bis zu dreifach mit Fluor substituiert sein kann,
und wobei
die genannten Heterocyclyl-Gruppen bis zu zweifach, gleich oder verschieden, mit (C₁-C₄)-Alkyl, Oxo, Hydroxy und (C₁-C₄)-Alkoxy substituiert sein können
sowie
die genannte Phenyl-Gruppe und die genannten Heteroaryl-Gruppen bis zu zweifach, gleich oder verschieden, mit Fluor, Chlor, Cyano, (C₁-C₄)-Alkyl, Trifluormethyl und (C₁-C₄)-Alkoxy substituiert sein können,
und worin
R^{Z5} und R^{Z6} gleich oder verschieden sind und unabhängig voneinander Wasserstoff oder (C₁-C₄)-Alkyl darstellen
oder
R^{Z5} und R^{Z6} zusammen mit dem Stickstoffatom, an das sie gebunden sind, einen 5- oder 6-gliedrigen Aza-Heterocyclus bilden, der ein weiteres Ring-Heteroatom aus der Reihe N oder O enthalten und mit (C₁-C₄)-Alkyl, Oxo, Hydroxy oder (C₁-C₄)-Alkoxy substituiert sein kann,
oder
R^{Z1} und R^{Z2} zusammen mit dem Stickstoffatom, an das sie gebunden sind, einen 5- bis 10-gliedrigen Aza-Heterocyclus bilden, der ein weiteres Ring-Heteroatom aus der Reihe N oder O enthalten und bis zu zweifach, gleich oder verschieden, mit (C₁-C₄)-Alkyl, Oxo, Hydroxy und (C₁-C₄)-Alkoxy substituiert sein kann,
R^{Z3} (C₁-C₄)-Alkyl, das mit (C₃-C₆)-Cycloalkyl oder bis zu dreifach mit Fluor substituiert sein kann, oder Phenyl, das bis zu zweifach, gleich oder verschieden, mit Fluor, Chlor, Cyano, Methyl und Trifluormethyl substituiert sein kann, oder (C₃-C₆)-Cycloalkyl bedeutet,
und
R^{Z4} Wasserstoff, Methyl oder Cyclopropyl bedeutet,
R¹ für Cyano steht,
R² für Wasserstoff, (C₁-C₄)-Alkyl oder (C₁-C₄)-Alkylsulfonyl, welche jeweils bis zu dreifach mit Fluor substituiert sein können, oder eine Gruppe der Formel -CH₂-C(=O)-NH-R⁴ steht, worin
R⁴ Wasserstoff, Methyl, Cyclopropyl oder Cyclopropylmethyl bedeutet,
und
R³ für Wasserstoff oder Fluor steht,
sowie ihre Salze, Solvate und Solvate der Salze.

3. Verbindung der Formel (I) nach Anspruch 1 oder 2, in welcher
Z für eine Sulfonamid-Gruppierung der Formel 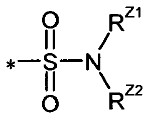 steht, worin
* die Verknüpfungsstelle mit dem Phenyl-Ring kennzeichnet,
R^{Z1} Wasserstoff, Methyl oder 2-Hydroxyethyl bedeutet,
R^{Z2} Wasserstoff, Cyclopropyl, 5- oder 6-gliedriges Heterocyclyl oder 5- oder 6-gliedriges Heteroaryl bedeutet
oder
(C₁-C₄)-Alkyl bedeutet, das mit Hydroxy, Methoxy, Ethoxy, Amino, Methylamino, Ethylamino, Dimethylamino, Diethylamino, Acetylamino, Cyclopropyl, 5- oder 6-gliedrigem Heterocyclyl oder einer Gruppe der Formel -C(=O)-NR^{Z5}R^{Z6} substituiert sein kann,
wobei die genannten Methoxy- und Ethoxy-Substituenten ihrerseits bis zu dreifach mit Fluor substituiert sein können,
und wobei
die genannten Heterocyclyl-Gruppen bis zu zweifach, gleich oder verschieden, mit Methyl, Ethyl, Oxo, Hydroxy, Methoxy und Ethoxy substituiert sein können
sowie
die genannte Heteroaryl-Gruppe bis zu zweifach, gleich oder verschieden, mit Fluor, Chlor, Cyano, Methyl, Ethyl, Trifluormethyl, Methoxy und Ethoxy substituiert sein kann,
und worin
R^{Z5} und R^{Z6} unabhängig voneinander Wasserstoff oder Methyl darstellen oder zusammen mit dem Stickstoffatom, an das sie gebunden sind, einen Pyrrolidin-, Piperidin- oder Morpholin-Ring bilden,
oder
R^{Z1} und R^{Z2} zusammen mit dem Stickstoffatom, an das sie gebunden sind, einen Pyrrolidin-, Piperidin- oder Morpholin-Ring bilden,
R¹ für Cyano steht,
R² für Wasserstoff, Methyl, Methylsulfonyl oder die Gruppe der Formel -CH₂-C(=O)-NH₂ steht,
und
R³ für Wasserstoff steht,
sowie ihre Salze, Solvate und Solvate der Salze.

4. Verbindung der Formel (I) nach Anspruch 1 oder 2, in welcher
Z für eine Sulfoximin-Gruppierung der Formel 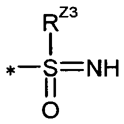 steht, worin
* die Verknüpfungsstelle mit dem Phenyl-Ring kennzeichnet
und
R^{Z3} (C₁-C₄)-Alkyl, das mit Cyclopropyl oder bis zu dreifach mit Fluor substituiert sein kann, oder Cyclopropyl bedeutet,
R¹ für Cyano steht,
R² für Wasserstoff, Methyl, Methylsulfonyl oder die Gruppe der Formel -CH₂-C(=O)-NH₂ steht,
und
R³ für Wasserstoff steht,
sowie ihre Salze, Solvate und Solvate der Salze.

5. Verbindung der Formel (I) nach Anspruch 1, 2 oder 3, in welcher
Z für eine Sulfonamid-Gruppierung der Formel 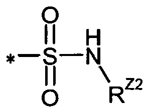 steht, worin
* die Verknüpfungsstelle mit dem Phenyl-Ring kennzeichnet
und
R^{Z2} Wasserstoff, Methyl oder die Gruppe der Formel -CH₂-C(=O)-NH₂ bedeutet,
R¹ für Cyano steht,
R² für Wasserstoff, Methyl oder Methylsulfonyl steht,
und
R³ für Wasserstoff steht,
sowie ihre Salze, Solvate und Solvate der Salze.

6. Verbindung der Formel (I) nach Anspruch 1, 2 oder 4, in welcher
Z für eine Sulfoximin-Gruppierung der Formel 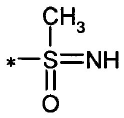 steht, worin
* die Verknüpfungsstelle mit dem Phenyl-Ring kennzeichnet,
R¹ für Cyano steht,
R² für Wasserstoff, Methyl oder Methylsulfonyl steht,
und
R³ für Wasserstoff steht,
sowie ihre Salze, Solvate und Solvate der Salze.

7. Verfahren zur Herstellung von Verbindungen der Formel (I), in welcher
Z für eine Sulfonamid-Gruppierung der Formel 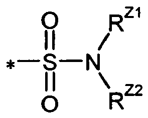 steht, worin
* die Verknüpfungsstelle mit dem Phenyl-Ring kennzeichnet
und
R^{Z1} und R^{Z2} die in den Ansprüchen 1, 2, 3 und 5 angegebenen Bedeutungen haben,
**dadurch gekennzeichnet, dass** man ein Anilin-Derivat der Formel (II) 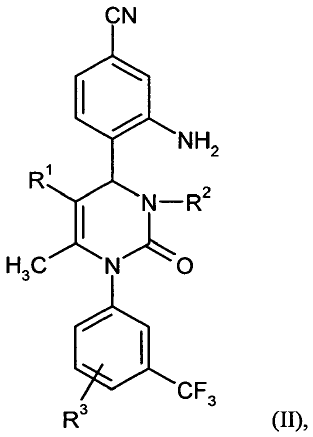 in welcher R¹, R² und R³ die in den Ansprüchen 1, 2, 3 und 5 angegebenen Bedeutungen haben,
zunächst mit Natriumnitrit und Salzsäure in das entsprechende Diazonium-Salz überführt, anschließend in einer Eintopf-Reaktion mit Schwefeldioxid in Gegenwart von Kupfer(I)-chlorid zu einem Sulfochlorid der Formel (III) 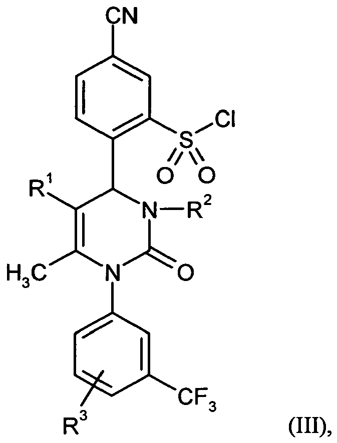 in welcher R¹, R² und R³ die oben angegebenen Bedeutungen haben,
umsetzt und dieses dann mit einem Amin der Formel (IV) 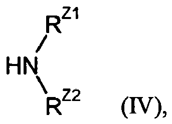 in welcher R^{Z1} und R^{Z2} die in den Ansprüchen 1, 2, 3 und 5 angegebenen Bedeutungen haben,
gegebenenfalls in Gegenwart einer Hilfsbase zum Sulfonamid der Formel (I-A) 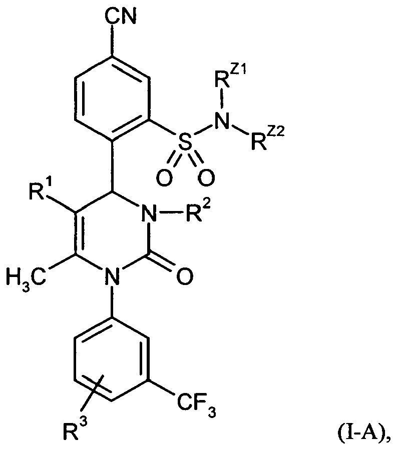 in welcher R¹, R², R³, R^{Z1} und R^{Z2} die oben angegebenen Bedeutungen haben,
reagiert
und gegebenenfalls die so erhaltenen Verbindungen der Formel (I-A) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

8. Verfahren zur Herstellung von Verbindungen der Formel (I), in welcher
Z für eine Sulfoximin-Gruppierung der Formel 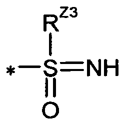 steht, worin
* die Verknüpfungsstelle mit dem Phenyl-Ring kennzeichnet
und
R^{Z3} die in den Ansprüchen 1, 2, 4 und 6 angegebene Bedeutung hat,
**dadurch gekennzeichnet, dass** man ein Phenylthioether-Derivat der Formel (V) 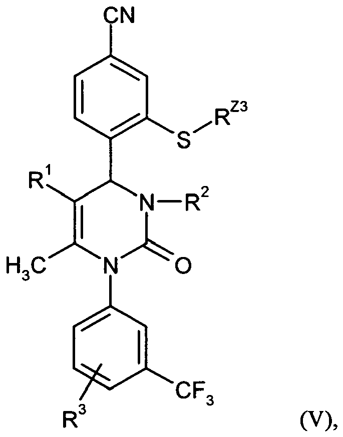 in welcher R¹, R², R³ und R^{Z3} die in den Ansprüchen 1, 2, 4 und 6 angegebenen Bedeutungen haben,
zunächst mit Wasserstoffperoxid, einer Persäure oder einem Periodat zum Sulfoxid der Formel (VI) 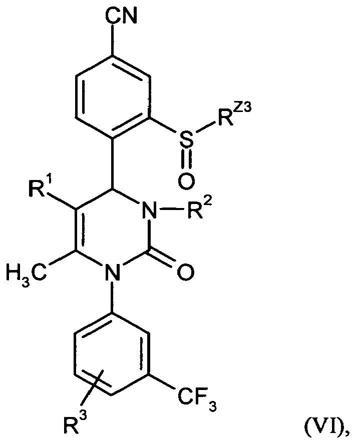 in welcher R¹, R², R³ und R^{Z3} die oben angegebenen Bedeutungen haben,
oxidiert, anschließend mit 2,2,2-Trifluoracetamid und (Diacetoxyiod)-benzol in Gegenwart von dimerem Rhodium(II)acetat als Katalysator und Magnesiumoxid als Base zu einem N-Acyl-Sulfoximin der Formel (VII) 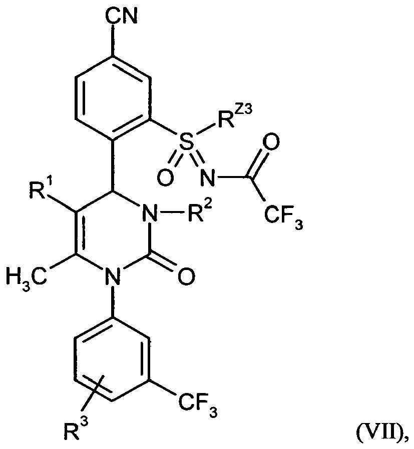 in welcher R¹, R², R³ und R^{Z3} die oben angegebenen Bedeutungen haben,
umsetzt und die Trifluoracetyl-Gruppe in (VII) dann unter basischen Bedingungen zum Sulfoximin der Formel (I-B) 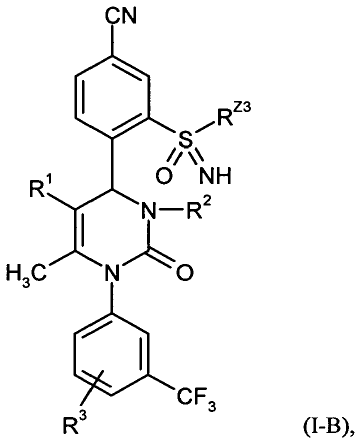 in welcher R¹, R², R³ und R^{Z3} die oben angegebenen Bedeutungen haben,
abspaltet
und gegebenenfalls die so erhaltenen Verbindungen der Formel (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

9. Verbindung, wie in einem der Ansprüche 1 bis 6 definiert, zur Behandlung und/oder Prävention von Krankheiten.

10. Verbindung, wie in einem der Ansprüche 1 bis 6 definiert, zur Verwendung in einem Verfahren zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

11. Verwendung einer Verbindung, wie in einem der Ansprüche 1 bis 6 definiert, zur Herstellung eines Arzneimittels zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

12. Arzneimittel enthaltend eine Verbindung, wie in einem der Ansprüche 1 bis 6 definiert, in Kombination mit einem oder mehreren inerten, nicht-toxischen, pharmazeutisch geeigneten Hilfsstoffen.

13. Arzneimittel enthaltend eine Verbindung, wie in einem der Ansprüche 1 bis 6 definiert, in Kombination mit einem oder mehreren weiteren Wirkstoffen ausgewählt aus der Gruppe bestehend aus Kinase-Inhibitoren, Matrixmetalloprotease-Inhibitoren, Stimulatoren und Aktivatoren der löslichen Guanylatcyclase, Prostacyclin-Analoga, Endothelinrezeptor-Antagonisten, Phosphodiesterase-Inhibitoren, beta-adrenerge Rezeptor-Agonisten, Anticholinergika und Glucocorticoide.

14. Arzneimittel nach Anspruch 12 oder 13 zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

## Claims

1. Compound of the formula (I) 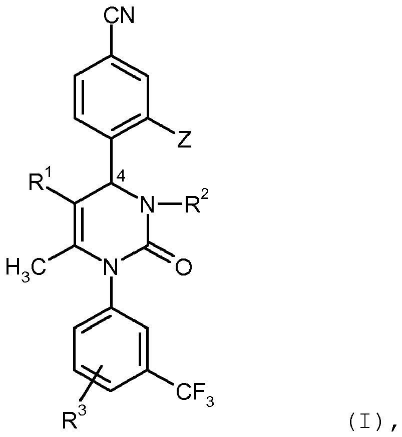 in which
Z represents a sulfonamide grouping of the formula 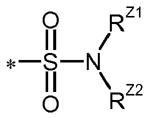 or represents a sulfoximine grouping of the formula 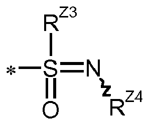 in which
* denotes the point of attachment to the phenyl ring,
R^{Z1} represents hydrogen, or represents (C₁-C₆)-alkyl which may be substituted by hydroxyl, (C₁-C₄) -alkoxy, amino, mono- or di-(C₁-C₄)-alkylamino and up to three times by fluorine,
R^{Z2} represents hydrogen, (C₃-C₆)-cycloalkyl, 4- to 6-membered heterocyclyl or 5- or 6-membered heteroaryl
or
represents (C₁-C₆)-alkyl which may be substituted by hydroxyl, (C₁-C₄) -alkoxy, amino, mono- or di-(C₁-C₄)-alkylamino, (C₁-C₄) -alkylcarbonylamino, (C₁-C₄) -alkoxycarbonylamino, (C₁-C₄) -alkylsulfinyl, (C₁-C₄) -alkylsulfonyl, (C₃-C₆)-cycloalkyl, phenyl, 4- to 6-membered heterocyclyl, 5- or 6-membered heteroaryl or a group of the formula -C(=O)-NR^{Z5}R^{Z6} and up to three times by fluorine, where the alkoxy substituent mentioned for its part may be substituted up to three times by fluorine,
and where
the heterocyclyl groups mentioned may be substituted up to two times by identical or different substituents from the group consisting of fluorine, (C₁-C₄)-alkyl, oxo, hydroxyl, (C₁-C₄)-alkoxy, amino, mono- and di-(C₁-C₄)-alkylamino
and
the phenyl group mentioned and the heteroaryl groups mentioned may be substituted up to two times by identical or different substituents from the group consisting of fluorine, chlorine, cyano, (C₁-C₄)-alkyl, difluoromethyl, trifluoromethyl and (C₁-C₄)-alkoxy,
and where
R^{Z5} and R^{Z6} are identical or different and independently of one another represent hydrogen or (C₁-C₄) -alkyl
or
R^{Z5} and R^{Z6} together with the nitrogen atom to which they are attached form a 4- to 6-membered aza heterocycle which may contain a further ring heteroatom from the group consisting of N, O and S and may be substituted by (C₁-C₄) -alkyl, oxo, hydroxyl, (C₁-C₄) -alkoxy, amino, mono- or di- (C₁-C₄)-alkylamino,
or
R^{Z1} and R^{Z2} together with the nitrogen atom to which they are attached form a 4- to 10-membered aza heterocycle which may contain a further ring heteroatom from the group consisting of N, O and S and may be substituted up to two times by identical or different substituents from the group consisting of fluorine, (C₁-C₄) -alkyl, oxo, hydroxyl, (C₁-C₄) -alkoxy, amino, mono- and di- (C₁-C₄) -alkylamino,
R^{Z3} represents (C₁-C₆)-alkyl which may be substituted by (C₃-C₆)-cycloalkyl or up to three times by fluorine, or represents phenyl which may be substituted up to two times by identical or different substituents from the group consisting of fluorine, chlorine, cyano, (C₁-C₄) -alkyl, difluoromethyl and trifluoromethyl, or represents (C₃-C₆)-cycloalkyl,
and
R^{Z4} represents hydrogen, (C₁-C₄)-alkyl or (C₃-C₆)-cycloalkyl,
R¹ represents cyano or acetyl,
R² represents hydrogen, represents (C₁-C₄)-alkyl or (C₁-C₄)-alkylsulfonyl which may be substituted up to three times by fluorine, or represents a group of the formula -CH₂-C(=O)-NH-R⁴ in which
R⁴ represents hydrogen, represents (C₁-C₄)-alkyl which may be substituted by (C₃-C₆)-cycloalkyl or up to three times by fluorine, or represents (C₃-C₆)-cycloalkyl,
and
R³ represents hydrogen, fluorine or chlorine,
and also the salts, solvates and solvates of the salts thereof.

2. Compound of the formula (I) according to Claim 1 in which
Z represents a sulfonamide grouping of the formula 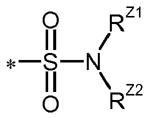 or represents a sulfoximine grouping of the formula 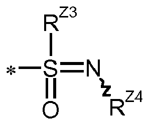 in which
* denotes the point of attachment to the phenyl ring,
R^{Z1} represents hydrogen or represents (C₁-C₄)-alkyl which may be substituted by hydroxyl, methoxy or ethoxy,
R^{Z2} represents hydrogen, (C₃-C₆)-cycloalkyl, 5- or 6-membered heterocyclyl or 5- or 6-membered heteroaryl
or
represents (C₁-C₄)-alkyl which may be substituted by hydroxyl, (C₁-C₄) -alkoxy, amino, mono- or di-(C₁-C₄)-alkylamino, (C₁-C₄) -alkylcarbonylamino, (C₁-C₄) -alkoxycarbonylamino, (C₁-C₄) -alkylsulfinyl, (C₁-C₄) -alkylsulfonyl, (C₃-C₆)-cycloalkyl, phenyl, 5- or 6-membered heterocyclyl, 5- or 6-membered heteroaryl or a group of the formula -C(=O)-NR^{Z5}R^{Z6} and up to three times by fluorine,
where the alkoxy substituent mentioned for its part may be substituted up to three times by fluorine,
and where
the heterocyclyl groups mentioned may be substituted up to two times by identical or different substituents from the group consisting of (C₁-C₄)-alkyl, oxo, hydroxyl and (C₁-C₄) -alkoxy
and
the phenyl group mentioned and the heteroaryl groups mentioned may be substituted up to two times by identical or different substituents from the group consisting of fluorine, chlorine, cyano, (C₁-C₄)-alkyl, trifluoromethyl and (C₁-C₄) -alkoxy,
and where
R^{Z5} and R^{Z6} are identical or different and independently of one another represent hydrogen or (C₁-C₄) -alkyl
or
R^{Z5} and R^{Z6} together with the nitrogen atom to which they are attached form a 5- or 6-membered aza heterocycle which may contain a further ring heteroatom from the group consisting of N and O and may be substituted by (C₁-C₄)-alkyl, oxo, hydroxyl or (C₁-C₄) -alkoxy,
or
R^{Z1} and R^{Z2} together with the nitrogen atom to which they are attached form a 5- to 10-membered aza heterocycle which may contain a further ring heteroatom from the group consisting of N and O and may be substituted up to two times by identical or different substituents from the group consisting of (C₁-C₄) -alkyl, oxo, hydroxyl and (C₁-C₄) -alkoxy,
R^{Z3} represents (C₁-C₄)-alkyl which may be substituted by (C₃-C₆)-cycloalkyl or up to three times by fluorine, or represents phenyl which may be substituted up to two times by identical or different substituents from the group consisting of fluorine, chlorine, cyano, methyl and trifluoromethyl, or represents (C₃-C₆)-cycloalkyl,
and
R^{Z4} represents hydrogen, methyl or cyclopropyl,
R¹ represents cyano,
R² represents hydrogen, (C₁-C₄)-alkyl or (C₁-C₄)-alkylsulfonyl, each of which may be substituted up to three times by fluorine, or represents a group of the formula -CH₂-C(=O)-NH-R⁴ in which
R⁴ represents hydrogen, methyl, cyclopropyl or cyclopropylmethyl,
and
R³ represents hydrogen or fluorine,
and also the salts, solvates and solvates of the salts thereof.

3. Compound of the formula (I) according to Claim 1 or 2 in which
Z represents a sulfonamide grouping of the formula 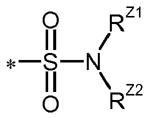 in which
* denotes the point of attachment to the phenyl ring,
R^{Z1} represents hydrogen, methyl or 2-hydroxyethyl,
R^{Z2} represents hydrogen, cyclopropyl, 5- or 6-membered heterocyclyl or 5- or 6-membered heteroaryl
or
represents (C₁-C₄)-alkyl which may be substituted by hydroxyl, methoxy, ethoxy, amino, methylamino, ethylamino, dimethylamino, diethylamino, acetylamino, cyclopropyl, 5- or 6-membered heterocyclyl or a group of the formula -C(=O)-NR^{Z5}R^{Z6},
where methoxy and ethoxy substituents mentioned for their part may be substituted up to three times by fluorine,
and where
the heterocyclyl groups mentioned may be substituted up to two times by identical or different substituents from the group consisting of methyl, ethyl, oxo, hydroxyl, methoxy and ethoxy
and
the heteroaryl group mentioned may be substituted up to two times by identical or different substituents from the group consisting of fluorine, chlorine, cyano, methyl, ethyl, trifluoromethyl, methoxy and ethoxy,
and where
R^{Z5} and R^{Z6} independently of one another represent hydrogen or methyl or together with the nitrogen atom to which they are attached form a pyrrolidine, piperidine or morpholine ring,
or
R^{Z1} and R^{Z2} together with the nitrogen atom to which they are attached form a pyrrolidine, piperidine or morpholine ring,
R¹ represents cyano,
R² represents hydrogen, methyl, methylsulfonyl or the group of the formula -CH₂-C(=O)-NH₂,
and
R³ represents hydrogen,
and also the salts, solvates and solvates of the salts thereof.

4. Compound of the formula (I) according to Claim 1 or 2 in which
Z represents a sulfoximine grouping of the formula 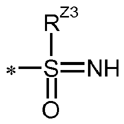 in which
* denotes the point of attachment to the phenyl ring
and
R^{Z3} represents (C₁-C₄)-alkyl which may be substituted by cyclopropyl or up to three times by fluorine, or represents cyclopropyl,
R¹ represents cyano,
R² represents hydrogen, methyl, methylsulfonyl or the group of the formula -CH₂-C(=O)-NH₂,
and
R³ represents hydrogen,
and also the salts, solvates and solvates of the salts thereof.

5. Compound of the formula (I) according to Claim 1, 2 or 3 in which
Z represents a sulfonamide grouping of the formula 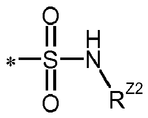 in which
* denotes the point of attachment to the phenyl ring
and
R^{Z2} represents hydrogen, methyl or the group of the formula -CH₂-C(=O)-NH₂,
R¹ represents cyano,
R² represents hydrogen, methyl or methylsulfonyl,
and
R³ represents hydrogen,
and also the salts, solvates and solvates of the salts thereof.

6. Compound of the formula (I) according to Claim 1, 2 or 4 in which
Z represents a sulfoximine grouping of the formula 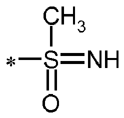 in which
* denotes the point of attachment to the phenyl ring,
R¹ represents cyano,
R² represents hydrogen, methyl or methylsulfonyl,
and
R³ represents hydrogen,
and also the salts, solvates and solvates of the salts thereof.

7. Process for preparing compounds of the formula (I) in which
Z represents a sulfonamide grouping of the formula 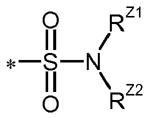 in which
* denotes the point of attachment to the phenyl ring
and
R^{Z1} and R^{Z2} have the meanings given in any of Claims 1, 2, 3 and 5,
**characterized in that** an aniline derivative of the formula (II) 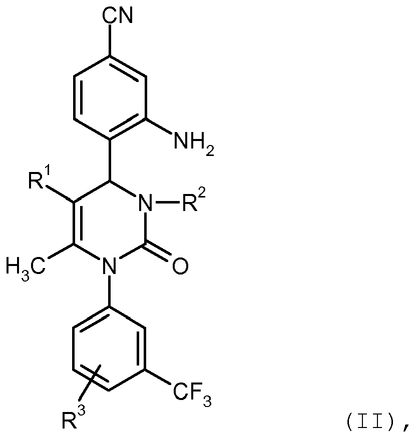 in which R¹, R² and R³ have the meanings given in any of Claims 1, 2, 3 and 5,
is initially converted with sodium nitrite and hydrochloric acid into the corresponding diazonium salt and then reacted in a one-pot reaction with sulfur dioxide in the presence of copper(I) - chloride to give a sulfonyl chloride of the formula (III) 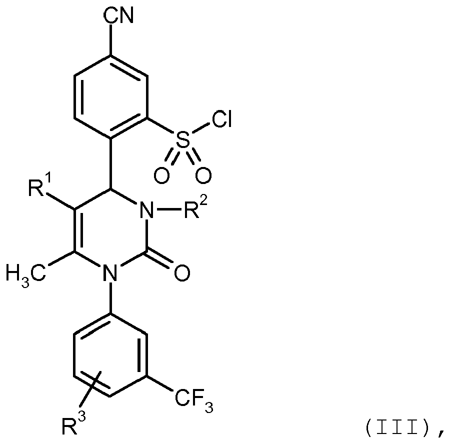 in which R¹, R² and R³ have the meanings given above,
and this is then reacted with an amine of the formula (IV) 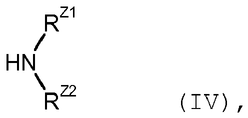 in which R^{Z1} and R^{Z2} have the meanings given in any of Claims 1, 2, 3 and 5,
if appropriate in the presence of an auxiliary base, to give the sulfonamide of the formula (I-A) 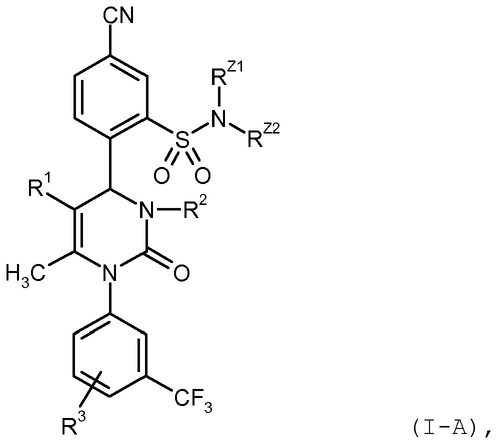 in which R¹, R², R³, R^{Z1} and R^{Z2} have the meanings given above,
and the compounds of the formula (I-A) obtained in this manner are, if appropriate, separated by methods known to the person skilled in the art into their enantiomers and/or diastereomers and/or converted with the appropriate (i) solvents and/or (ii) bases or acids into their solvates, salts and/or solvates of the salts.

8. Process for preparing compounds of the formula (I) in which
Z represents a sulfoximine grouping of the formula 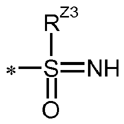 in which
* denotes the point of attachment to the phenyl ring
and
R^{Z3} has the meaning given in any of Claims 1, 2, 4 and 6,
**characterized in that** a phenyl thioether derivative of the formula (V) 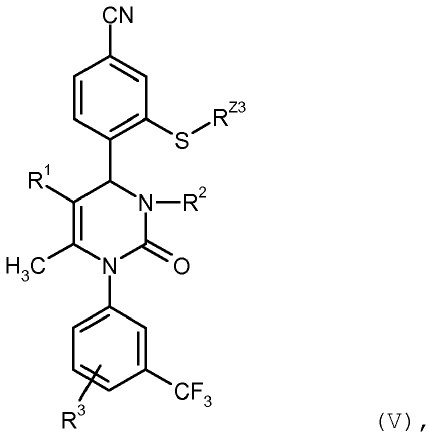 in which R¹, R², R³ and R^{Z3} have the meanings given in any of Claims 1, 2, 4 and 6,
is initially oxidized with hydrogen peroxide, a peracid or a periodate to give the sulfoxide of the formula (VI) 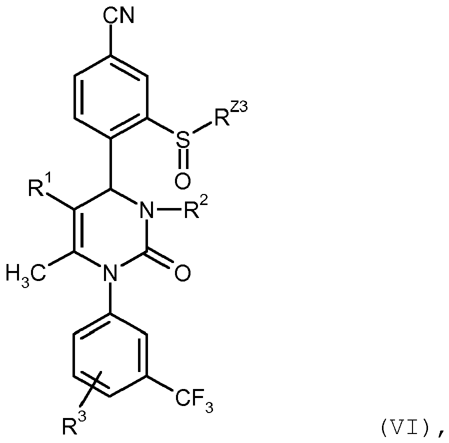 in which R¹, R², R³ and R^{Z3} have the meanings given above,
then converted with 2,2,2-trifluoroacetamide and (diacetoxyiodo)benzene in the presence of dimeric rhodium(II) acetate as catalyst and magnesium oxide as base into an N-acylsulfoximine of the formula (VII) 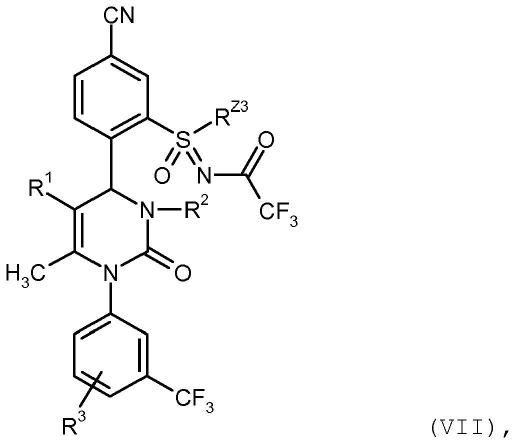 in which R¹, R², R³ and R^{Z3} have the meanings given above,
and the trifluoroacetyl group in (VII) is then removed under basic conditions to give the sulfoximine of the formula (I-B) 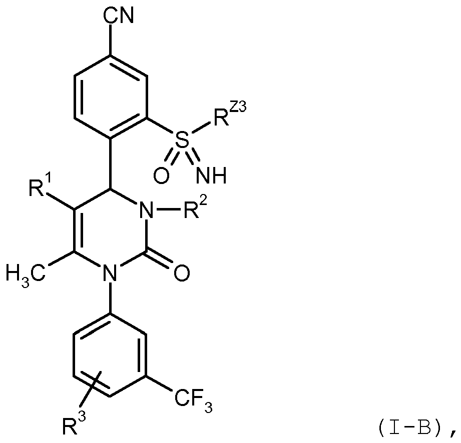 in which R¹, R², R³ and R^{Z3} have the meanings given above,
and the compounds of the formula (I-B) obtained in this manner are, if appropriate, separated by methods known to the person skilled in the art into their enantiomers and/or diastereomers and/or converted with the appropriate (i) solvents and/or (ii) bases or acids into their solvates, salts and/or solvates of the salts.

9. Compound as defined in any of Claims 1 to 6 for the treatment and/or prevention of diseases.

10. Compound as defined in any of Claims 1 to 6 for use in a method for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

11. Use of a compound as defined in any of Claims 1 to 6 for preparing a medicament for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

12. Medicament comprising a compound as defined in any of Claims 1 to 6 in combination with one or more inert non-toxic pharmaceutically acceptable auxiliaries.

13. Medicament comprising a compound as defined in any of Claims 1 to 6 in combination with one or more further active compounds selected from the group of the kinase inhibitors, matrix metalloprotease inhibitors, stimulators and activators of soluble guanylate cyclase, prostacyclin analogs, endothelin receptor antagonists, phosphodiesterase inhibitors, beta-adrenergic receptor agonists, anticholinergics and glucocorticoids.

14. Medicament according to Claim 12 or 13 for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

## Revendications

1. Composé de formule (I) 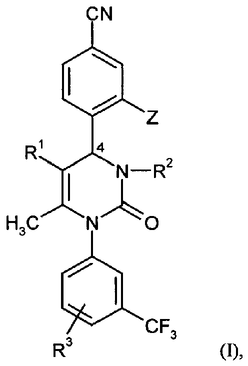 dans laquelle
Z représente un groupement sulfonamido de formule 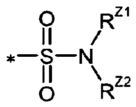 ou un groupement sulfoximine de formule 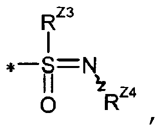 où
* désigne le point d'attachement au cycle phényle,
R^{Z1} représente un atome d'hydrogène ou un groupe alkyle en C₁-C₆, qui peut être substitué par hydroxy, alcoxy en C₁-C₄, amino, mono- ou dialkyl (C₁-C₄) amino ainsi que jusqu'à trois fois par fluoro,
R^{Z2} représente un atome d'hydrogène, un groupe cycloalkyle en C₃-C₆, hétérocyclyle à 4 à 6 chaînons ou hétéroaryle à 5 ou 6 chaînons
ou
représente un groupe alkyle en C₁-C₆ qui peut être substitué par hydroxy, alcoxy en C₁-C₄, amino, mono- ou dialkyl(C₁-C₄)amino, alkyl (C₁-C₄) carbonylamino, alcoxy (C₁-C₄) - carbonylamino, alkyl(C₁-C₄)sulfinyle, alkyl(C₁-C₄)sulfonyle, cycloalkyle en C₃-C₆, phényle, hétérocyclyle à 4 à 6 chaînons, hétéroaryle à 5 ou 6 chaînons ou par un groupe de formule -C(=O)-NR^{Z5}R^{Z6} ainsi que jusqu'à trois fois par fluoro,
ledit substituant alcoxy pouvant pour sa part être jusqu'à trois fois substitué par fluoro,
et
lesdits groupes hétérocyclyle pouvant porter jusqu'à deux substituants, identiques ou différents, fluoro, alkyle en C₁-C₄, oxo, hydroxy, alcoxy en C₁-C₄, amino, mono- et dialkyl (C₁-C₄) amino
ainsi que
ledit groupe phényle et lesdits groupes hétéroaryle pouvant porter jusqu'à deux substituants, identiques ou différents, fluoro, chloro, cyano, alkyle en C₁-C₄, difluorométhyle, trifluorométhyle et alcoxy en C₁-C₄,
et où
R^{Z5} et R^{Z6} sont identiques ou différents et représentent indépendamment l'un de l'autre un atome d'hydrogène ou un groupe alkyle en C₁-C₄
ou
R^{Z5} et R^{Z6} forment ensemble avec l'atome d'azote, auquel ils sont liés, un azahétérocycle à 4 à 6 chaînons, qui peut contenir un autre hétéroatome formant le cycle, choisi dans la série N, O et S, et être substitué par alkyle en C₁-C₄, oxo, hydroxy, alcoxy en C₁-C₄, amino, mono- ou dialkyl(C₁-C₄)amino,
ou
R^{Z1} et R^{Z2} forment ensemble avec l'atome d'azote, auquel ils sont liés, un aza-hétérocycle à 4 à 10 chaînons, qui peut contenir un autre hétéroatome formant le cycle, choisi dans la série N, O et S, et porter jusqu'à deux substituants, identiques ou différents, fluoro, alkyle en C₁-C₄, oxo, hydroxy, alcoxy en C₁-C₄, amino, mono- ou dialkyl (C₁-C₄) amino,
R^{Z3} représente un groupe alkyle en C₁-C₆, qui peut être substitué par cycloalkyle en C₃-C₆ ou jusqu'à trois fois par fluoro, ou un groupe phényle qui peut porter jusqu'à deux substituants, identiques ou différents, fluoro, chloro, cyano, alkyle en C₁-C₄, difluorométhyle et trifluorométhyle, ou un groupe cycloalkyle en C₃-C₆,
et
R^{Z4} représente un atome d'hydrogène, un groupe alkyle en C₁-C₄ ou cycloalkyle en C₃-C₆,
R¹ représente un groupe cyano ou acétyle,
R² représente un atome d'hydrogène, un groupe alkyle en C₁-C₄ ou alkyl (C₁-C₄) sulfonyle, qui peuvent être substitués chacun jusqu'à trois fois par fluoro, ou représente un groupe de formule -CH₂-C(=O)-NH-R⁴, dans laquelle
R⁴ représente un atome d'hydrogène, un groupe alkyle en C₁-C₄ qui peut être substitué par cycloalkyle en C₃-C₆ ou jusqu'à trois fois par fluoro, ou un groupe cycloalkyle en C₃-C₆,
et
R³ représente un atome d'hydrogène, de fluor ou de chlore,
ainsi que ses sels, produits de solvatation et produits de solvatation des sels.

2. Composé de formule (I) selon la revendication 1, dans lequel
Z représente un groupement sulfonamido de formule 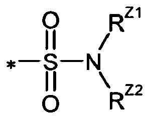 ou un groupement sulfoximine de formule 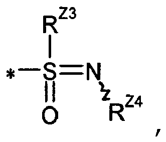 où
* désigne le point d'attachement au cycle phényle,
R^{Z1} représente un atome d'hydrogène ou un groupe alkyle en C₁-C₄, qui peut être substitué par hydroxy, méthoxy ou éthoxy,
R^{Z2} représente un atome d'hydrogène, un groupe cycloalkyle en C₃-C₆, hétérocyclyle à 5 ou 6 chaînons ou hétéroaryle à 5 ou 6 chaînons
ou
représente un groupe alkyle en C₁-C₄ qui peut être substitué par hydroxy, alcoxy en C₁-C₄, amino, mono- ou dialkyl(C₁-C₄)amino, alkyl(C₁-C₄)carbonylamino, alcoxy (C₁-C₄) - carbonylamino alkyl(C₁-C₄)sulfinyle, alkyl(C₁-C₄)sulfonyle, cycloalkyle en C₃-C₆, phényle, hétérocyclyle à 5 ou 6 chaînons, hétéroaryle à 5 ou 6 chaînons ou par un groupe de formule -C(=O)-NR^{Z5}R^{Z6} ainsi que jusqu'à trois fois par fluoro,
ledit substituant alcoxy pouvant pour sa part être jusqu'à trois fois substitué par fluoro,
et
lesdits groupes hétérocyclyle pouvant porter jusqu'à deux substituants, identiques ou différents, alkyle en C₁-C₄, oxo, hydroxy et alcoxy en C₁-C₄,
ainsi que
ledit groupe phényle et lesdits groupes hétéroaryle pouvant porter jusqu'à deux substituants, identiques ou différents, fluoro, chloro, cyano, alkyle en C₁-C₄, trifluorométhyle et alcoxy en C₁-C₄,
et où
R^{Z5} et R^{Z6} sont identiques ou différents et représentent indépendamment l'un de l'autre un atome d'hydrogène ou un groupe alkyle en C₁-C₄
ou
R^{Z5} et R^{Z6} forment ensemble avec l'atome d'azote, auquel ils sont liés, un azahétérocycle à 5 ou 6 chaînons, qui peut contenir un autre hétéroatome formant le cycle, choisi dans la série N et O, et être substitué par alkyle en C₁-C₄, oxo, hydroxy ou alcoxy en C₁-C₄,
ou
R^{Z1} et R^{Z2} forment ensemble avec l'atome d'azote, auquel ils sont liés, un aza-hétérocycle à 5 à 10 chaînons, qui peut contenir un autre hétéroatome formant le cycle, choisi dans la série N et O, et porter jusqu'à deux substituants, identiques ou différents, alkyle en C₁-C₄, oxo, hydroxy et alcoxy en C₁-C₄,
R^{Z3} représente un groupe alkyle en C₁-C₄, qui peut être substitué par cycloalkyle en C₃-C₆ ou jusqu'à trois fois par fluoro, ou un groupe phényle qui peut porter jusqu'à deux substituants, identiques ou différents, fluoro, chloro, cyano, méthyle et trifluorométhyle, ou un groupe cycloalkyle en C₃-C₆,
et
R^{Z4} représente un atome d'hydrogène, le groupe méthyle ou cyclopropyle,
R¹ représente le groupe cyano,
R² représente un atome d'hydrogène, un groupe alkyle en C₁-C₄ ou alkyl (C₁-C₄) sulfonyle, qui peuvent être substitués chacun jusqu'à trois fois par fluoro, ou représente un groupe de formule -CH₂-C(=O)-NH-R⁴, dans laquelle
R⁴ représente un atome d'hydrogène, le groupe méthyle, cyclopropyle ou cyclopropylméthyle,
et
R³ représente un atome d'hydrogène ou de fluor,
ainsi que ses sels, produits de solvatation et produits de solvatation des sels.

3. Composé de formule (I) selon la revendication 1 ou 2, dans lequel
Z représente un groupement sulfonamido de formule 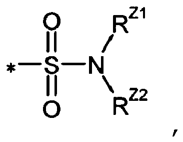 dans laquelle
* désigne le point d'attachement au cycle phényle,
R^{Z1} représente un atome d'hydrogène, le groupe méthyle ou 2-hydroxyéthyle,
R^{Z2} représente un atome d'hydrogène, un groupe cyclopropyle, hétérocyclyle à 5 ou 6 chaînons ou hétéroaryle à 5 ou 6 chaînons
ou
représente un groupe alkyle en C₁-C₄ qui peut être substitué par hydroxy, méthoxy, éthoxy, amino, méthylamino, éthylamino, diméthylamino, diéthylamino, acétylamino, cyclopropyle, hétérocyclyle à 5 ou 6 chaînons ou par un groupe de formule -C(=O)-NR^{Z5}R^{Z6},
lesdits substituants méthoxy et éthoxy pouvant pour leur part être jusqu'à trois fois substitués par fluoro,
et
lesdits groupes hétérocyclyle pouvant porter jusqu'à deux substituants, identiques ou différents, méthyle, éthyle, oxo, hydroxy, méthoxy et éthoxy,
ainsi que
ledit groupe hétéroaryle pouvant porter jusqu'à deux substituants, identiques ou différents, fluoro, chloro, cyano, méthyle, éthyle, trifluorométhyle, méthoxy et éthoxy,
et où
R^{Z5} et R^{Z6} représentent indépendamment l'un de l'autre un atome d'hydrogène ou le groupe méthyle ou forment ensemble avec l'atome d'azote, auquel ils sont liés, un cycle pyrrolidine, pipéridine ou morpholine,
ou
R^{Z1} et R^{Z2} forment ensemble avec l'atome d'azote, auquel ils sont liés, un cycle pyrrolidine, pipéridine ou morpholine,
R¹ représente le groupe cyano,
R² représente un atome d'hydrogène, le groupe méthyle, méthylsulfonyle ou le groupe de formule -CH₂-C(=O)-NH₂, et
R³ représente un atome d'hydrogène,
ainsi que ses sels, produits de solvatation et produits de solvatation des sels.

4. Composé de formule (I) selon la revendication 1 ou 2, dans lequel
Z représente un groupement sulfoximine de formule 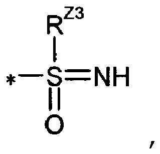 dans laquelle
* désigne le point d'attachement au cycle phényle,
et
R^{Z3} représente un groupe alkyle en C₁-C₄ qui peut être substitué par cyclopropyle ou jusqu'à trois fois par fluoro, ou le groupe cyclopropyle,
R¹ représente le groupe cyano,
R² représente un atome d'hydrogène, le groupe méthyle, méthylsulfonyle ou le groupe de formule -CH₂-C(=O)-NH₂,
et
R³ représente un atome d'hydrogène,
ainsi que ses sels, produits de solvatation et produits de solvatation des sels.

5. Composé de formule (I) selon la revendication 1, 2 ou 3, dans lequel
Z représente un groupement sulfonamido de formule 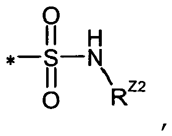 dans laquelle
* désigne le point d'attachement au cycle phényle,
et
R^{Z2} représente un atome d'hydrogène, le groupe méthyle ou le groupe de formule -CH₂-C(=O)-NH₂,
R¹ représente le groupe cyano,
R² représente un atome d'hydrogène, le groupe méthyle ou méthylsulfonyle,
et
R³ représente un atome d'hydrogène,
ainsi que ses sels, produits de solvatation et produits de solvatation des sels.

6. Composé de formule (I) selon la revendication 1, 2 ou 4, dans lequel
Z représente un groupement sulfoximine de formule 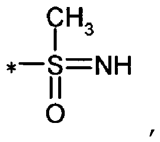 dans laquelle
* désigne le point d'attachement au cycle phényle,
R¹ représente le groupe cyano,
R² représente un atome d'hydrogène, le groupe méthyle ou méthylsulfonyle,
et
R³ représente un atome d'hydrogène,
ainsi que ses sels, produits de solvatation et produits de solvatation des sels.

7. Procédé pour la préparation des composés de formule (I) dans lesquels
Z représente un groupement sulfonamido de formule 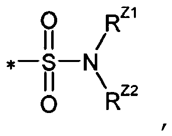 dans laquelle
* désigne le point d'attachement au cycle phényle,
et
R^{Z1} et R^{Z2} ont les significations indiquées dans les revendications 1, 2, 3 et 5,
**caractérisé en ce que** d'abord on convertit un dérivé d'aniline de formule (II) 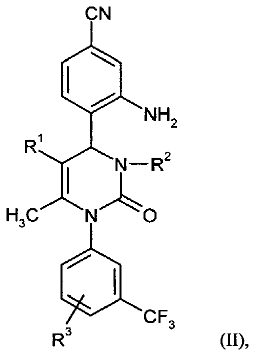 dans laquelle R¹, R² et R³ ont les significations indiquées dans les revendications 1, 2, 3 et 5,
avec du nitrite de sodium et de l'acide chlorhydrique en le sel de diazonium correspondant, ensuite on fait réagir ce dernier, dans une réaction en un seul récipient, avec du dioxyde de soufre, en présence de chlorure de cuivre(I), pour aboutir à un sulfochlorure de formule (III) 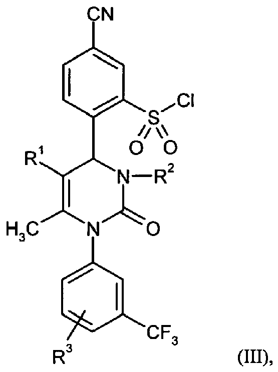 dans laquelle R¹, R² et R³ ont les significations indiquées plus haut,
et on fait ensuite réagir ce dernier avec une amine de formule (IV) 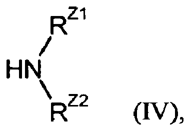 dans laquelle R^{Z1} et R^{Z2} ont les significations indiquées dans les revendications 1, 2, 3 et 5,
éventuellement en présence d'une base auxiliaire, pour aboutir au sulfonamide de formule (I-A) 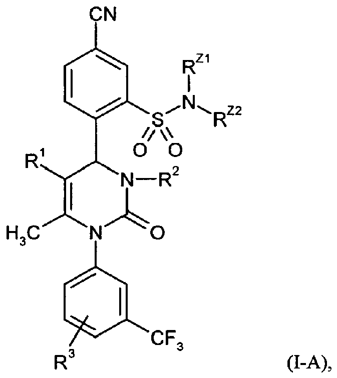 dans laquelle R¹, R², R³, R^{Z1} et R^{Z2} ont les significations indiquées plus haut
et éventuellement on fractionne les composés de formule (I-A) ainsi obtenus, selon des méthodes connues de l'homme de métier, en leurs énantiomères et/ou diastéréoisomères et/ou on les convertit, à l'aide (i) des solvants correspondants et/ou (ii) des bases correspondantes ou des acides correspondants, en leurs produits de solvatation, sels et/ou produits de solvatation des sels.

8. Procédé pour la préparation des composés de formule (I) dans lesquels
Z représente un groupement sulfoximine de formule 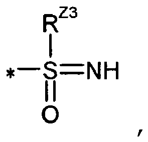 dans laquelle
* désigne le point d'attachement au cycle phényle,
et
R^{Z3} a la signification indiquée dans les revendications 1, 2, 4 et 6,
**caractérisé en ce que** d'abord on oxyde un dérivé de phénylthioéther de formule (V) 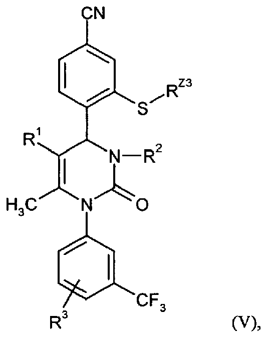 dans laquelle R¹, R², R³ et R^{Z3} ont les significations indiquées dans les revendications 1, 2, 4 et 6,
avec du peroxyde d'hydrogène, un peracide ou un periodate, pour aboutir au sulfoxyde de formule (VI) 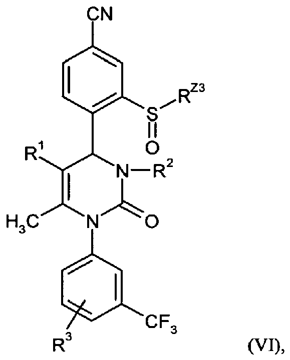 dans laquelle R¹, R², R³ et R^{Z3} ont les significations indiquées plus haut,
ensuite on fait réagir ce dernier avec du 2,2,2-trifluoroacétamide et du (diacétoxyiodo)-benzène en présence d'acétate de rhodium(II) dimère en tant que catalyseur et d'oxyde de magnésium en tant que base, pour aboutir à une N-acyl-sulfoximine de formule (VII) 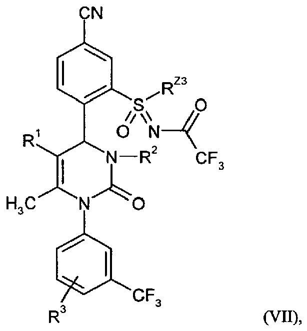 dans laquelle R¹, R², R³ et R^{Z3} ont les significations indiquées plus haut
et ensuite on élimine dans (VII) le groupe trifluoroacétyle, dans des conditions basiques, pour aboutir à la sulfoximine de formule (I-B) 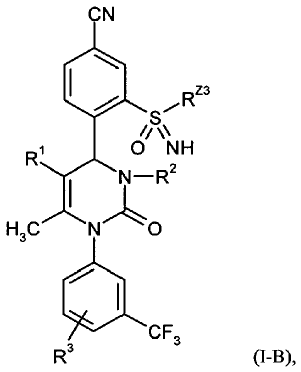 dans laquelle R¹, R², R³ et R^{Z3} ont les significations indiquées plus haut,
et éventuellement on fractionne les composés de formule (I-B) ainsi obtenus, selon des méthodes connues de l'homme de métier, en leurs énantiomères et/ou diastéréoisomères et/ou on les convertit, à l'aide (i) des solvants correspondants et/ou (ii) des bases correspondantes ou des acides correspondants, en leurs produits de solvatation, sels et/ou produits de solvatation des sels.

9. Composé, tel que défini dans l'une quelconque des revendications 1 à 6, destiné au traitement et/ou à la prévention de maladies.

10. Composé, tel que défini dans l'une quelconque des revendications 1 à 6, pour utilisation dans un procédé visant au traitement et/ou à la prévention de l'hypertonie artérielle pulmonaire (PAH) et d'autres formes de l'hypertonie pulmonaire (PH), de pneumopathies chroniques obstructives (COPD), de la lésion pulmonaire aiguë (ALI), du syndrome de détresse respiratoire aigu (ARDS), de l'emphysème pulmonaire, de la déficience en antitrypsine-alpha-1 (AATD) et de la fibrose kystique (CF).

11. Utilisation d'un composé, tel que défini dans l'une quelconque des revendications 1 à 6, pour la fabrication d'un médicament destiné au traitement et/ou à la prévention de l'hypertonie artérielle pulmonaire (PAH) et d'autres formes de l'hypertonie pulmonaire (PH), de pneumopathies chroniques obstructives (COPD), de la lésion pulmonaire aiguë (ALI), du syndrome de détresse respiratoire aigu (ARDS), de l'emphysème pulmonaire, de la déficience en antitrypsine-alpha-1 (AATD) et de la fibrose kystique (CF).

12. Médicament contenant un composé tel que défini dans l'une quelconque des revendications 1 à 6, en association avec un ou plusieurs adjuvants inertes, non toxiques, pharmaceutiquement appropriés.

13. Médicament contenant un composé tel que défini dans l'une quelconque des revendications 1 à 6, en association avec une ou plusieurs autres substances actives choisies dans le groupe constitué par des inhibiteurs de kinases, des inhibiteurs de métalloprotéases matricielles, des stimulants et des activateurs de la guanylate cyclase soluble, des analogues de prostacycline, des antagonistes des récepteurs de l'endothéline, des inhibiteurs de phosphodiestérases, des agonistes des récepteurs bêta-adrénergiques, des anticholinergiques et des glucocorticoïdes.

14. Médicament selon la revendication 12 ou 13, destiné au traitement et/ou à la prévention de l'hypertonie artérielle pulmonaire (PAH) et d'autres formes de l'hypertonie pulmonaire (PH), de pneumopathies chroniques obstructives (COPD), de la lésion pulmonaire aiguë (ALI), du syndrome de détresse respiratoire aigu (ARDS), de l'emphysème pulmonaire, de la déficience en antitrypsine-alpha-1 (AATD) et de la fibrose kystique (CF).
